# EUROPEAN PATENT APPLICATION

(11) **EP 4 238 567 A1**
(43) Date of publication of application: **06.09.2023**
(21) Application number: 21886411.4
(22) Date of filing: 29.10.2021
(51) Int. Cl.: A61K 31/675, A61K 31/519, A61K 31/53, A61K 31/551, A61P 1/00, A61P 3/00, A61P 5/00, A61P 9/00, A61P 11/00, A61P 13/00, A61P 15/00, A61P 19/10, A61P 21/00, A61P 25/00, A61P 25/02, A61P 25/28, A61P 35/00

(54) **NOVEL TREATMENT AND PREVENTION OF SARCOPENIA-RELATED DISEASES**

(30) Priority: 30.10.2020 JP 2020182813
(71) Applicant: Keio University, Tokyo, 108-8345 (JP); IRIMAJIRI THERAPEUTICS INC., Kochi-shi Kochi 781-0112 (JP)
(72) Inventor: HAYANO, Motoshi, Tokyo 160-8582 (JP); KOUJI, Hiroyuki, Fujisawa-shi, Kanagawa 251-8555 (JP)
(74) Representative: Vossius & Partner Patentanwälte Rechtsanwälte mbB
(86) International application number: PCT/JP2021/040137
(87) International publication number: WO 2022/092294

(57) **Abstract**

The present disclosure has solved problems associated with the TGF-β family or the like, which is called Myostatin/GDF-8 as a molecular mechanism and targeted by sarcopenia, by evaluating a pharmaceutical product in physiological aging models, and in particular, by evaluating an increase in muscle mass and an increase in muscle strength; and has overcome harmful effects by applying targets of diseases different from sarcopenia such as dystrophia. The present disclosure has unexpectedly found a use for an OK-1-related compound as a sarcopenia treatment method through a pathway different from that of Wnt signal (proliferation of muscle stem cells) associated with Wnt/β-catenin inhibition, and is thus provided due to the findings.

## Description

### [Technical Field]

The present disclosure relates to novel treatment/prevention of sarcopenia related diseases, disorders, or symptoms, etc. The present disclosure also provides a compound, composition, medicament, and therapeutic method that are useful for novel treatment/prevention of sarcopenia related diseases, disorders, or symptoms, etc.

### [Background Art]

Currently, development is ongoing at many companies, primarily targeting muscle differentiation or muscle cell death targeting a TGF-β family factor known as Myostatin/GDF-8. Thus, development targeting not only sarcopenia, but also muscular dystrophy as target diseases is ongoing (Patent Literatures 1 to 4 and Non Patent Literature 1) . In addition, monoclonal antibodies (bimagrumab) are developed by targeting Activin Receptor and ACVR2B. As of 2017, a phase 2 trial has been completed. Bimagrumab inhibits signaling by myostatin or activin A and promotes muscle differentiation to increase muscle mass. However, this has not resulted in dramatic improvement in sarcopenia symptoms including the gait speed and grip strength. Thus, development was discontinued in 2018.

While development targeting androgen receptors (MK-2866) is also ongoing, the relationship between hormone and muscle is not clear. Thus, development is uncertain.

### [Citation List]

### [Patent Literature]

[PTL 1] International Publication No. WO 2016/168613
[PTL 2] International Publication No. WO 2013/137832
[PTL 3] International Publication No. WO 2017/072515
[PTL 4] International Publication No. WO 2015/162590

### [Non Patent Literature]

[NPL 1] Arounleut P, Bialek P, Liang LF, Upadhyay S, Fulzele S, Johnson M, Elsalanty M, Isales CM, Hamrick MW. A myostatin inhibitor (propeptide-Fc) increases muscle mass and muscle fiber size in aged mice but does not increase bone density or bone strength. Exp Gerontol. 2013 Sep; 48(9): 898-904.

### [Summary of Invention]

### [Solution to Problem]

The present disclosure solves the problem associated with TGF-β family known as Myostatin/GDF-8, etc. being targeted for sarcopenia as a molecular mechanism by physiologically evaluating pharmaceutical products in aging models, especially by evaluating the increase in muscle mass or increase in muscle strength, to solve a negative effect from applying a target of disease that is different from sarcopenia such as dystrophy. The present disclosure is provided by unexpectedly discovering the use of OK-1 related compounds as a method of treating sarcopenia via a pathway that is different from Wnt signaling (growth of muscle stem cells) associated with Wnt/β catenin inhibition.

The present disclosure provides, for example, the following.

### (Item A1)

A composition for modulating, slowing the progression of, preventing, or treating a sarcopenia related disease, disorder, or symptom, comprising a compound selected from an OK-1 related compound group or a pharmaceutically acceptable salt thereof, or a solvate thereof.

### (Item 1)

A composition for modulating, slowing the progression of, preventing, or treating a sarcopenia related disease, disorder, or symptom, comprising a compound or a pharmaceutically acceptable salt thereof, or a solvate thereof, wherein the compound is a compound represented by compound group [1] 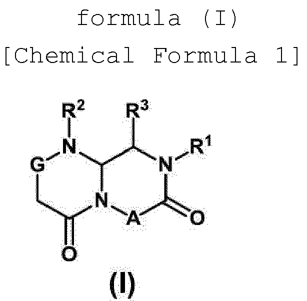 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O) (OH)₂, -OP(=O) (ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen,
R² is -W²¹-W²²-Rb-R²⁰ (wherein W²¹ is - (CO) -, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon,
a compound represented by compound group [2] 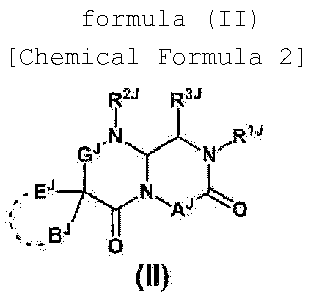 wherein
A^{J} is - (CHR^{7J})- (wherein R^{7J} is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
B^{J} and E^{J} are the same or different, independently selected from hydrogen, optionally substituted alkyl, optionally substituted alkenyl, and optionally substituted alkynyl, or form an optionally substituted spiro ring represented by a dotted line,
G^{J} is -NH-, -NR^{6J}-, -O-, -CH₂-, -CHR^{6J}-, or -C (R^{6J}) ₂-(wherein R^{6J} is each the same or different, independently selected from optionally substituted alkyl, optionally substituted alkenyl, and optionally substituted alkynyl),
R^{1J} is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R^{2J} is -W^{21J}-W^{22J}-Rb^{J}-R^{20J} (wherein W^{21J} is - (CO)- or - (SO₂)-, W^{22J} is a bond, -O-, -NH-, or optionally substituted lower alkylene, Rb^{J} is a bond or optionally substituted lower alkylene, and R^{20J} is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and R^{3J} is hydrogen, optionally substituted alkyl, optionally substituted alkenyl, or optionally substituted alkynyl,
a compound represented by compound group [3] 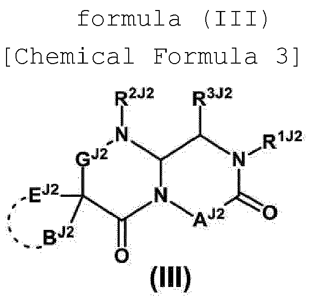 wherein
A^{J2} is - (CHR^{7J2}) - (wherein R^{7J2} is C₁₋₁₀ alkyl optionally substituted with a substituent selected from OH, -OR^{8J2} (wherein R^{8J2} represents C₆₋₁₄ aryl-C₁₋₁₀ alkyl), -NH₂, -COOH, and -CONH₂, C₆₋₁₄ aryl, or C₆₋₁₄ aryl-C₁₋₁₀ alkyl optionally substituted with OH);
B^{J2} and E^{J2} are hydrogen,
G^{J2} is -O-,
R^{1J2} is C₁₋₁₀ alkyl optionally substituted with a substituent selected from OH, -COOH, and -COOR^{8J2}' (wherein R^{8J2}' represents C₁₋₁₀ alkyl), C₆₋₁₄ aryl-C₁₋₁₀ alkyl, heteroaryl-C₁₋₁₀ alkyl optionally substituted with -NH₂, or C₃₋₁₀ cycloalkyl-C₁₋₁₀ alkyl,
R^{2J2} is -W^{21J2}-W^{22J2}-Rb^{J2}-R^{20J2} (wherein W^{21J2} is - (CO) - or - (SO₂) -, W^{22J2} is a bond, -O-, or C₁₋₆ alkylene, and Rb^{J2} is a bond or C₁₋₆ alkylene); and
R^{20J2} is C₁₋₁₀ alkyl, C₆₋₁₄ aryl optionally substituted with halogen, or heteroaryl); and
R^{3J2} is hydrogen;
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon,
compound represented by compound group [4] 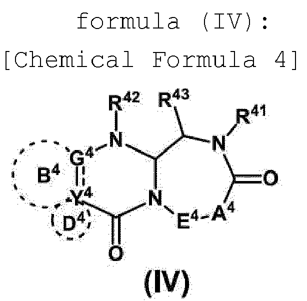 wherein 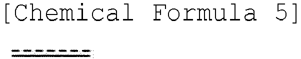 is a single bond or a double bond,
A⁴ is -CHR⁴⁷- (wherein R⁴⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
E⁴ is a bond, -CHR⁴⁵-, -O-, or -NR⁴⁸- (wherein R⁴⁵ is hydrogen, optionally substituted alkyl, optionally substituted alkenyl, optionally substituted alkynyl, optionally substituted arylalkyl, or optionally substituted heteroarylalkyl, and R⁴⁸ is hydrogen, optionally substituted alkyl, optionally substituted alkenyl, or optionally substituted alkynyl),
B⁴ is absent, or an optionally substituted monocyclic ring formed together with G⁴ and Y⁴,
D⁴ is absent, or an optionally substituted spiro ring formed together with Y⁴,
   with the proviso that not both of B⁴ and D⁴ are present,
if B⁴ is present, G⁴ and Y⁴ are independently a carbon atom or a nitrogen atom,
if D⁴ is present, Y⁴ is a carbon atom, and G⁴ is -NR⁴⁶-, -O-, -CHR⁴⁶-, or -C(R⁴⁶)₂-,
if both B⁴ and D⁴ are absent, G⁴ and Y⁴ are the same or different, each -NR⁴⁶-, -O-, -CHR⁴⁶-, or -C(R⁴⁶)₂- (wherein R⁴⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl), and
if E⁴ is a bond, D⁴ is absent, B⁴ is an optionally substituted monocyclic ring, and B⁴ and Y⁴ are independently a carbon atom or a nitrogen atom,
R⁴¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁴² is -W⁴²¹-W⁴²²-Rb⁴-R⁴²⁰ (wherein W⁴²¹ is - (CO) - or - (SO₂)-, W⁴²² is a bond, -O-, -NH-, or optionally substituted lower alkylene, Rb⁴ is a bond or optionally substituted alkylene, and R⁴²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁴³ is hydrogen, optionally substituted alkyl, optionally substituted alkenyl, or optionally substituted alkynyl,
a compound represented by 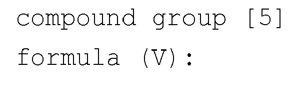 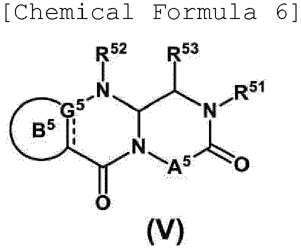 wherein 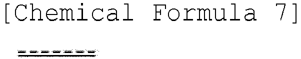 is a single bond or a double bond,
A⁵ is - (CHR⁵⁷) - (wherein R⁵⁷ is C₁₋₁₀ alkyl optionally substituted with a substituent selected from -OH, -OR⁵⁹ (wherein R⁵⁹ represents C₁₋₁₀ alkyl), -NH₂, -COOH, and -CONH₂, C₆₋₁₄ aryl, or C₆₋₁₄ aryl-C₁₋₁₀ alkyl optionally substituted with -OH),
B⁵ is a 5- or 6-membered monocyclic saturated carbon ring, or a saturated or unsaturated 4-, 5-, 6-, or 7-membered heterocyclic ring optionally substituted with a formyl group, formed together with G⁵, wherein a heteroatom is selected from S, N, and O, and the number of heteroatoms is an integer from 1 to 3,
G⁵ is a carbon atom or a nitrogen atom,
R⁵¹ is C₁₋₁₀ alkyl, C₆₋₁₄ aryl-C₁₋₁₀ alkyl optionally substituted with a substituent selected from -R^{59'} and halogen, or 5- to 14-membered heteroaryl-C₁₋₁₀ alkyl optionally substituted with -NHCOOR^{59'}, wherein R^{59'} is C₁₋₁₀ alkyl or C₆₋₁₄ aryl,
R⁵² is -W⁵²¹-W⁵²²-Rb⁵-R⁵²⁰ (wherein
   W⁵²¹ is - (CO) -,
   W⁵²² is -NH-,
   Rb⁵ is a bond, or C₁₋₆ alkylene optionally substituted with C₆₋₁₄ aryl, and
   R⁵²⁰ is C₁₋₁₀ alkyl optionally substituted with a substituent selected from -OH and -COOR^{59"} (wherein R^{59"} represents a hydrogen atom or C₁₋₁₀ alkyl), C₆₋₁₄ aryl optionally substituted with a substituent selected from halogen, methylenedioxy, and C₁₋₁₀ alkyl, 5- to 14-membered heteroaryl, or C₃₋₁₀ cycloalkyl), and
R⁵³ is hydrogen or C₁₋₁₀ alkyl,
a compound represented by 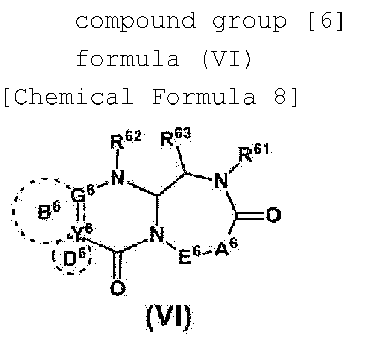 wherein 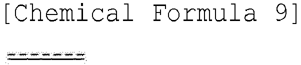 is a single bond or a double bond,
A is -CHR⁶⁷- (wherein R⁶⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
E⁶ is -CHR⁶⁵-, -O-, or -NR⁶⁸- (wherein R⁶⁵ is hydrogen, optionally substituted alkyl, optionally substituted alkenyl, optionally substituted alkynyl, optionally substituted arylalkyl, or optionally substituted heteroarylalkyl, and R⁶⁸ is hydrogen, optionally substituted alkyl, optionally substituted alkenyl, or optionally substituted alkynyl),
B⁶ is absent or an optionally substituted monocyclic ring formed together with G⁶ and Y⁶,
D⁶ is absent, or an optionally substituted spiro ring formed together with Y⁶, with the proviso that not both B⁶ and D⁶ are present,
if B⁶ is present, G⁶ and Y⁶ are independently a carbon atom or a nitrogen atom,
if D⁶ is present, Y⁶ is a carbon atom, and G⁶ is -NR⁶⁶-, -O-, -CHR⁶⁶-, or -C(R⁶⁶)₂-,
if B⁶ and D⁶ are both absent, G⁶ and Y⁶ are the same or different, each -NR⁶⁶-, -O-, -CHR⁶⁶-, or -C(R⁶⁶)₂- (wherein R⁶⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl),
R⁶¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁶² is -W⁶²¹-W⁶²²-Rb⁶-R⁶²⁰ (wherein W⁶²¹ is - (CO) - or - (SO₂) -, W⁶²² is a bond, -O-, -NH-, or optionally substituted lower alkylene, Rb⁶ is a bond or optionally substituted alkylene, and R⁶²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁶³ is hydrogen, optionally substituted alkyl, optionally substituted alkenyl, or optionally substituted alkynyl,
a compound represented by 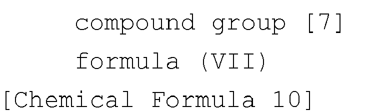 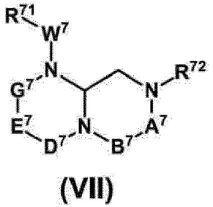 wherein
A⁷ is - (CHR⁷³) - (C=O) -, B⁷ is - (NR⁷⁴) -, D⁷ is - (CHR⁷⁵) - or - (C=O) -, E⁷ is -(Z⁷R⁷⁶)- or -(C=O)-, G⁷ is -(X⁷R⁷⁷)ₙ₇-, -(CHR⁷⁷)-(NR⁷⁸)-, -(C=O)-(X⁷R⁷⁹-, or -(C=O)-, W⁷ is -Y⁷(C=O)-, - (C=O)NH-, -(SO₂)-, or absent, Y⁷ is oxygen or sulfur, X⁷ and Z⁷ are independently nitrogen or CH, n7 = 0 or 1, and R⁷¹, R⁷², R⁷³, R⁷⁴, R⁷⁵, R⁷⁶, R⁷⁷, R⁷⁸, and R⁷⁹ are the same or different, independently selected from an amino acid side chain, a derivative of an amino acid side chain, a linker facilitating the linkage of the compound to another moiety or compound, a linker attaching the compound to a solid support, and a solid support, and stereoisomers thereof,
a compound represented by 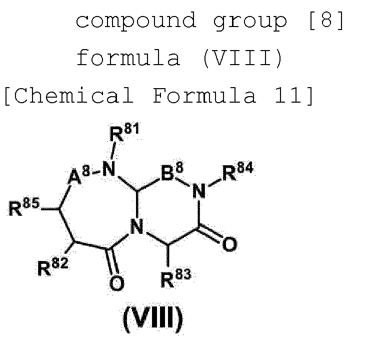 wherein
A⁸ is -(CHR⁸')ₙ₈-, B⁸ is -(CHR^{8"})ₘ₈-, n8 is 0, 1, or 2, m8 is 1, 2, or 3, R^{8'}, R^{8"}, R⁸², R⁸³, and R⁸⁵ are the same or different, each of R⁸', R^{8"}, R⁸², R⁸³, and R⁸⁵ that are present is independently selected from the group consisting of an amino acid side chain moiety, an amino acid side chain derivative, a linker, and a solid support, R⁸¹ and R⁸⁴ represent the remaining portions of the compound, wherein R⁸¹ and R⁸⁴ are the same or different, selected from the group consisting of a certain moiety, an active substance, a compound, a support, a molecule, a linker, an amino acid, a peptide, and a protein, or a compound represented by 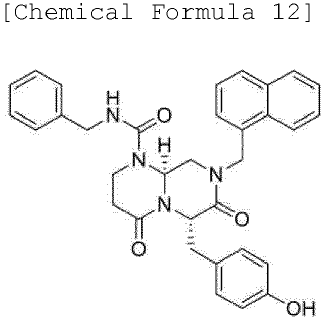 or a derivative thereof, or
a compound represented by 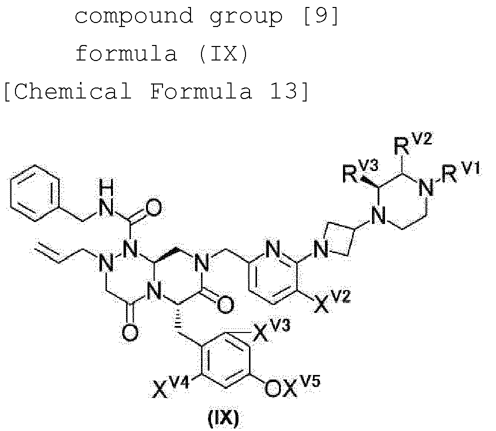 wherein R^{V1} refers to a C₁₋₆ alkyl group, R^{V2} and R^{V3} are each the same or different, referring to a hydrogen atom or a C₁₋₆ alkyl group, X^{V2}, X^{V3}, and X^{V4} are each the same or different, referring to a hydrogen atom or a halogen atom, and X^{V5} refers to a hydrogen atom or -P(=O) (OH)₂.

### (Item 2)

The composition of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item 3)

The composition of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item 4)

The composition of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item 5)

The composition of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item 6)

The composition of any one of the preceding items, wherein the compound is selected from the compound group [1].

### (Item 7)

The composition of any one of the preceding items, wherein the compound or pharmaceutically acceptable salt thereof or solvate thereof can be orally administrated.

### (Item 8)

The composition of any one of the preceding items, wherein the composition is administered in combination with at least one of physical therapy, dietary therapy, and other drug therapy.

### (Item 9)

The composition of any one of the preceding items, wherein the composition is administered in combination with physical therapy.

### (Item 10)

The composition of any one of the preceding items, wherein the modulation, slowing of progression, prevention, or treatment comprises modulation, slowing of progression, prevention, or treatment in at least one of reduced muscle mass, reduced (muscular) endurance, reduced short-term memory, reduced bone density, and osteoporosis.

### (Item 11)

The composition of any one of the preceding items, wherein the modulation, slowing of progression, prevention, or treatment comprises modulation, slowing of progression, prevention, or treatment in at least one of reduced muscle mass and reduced (muscular) endurance.

### (Item 12)

The composition of any one of the preceding items, wherein the modulation, slowing of progression, prevention, or treatment is confirmed by modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item 13)

The composition of any one of the preceding items, wherein the modulation, slowing of progression, prevention, or treatment is confirmed by modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item 14)

A composition for use in diagnosis or testing for a sarcopenia related disease, disorder, or symptom of a subject, wherein the composition comprises means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 15)

The composition of any one of the preceding items, wherein the composition comprises means or a reagent for identifying the presence/absence or level of expression of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 16)

A composition for modulating, slowing the progression of, preventing, or treating a sarcopenia related disease, disorder, or symptom, comprising a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 17)

The composition of any one of the preceding items, wherein the composition comprises a substance or agent for modulating at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 18)

The composition of any one of the preceding items for modulating, slowing the progression of, preventing, or treating a sarcopenia related disease, disorder, or symptom of a subject, wherein the composition is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item 19)

The composition of any one of the preceding items, wherein a status of modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2, is checked.

### (Item 20)

The composition of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item 21)

The composition of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item 22)

The composition of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item 23)

The composition of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item 24)

A method for screening a substance or agent for modulating, slowing the progression of, preventing, or treating a sarcopenia related disease, disorder, or symptom, the method comprising:
A) administering a candidate substance or agent to a subject;
B) checking modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 in the subject; and
C) identifying the candidate substance or agent as a substance or agent that can have an ability to modulate, slow the progression of, prevent, or treat a sarcopenia related disease, disorder, or symptom based on a result of B) .

### (Item 25)

The method of any one of the preceding items, wherein B) checks modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 26)

The method of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item 27)

The method of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item 28)

The method of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders.

### (Item 29)

The composition of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item 1A)

A composition for modulating, slowing the progression of, preventing, or treating a senescence related disease, disorder, or symptom comprising at least one of an OK-1 related compound group.

### (Item 2A)

The composition of any one of the preceding items, wherein the senescence related disease, disorder, or symptom is selected from the group consisting of sarcopenia, a lifestyle disease (non-wasting disease), a wasting disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item 3A)

The composition of any one of the preceding items, wherein the senescence related disease, disorder, or symptom is selected from the group consisting of sarcopenia, dementia, a metabolic disease, rheumatoid arthritis, osteoporosis, and reduced muscle mass.

### (Item 4A)

The composition of any one of items 1A to 3A, further comprising one or more features of any one or more of items A1 and 1 to 11.

### (Item 5A)

A composition for use in diagnosis or testing for a senescence related disease, disorder, or symptom of a subject, wherein the composition comprises means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 6A)

The composition of any one of the preceding items, wherein the composition comprises means or a reagent for identifying the presence/absence or level of expression of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 7A)

A composition for modulating, slowing the progression of, preventing, or treating a senescence related disease, disorder, or symptom, comprising a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 8A)

The composition of any one of the preceding items, wherein the composition comprises a substance or agent for modulating at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 9A)

The composition of any one of the preceding items for modulating, slowing the progression of, preventing, or treating a senescence related disease, disorder, or symptom of a subject, wherein the composition is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item 10A)

The composition of any one of the preceding items, wherein a status of modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 is checked.

### (Item 11A)

The composition of any one of the preceding items, wherein the senescence related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item 12A)

The composition of any one of the preceding items, wherein the senescence related disease, disorder, or symptom comprises at least one selected from the group consisting of primary sarcopenia, secondary sarcopenia, and a disease, disorder, or symptom accompanied therewith.

### (Item 13A)

The composition of any one of the preceding items, wherein the senescence related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item 14A)

The composition of any one of the preceding items, wherein the senescence related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item 15A)

A method for screening a substance or agent for modulating, slowing the progression of, preventing, or treating a senescence related disease, disorder, or symptom, the method comprising:
A) administering a candidate substance or agent to a subject;
B) checking modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 in the subject; and
C) identifying the candidate substance or agent as a substance or agent that can have an ability to modulate, slow the progression of, prevent, or treat a senescence related disease, disorder, or symptom based on a result of B) .

### (Item 16A)

The method of any one of the preceding items, wherein B) checks modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item 17A)

The composition of any one of the preceding items, wherein the senescence related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item 18A)

The method of any one of the preceding items, wherein the senescence related disease, disorder, or symptom comprises at least one selected from the group consisting of primary sarcopenia, secondary sarcopenia, and a disease, disorder, or symptom accompanied therewith.

### (Item 19A)

The method of any one of the preceding items, wherein the senescence related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders.

### (Item 20A)

The composition of any one of the preceding items, wherein the senescence related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item X1)

Use of a compound or a pharmaceutically acceptable salt thereof, or a solvate thereof, selected from an OK-1 related compound group in the manufacture of a medicament for modulating, slowing the progression of, preventing, or treating a sarcopenia related disease, disorder, or symptom.

### (Item X2)

The use of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item X3)

The use of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item X4)

The use of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item X5)

The use of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item X6)

The use of any one of the preceding items, wherein the compound is selected from the compound group [1].

### (Item X7)

The use of any one of the preceding items, wherein the compound or pharmaceutically acceptable salt thereof or solvate thereof can be orally administrated.

### (Item X8)

The use of any one of the preceding items, wherein the medicament is administered in combination with at least one of physical therapy, dietary therapy, and other drug therapy.

### (Item X9)

The use of any one of the preceding items, wherein the medicament is administered in combination with physical therapy.

### (Item X10)

The use of any one of the preceding items, wherein the modulation, slowing of progression, prevention, or treatment comprises modulation, slowing of progression, prevention, or treatment in at least one of reduced muscle mass, reduced (muscular) endurance, reduced short-term memory, reduced bone density, and osteoporosis.

### (Item X11)

The use of any one of the preceding items, wherein the modulation, slowing of progression, prevention, or treatment comprises modulation, slowing of progression, prevention, or treatment in at least one of reduced muscle mass and reduced (muscular) endurance.

### (Item X12)

The use of any one of the preceding items, wherein the modulation, slowing of progression, prevention, or treatment is confirmed by modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item X13)

The use of any one of the preceding items, wherein the modulation, slowing of progression, prevention, or treatment of the subject is confirmed by modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item X14)

Use of means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2, in the manufacture of a medicament for use in diagnosis or testing for a sarcopenia related disease, disorder, or symptom of a subject.

### (Item X15)

The use of any one of the preceding items, wherein the medicament comprises means or a reagent for identifying the presence/absence or level of expression of at least one selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf.

### (Item X16)

Use of a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2, in the manufacture of a medicament for modulating, slowing the progression of, preventing, or treating a sarcopenia related disease, disorder, or symptom.

### (Item X17)

The use of any one of the preceding items, wherein the medicament comprises a substance or agent for modulating at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

### (Item X18)

Use of an OK-1 related compound in the manufacture of a medicament of any one of the preceding items, for modulating, slowing the progression of, preventing, or treating a sarcopenia related disease, disorder, or symptom of a subject, wherein the medicament is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

### (Item X19)

The use of any one of the preceding items, wherein a status of modulation of at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2, is checked.

### (Item X20)

The use of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item X21)

The use of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item X22)

The use of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item X23)

The use of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item X1A)

Use of a compound or a pharmaceutically acceptable salt thereof, or a solvate thereof, comprising at least one of an OK-1 related compound group in the manufacture of a medicament for modulating, slowing the progression of, preventing, or treating a senescence related disease, disorder, or symptom.

### (Item X2A)

The use of any one of the preceding items, wherein the senescence related disease, disorder, or symptom is selected from the group consisting of sarcopenia, a lifestyle disease (non-wasting disease), a wasting disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item X3A)

The use of any one of the preceding items, wherein the senescence related disease, disorder, or symptom is selected from the group consisting of sarcopenia, dementia, a metabolic disease, rheumatoid arthritis, osteoporosis, and reduced muscle mass.

### (Item X4A)

The use of any one of items X1A to X3A, further comprising one or more features of any one or more of items A1, 1 to 11, and X1 to X11.

### (Item X5A)

Use of means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2, in the manufacture of a medicament for use in diagnosis or testing for a senescence related disease, disorder, or symptom of a subject.

### (Item X6A)

The use or medicament of any one of the preceding items, wherein the medicament comprises means or a reagent for identifying the presence/absence or level of expression of at least one selected from the group consisting of Pkp1, Sprr1a, Lce1a1, Lce1b, Lcela2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2.

### (Item X7A)

Use of a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2, in the manufacture of a medicament for modulating, slowing the progression of, preventing, or treating a senescence related disease, disorder, or symptom.

### (Item X8A)

The use of any one of the preceding items, wherein the substance or agent comprises a substance or agent for modulating at least one gene selected from the group consisting of Pkp1, Sprrla, Lce1a1, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2.

### (Item X9A)

Use of an OK-1 related compound in the manufacture of a medicament of any one of the preceding items, for modulating, slowing the progression of, preventing, or treating a senescence related disease, disorder, or symptom of a subject, wherein the composition is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2 of the subject.

### (Item X10A)

The use of any one of the preceding items, wherein a status of modulation of at least one gene selected from the group consisting of Pkp1, Sprr1a, Lce1a1, Lce1b, Lcela2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf2, is checked.

### (Item X11A)

The use of any one of the preceding items, wherein the senescence related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item X12A)

The use of any one of the preceding items, wherein the senescence related disease, disorder, or symptom comprises at least one selected from the group consisting of primary sarcopenia, secondary sarcopenia, and a disease, disorder, or symptom accompanied therewith.

### (Item X13A)

The use of any one of the preceding items, wherein the senescence related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item X14A)

The use of any one of the preceding items, wherein the senescence related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Y1)

A method of modulating, slowing the progression of, preventing, or treating a sarcopenia related disease, disorder, or symptom of a subject, comprising administering to the subject an effective amount of a compound selected from an OK-1 related compound group or a pharmaceutically acceptable salt thereof, or a solvate thereof.

### (Item Y2)

The method of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Y3)

The method of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item Y4)

The method of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Y5)

The method of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Y6)

The method of any one of the preceding items, wherein the compound or pharmaceutically acceptable salt thereof, or solvate thereof, is selected from the compound group [1].

### (Item Y7)

The method of any one of the preceding items, wherein the compound or pharmaceutically acceptable salt thereof, or solvate thereof, or a pharmaceutically acceptable salt thereof, or a solvate thereof, can be orally administrated.

### (Item Y8)

The method of any one of the preceding items, wherein the compound or pharmaceutically acceptable salt thereof, or solvate thereof, is administered in combination with at least one of physical therapy, dietary therapy, and other drug therapy.

### (Item Y9)

The method of any one of the preceding items, wherein the compound is administered in combination with physical therapy.

### (Item Y10)

The method of any one of the preceding items, wherein the modulation, slowing of progression, prevention, or treatment comprises modulation, slowing of progression, prevention, or treatment in at least one of reduced muscle mass, reduced (muscular) endurance, reduced short-term memory, reduced bone density, and osteoporosis.

### (Item Y11)

The method of any one of the preceding items, wherein the modulation, slowing of progression, prevention, or treatment comprises modulation, slowing of progression, prevention, or treatment in at least one of reduced muscle mass and reduced (muscular) endurance.

### (Item Y12)

The method of any one of the preceding items, wherein the modulation, slowing of progression, prevention, or treatment is confirmed by modulation of at least one gene selected from the group consisting of Ppara, Pou2f2, Foxo1, Pparg, Pou2f1, Trp63, Sp1, Tcf3, Zebl, Srebfl, Sp3, Ebfl, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtpl, Dsc1, Dsg1a, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf2 of the subject.

### (Item Y13)

The method of any one of the preceding items, wherein the modulation, slowing of progression, prevention, or treatment of the subject is confirmed by modulation of at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkbl, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf of the subject.

### (Item Y14)

A method of diagnosing or testing a sarcopenia related disease, disorder, or symptom of a subject, the method comprising determining the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebfl, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtpl, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosll, Nfkbl, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf of the subject.

### (Item Y15)

The method of any one of the preceding items, wherein the method comprises determining administration of means or a reagent for identifying the presence/absence or level of expression of at least one selected from the group consisting of Pkpl, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfbl, Gas2, Serpinhl, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkbl, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf.

### (Item Y16)

A method of modulating, slowing the progression of, preventing, or treating a sarcopenia related disease, disorder, or symptom in a subject, comprising administering to the subject an effective amount of a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbl1, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf.

### (Item Y17)

The method of any one of the preceding items, wherein the method comprises administering to the subject an effective amount of a substance or agent for modulating at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxrl, Fbln2, Tgfbl, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbpl, Myod1, Srebf1, Jun, Stat3, Sp3, Myf6, Nr3c1, Foxo3, Nfe212, Myf5, Ahr, Ep300, Myog, and Usf.

### (Item Y18)

The method of any one of the preceding items for modulating, slowing the progression of, preventing, or treating a sarcopenia related disease, disorder, or symptom of a subject, wherein the compound is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtpl, Dsc1, Dsgla, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbll, Gm26910, Pzp, 4933406L23Rik, Fbln1, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf of the subject.

### (Item Y19)

The method of any one of the preceding items, wherein a status of modulation of at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebpl, Adamts2, Collal, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf, is checked.

### (Item Y20)

The method of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Y21)

The method of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item Y22)

The method of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Y23)

The method of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Y1A)

A method of modulating, slowing the progression of, preventing, or treating a senescence related disease, disorder, or symptom in a subject, comprising administering to the subject an effective amount of a compound comprising at least one in an OK-1 related compound group or a pharmaceutically acceptable salt thereof, or a solvate thereof.

### (Item Y2A)

The method of any one of the preceding items, wherein the senescence related disease, disorder, or symptom is selected from the group consisting of sarcopenia, a lifestyle disease (non-wasting disease), a wasting disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Y3A)

The method of any one of the preceding items, wherein the senescence related disease, disorder, or symptom is selected from the group consisting of sarcopenia, dementia, a metabolic disease, rheumatoid arthritis, osteoporosis, and reduced muscle mass.

### (Item Y4A)

The method of any one of items 1A to Y3A, further comprising one or more features of any one or more of items A1, 1 to 11, X1 to X11, and Y1 to Y11.

### (Item Y5A)

A method of diagnosing or testing a senescence related disease, disorder, or symptom of a subject, the method comprising determining the presence/absence or level of expression of at least one gene selected from the group consisting of Ppara, Pou2f2, Foxo1, Pparg, Pou2f1, Trp63, Sp1, Tcf3, Zebl, Srebfl, Sp3, Ebfl, Mycn, Pbxl, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, I133, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf of the subject.

### (Item Y6A)

The method of any one of the preceding items, wherein the method comprises identifying the presence/absence or level of expression of at least one selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebpl, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Y7A)

A method for modulating, slowing the progression of, preventing, or treating a senescence related disease, disorder, or symptom in a subject, comprising administering to the subject an effective amount of a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosl1, Nfkb1, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Y8A)

The method of any one of the preceding items, wherein the method comprises administering to the subject an effective amount of a substance or agent for modulating at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxr1, Fbln2, Tgfbl, Gas2, Serpinhl, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, and Lox, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Y9A)

The method of any one of the preceding items for modulating, slowing the progression of, preventing, or treating a senescence related disease, disorder, or symptom of a subject, wherein an effective amount of the compound or pharmaceutically acceptable salt thereof, or solvate thereof, is administered to the subject when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zebl, Srebfl, Sp3, Ebfl, Mycn, Pbxl, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtpl, Dsc1, Dsgla, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbl1, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebfl, Jun, Stat3, Sp3, Myf6, Nr3c1, Foxo3, Nfe212, Myf5, Ahr, Ep300, Myog, and Usf of the subject.

### (Item Y10A)

The method of any one of the preceding items, wherein a status of modulation of at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxr1, Fbln2, Tgfb1, Gas2, Serpinhl, Comp, Mmp2, Mmp12, Mmp11, Lum, Aebpl, Adamts2, Collal, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkbl, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf, is checked.

### (Item Y11A)

The composition of any one of the preceding items, wherein the senescence related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Y12A)

The composition of any one of the preceding items, wherein the senescence related disease, disorder, or symptom comprises at least one selected from the group consisting of primary sarcopenia, secondary sarcopenia, and a disease, disorder, or symptom accompanied therewith.

### (Item Y13A)

The composition of any one of the preceding items, wherein the senescence related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Y14A)

The composition of any one of the preceding items, wherein the senescence related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Z1)

A compound selected from an OK-1 related compound group or a pharmaceutically acceptable salt thereof, or a solvate thereof, for modulating, slowing the progression of, preventing, or treating a sarcopenia related disease, disorder, or symptom.

### (Item Z2)

The compound or pharmaceutically acceptable salt thereof, or solvate thereof, of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Z3)

The compound or pharmaceutically acceptable salt thereof, or solvate thereof, of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item Z4)

The compound or pharmaceutically acceptable salt thereof, or solvate thereof, of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Z5)

The compound or pharmaceutically acceptable salt thereof, or solvate thereof, of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Z6)

The compound or pharmaceutically acceptable salt thereof, or solvate thereof, of any one of the preceding items, wherein the compound is selected from the compound group [1].

### (Item Z7)

The compound or pharmaceutically acceptable salt thereof, or solvate thereof, of any one of the preceding items, wherein the compound or pharmaceutically acceptable salt thereof or solvate thereof can be orally administrated.

### (Item Z8)

The compound or pharmaceutically acceptable salt thereof, or solvate thereof, of any one of the preceding items, wherein the compound or pharmaceutically acceptable salt thereof, or solvate thereof, is administered in combination with at least one of physical therapy, dietary therapy, and other drug therapy.

### (Item Z9)

The compound or pharmaceutically acceptable salt thereof, or solvate thereof, of any one of the preceding items, wherein the compound or pharmaceutically acceptable salt thereof, or solvate thereof, is administered in combination with physical therapy.

### (Item Z10)

The compound or pharmaceutically acceptable salt thereof, or solvate thereof, of any one of the preceding items, wherein the modulation, slowing of progression, prevention, or treatment comprises modulation, slowing of progression, prevention, or treatment in at least one of reduced muscle mass, reduced (muscular) endurance, reduced short-term memory, reduced bone density, and osteoporosis.

### (Item Z11)

The compound or pharmaceutically acceptable salt thereof, or solvate thereof, of any one of the preceding items, wherein the modulation, slowing of progression, prevention, or treatment comprises modulation, slowing of progression, prevention, or treatment in at least one of reduced muscle mass and reduced (muscular) endurance.

### (Item Z12)

The compound or pharmaceutically acceptable salt thereof, or solvate thereof, of any one of the preceding items, wherein the modulation, slowing of progression, prevention, or treatment is confirmed by modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbll, Gm26910, Pzp, 4933406L23Rik, Fbln1, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf of the subject.

### (Item Z13)

The compound or pharmaceutically acceptable salt thereof, or solvate thereof, of any one of the preceding items, wherein the modulation, slowing of progression, prevention, or treatment of the subject is confirmed by modulation of at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinhl, Comp, Mmp2, Mmp12, Mmp11, Lum, Aebpl, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf of the subject.

### (Item Z14)

Means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebfl, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtpl, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf, for diagnosing or testing a sarcopenia related disease, disorder, or symptom of a subject.

### (Item Z15)

The compound or pharmaceutically acceptable salt thereof, or solvate thereof, of any one of the preceding items, wherein the means or reagent comprises means or a reagent for identifying the presence/absence or level of expression of at least one selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebpl, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Z16)

A compound or a pharmaceutically acceptable salt thereof, or a solvate thereof, for modulating, slowing the progression of, preventing, or treating a sarcopenia related disease, disorder, or symptom, comprising a substance or agent for modulating at least one gene selected from the group consisting of Ppara, Pou2f2, Foxo1, Pparg, Pou2f1, Trp63, Sp1, Tcf3, Zebl, Srebfl, Sp3, Ebfl, Mycn, Pbxl, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Z17)

The compound or pharmaceutically acceptable salt thereof, or solvate thereof, of any one of the preceding items, wherein the substance or agent for modulating a gene comprises a substance or agent for modulating at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxrl, Fbln2, Tgfbl, Gas2, Serpinhl, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Z18)

The compound or pharmaceutically acceptable salt thereof, or solvate thereof, of any one of the preceding items for modulating, slowing the progression of, preventing, or treating a sarcopenia related disease, disorder, or symptom of a subject, wherein the composition is administered when it is determined to be necessary or suitable based on a status of modulation, which is checked for at least one gene selected from the group consisting of Ppara, Pou2f2, Foxol, Pparg, Pou2f1, Trp63, Sp1, Tcf3, Zebl, Srebfl, Sp3, Ebfl, Mycn, Pbxl, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosll, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, Il33, Nbl1, Gm26910, Pzp, 4933406L23Rik, Fbln1, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf of the subject.

### (Item Z19)

The compound or pharmaceutically acceptable salt thereof, or solvate thereof, of any one of the preceding items, wherein a status of modulation of at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf, is checked.

### (Item Z20)

The compound or pharmaceutically acceptable salt thereof, or solvate thereof, of any one of the preceding items, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Z21)

The compound or pharmaceutically acceptable salt thereof, or solvate thereof, of any one of the preceding items, wherein the sarcopenia related disease, disorder, or symptom comprises at least one selected from the group consisting of primary sarcopenia, secondary sarcopenia, and a disease, disorder, or symptom accompanied therewith.

### (Item Z22)

The compound or pharmaceutically acceptable salt thereof, or solvate thereof, of any one of the preceding items, wherein the sarcopenia related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Z23)

The compound or pharmaceutically acceptable salt thereof, or solvate thereof, of any one of the preceding items, wherein the sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### (Item Z1A)

A compound comprising at least one of an OK-1 related compound group, a pharmaceutically acceptable salt thereof, or a solvate thereof, for modulating, slowing the progression of, preventing, or treating a senescence related disease, disorder, or symptom.

### (Item Z2A)

The compound or pharmaceutically acceptable salt thereof, or solvate thereof, of any one of the preceding items, wherein the senescence related disease, disorder, or symptom is selected from the group consisting of sarcopenia, a lifestyle disease (non-wasting disease), a wasting disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Z3A)

The compound or pharmaceutically acceptable salt thereof, or solvate thereof, of any one of the preceding items, wherein the senescence related disease, disorder, or symptom is selected from the group consisting of sarcopenia, dementia, a metabolic disease, rheumatoid arthritis, osteoporosis, and reduced muscle mass.

### (Item Z4A)

The compound or pharmaceutically acceptable salt thereof, or solvate thereof, of any one of items Z1A to Z3A, further comprising one or more features of any one or more of items A1, 1 to 11, X1 to X11, Y1 to Y11, and Z1 to Z11.

### (Item Z5A)

Means or a reagent for identifying the presence/absence or level of expression of at least one gene selected from the group consisting of Ppara, Pou2f2, Foxo1, Pparg, Pou2f1, Trp63, Sp1, Tcf3, Zebl, Srebfl, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf, for diagnosing or testing a senescence related disease, disorder, or symptom of a subject.

### (Item Z6A)

The compound or pharmaceutically acceptable salt thereof, or solvate thereof, of any one of the preceding items, wherein the means or reagent comprises means or a reagent for identifying the presence/absence or level of expression of at least one selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxr1, Fbln2, Tgfbl, Gas2, Serpinhl, Comp, Mmp2, Mmp12, Mmp11, Lum, Aebpl, Adamts2, Collal, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf.

### (Item Z7A)

A composition for modulating, slowing the progression of, preventing, or treating a senescence related disease, disorder, or symptom, comprising a substance or agent for modulating at least one gene selected from the group consisting of Ppara, Pou2f2, Foxo1, Pparg, Pou2f1, Trp63, Sp1, Tcf3, Zeb1, Srebfl, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtpl, Dsc1, Dsgla, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, I133, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf.

### (Item Z8A)

The compound or pharmaceutically acceptable salt thereof, or solvate thereof, of any one of the preceding items, wherein the substance or agent for modulating a gene comprises a substance or agent for modulating at least one gene selected from the group consisting of Pkpl, Sprrla, Lcelal, Lcelb, Lcela2, Lcelc, Lcelm, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Colllal, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebpl, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, and Lox, preferably a group further comprising Nfkb1, Srebf2, Sp1, Trp53, Xbp1, Myod1, Srebfl, Jun, Stat3, Sp3, Myf6, Nr3c1, Foxo3, Nfe212, Myf5, Ahr, Ep300, Myog, and Usf.

### (Item Z9A)

The compound or pharmaceutically acceptable salt thereof, or solvate thereof, of any one of the preceding items for modulating, slowing the progression of, preventing, or treating a senescence related disease, disorder, or symptom of a subject, wherein the composition is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Poflb, Krtap6-1, Krtap14, 1110025L11Rik, Krtap8-1, Gm10229, Krt33a, Crct1, Igkv5-39, Tgtp1, Dsc1, Dsgla, Serpinb5, Krt14, Krtap21-1, Abca12, Gm10228, Krt77, S100a14, Gm49425, Krtap19-3, Ighgl, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, I133, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lparl, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, and Cx3cr1, preferably a group further comprising Nfkb1, Srebf2, Sp1, Trp53, Xbp1, Myod1, Srebf1, Jun, Stat3, Sp3, Myf6, Nr3c1, Foxo3, Nfe212, Myf5, Ahr, Ep300, Myog, and Usf of the subject.

### (Item Z10A)

The compound or pharmaceutically acceptable salt thereof, or solvate thereof, of any one of the preceding items, wherein a status of modulation of at least one gene selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsgla, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebpl, Adamts2, Collal, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, and Lox, preferably a group further comprising Nfkbl, Srebf2, Sp1, Trp53, Xbpl, Myod1, Srebfl, Jun, Stat3, Sp3, Myf6, Nr3c1, Foxo3, Nfe212, Myf5, Ahr, Ep300, Myog, and Usf, is checked.

### (Item Z11A)

The compound or pharmaceutically acceptable salt thereof, or solvate thereof, of any one of the preceding items, wherein the senescence related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

### (Item Z12A)

The compound or pharmaceutically acceptable salt thereof, or solvate thereof, of any one of the preceding items, wherein the senescence related disease, disorder, or symptom comprises at least one selected from the group consisting of primary sarcopenia, secondary sarcopenia, and a disease, disorder, or symptom accompanied therewith.

### (Item Z13A)

The compound or pharmaceutically acceptable salt thereof, or solvate thereof, of any one of the preceding items, wherein the senescence related disease, disorder, or symptom is selected from the group consisting of a sarcopenia associated life-style disease (non-wasting disease), a wasting disease, a tumor associated disease, a motor disease, a neurodegenerative disease, cognitive dysfunction, and other associated disorders (including undernutrition, frailty, spinal cord injury, inactivity, reduced activity, disuse syndrome, trauma, and invasive surgery, etc.).

### (Item Z14A)

The compound or pharmaceutically acceptable salt thereof, or solvate thereof, of any one of the preceding items, wherein the senescence related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

### [Item B1]

A composition for modulating, slowing the progression of, preventing, or treating a sarcopenia related disease, disorder, or symptom, comprising a compound or a pharmaceutically acceptable salt thereof, or a solvate thereof, wherein the compound is a compound represented by formula (I) 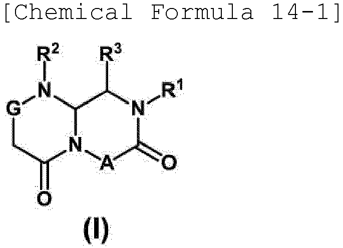 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP (=O) (OH)₂, -OP (=O) (ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen,
R² is -W²¹-W²²-Rb-R²⁰ (wherein W²¹ is -(CO)-, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon.

### [Item B2]

The composition of any one of the preceding items, wherein R⁷ is C₆₋₁₄ aryl-C₁₋₁₀ alkyl optionally substituted with a substituent selected from -OH, -OP(=O) (OH)₂, -OP(=O) (ONa)₂, and C₁₋₁₀ alkyl, heteroaryl-C₁₋₁₀ alkyl, or C₃₋₁₀ cycloalkyl-C₁₋₁₀ alkyl.

### [Item B3]

The composition of any one of the preceding items, wherein R² is -W²¹-W²²-Rb-R²⁰ (wherein W²¹ is - (CO)-, W²² is - NH-, Rb is C₁₋₆ alkylene optionally substituted with C₁₋₁₀ alkyl, and R²⁰ is C₆₋₁₄ aryl optionally substituted with halogen, heteroaryl, or C₃₋₁₀ cycloalkyl) .

### [Item B4]

The composition of any one of the preceding items, wherein R³ is C₁₋₄ alkyl.

### [Item B5]

The composition of any one of the preceding items, wherein R⁶ is C₁₋₆ alkyl or C₁₋₆ alkenyl.

### [Item B6]

The composition of any one of the preceding items, wherein
Ra is C₁₋₆ alkylene, and
R¹⁰ is naphthyl, quinolinyl, isoquinolinyl, quinoxalinyl, benzothienyl, benzothiazolyl optionally substituted with -NH₂, benzothiadiazolyl, or thienopyridinyl optionally substituted with halogen.

### [Item B7]

The composition of any one of the preceding items, wherein Ra is C₁₋₆ alkylene, and R¹⁰ is naphthyl, quinolinyl, isoquinolinyl, quinoxalinyl, benzothienyl, benzothiazolyl optionally substituted with -NH₂, benzothiadiazolyl, or thienopyridinyl optionally substituted with halogen.

### [Item B8]

The composition of any one of the preceding items, wherein R³ is C₁₋₄ alkyl.

### [Item B9]

The composition of any one of the preceding items, wherein R² is -W²¹-W²²-Rb-R²⁰ (wherein W²¹ is - (CO) -, W²² is - NH-, Rb is C₁₋₆ alkylene, and R²⁰ is C₆₋₁₄ aryl optionally substituted with halogen, or heteroaryl).

### [Item B10]

The composition of any one of the preceding items, wherein
R⁷ is C₆₋₁₄ aryl-C₁₋₁₀ alkyl optionally substituted with a substituent selected from -OH, -OP(=O) (OH)₂, -OP(=O) (ONa)₂, and C₁₋₁₀ alkyl, heteroaryl-C₁₋₁₀ alkyl, or C₃₋₁₀ cycloalkyl-C₁₋₁₀ alkyl,
R¹ is -Ra-R¹⁰ (wherein Ra is C₁₋₆ alkylene, and R¹⁰ is naphthyl, quinolinyl, isoquinolinyl, quinoxalinyl, benzothienyl, benzothiazolyl optionally substituted with - NH₂, benzothiadiazolyl, or thienopyridinyl optionally substituted with halogen),
R³ is C₁₋₄ alkyl, and
R² is -W²¹-W²²-Rb-R²⁰ (wherein W²¹ is - (CO) -, W²² is -NH-, Rb is C₁₋₆ alkylene, and R²⁰ is C₆₋₁₄ aryl optionally substituted with halogen or heteroaryl).

### [Item B11]

The composition of any one of the preceding items, wherein
R⁷ is C₆₋₁₄ aryl-C₁₋₁₀ alkyl optionally substituted with a substituent selected from -OH, -OP(=O) (OH)₂, -OP(=O) (ONa)₂, and C₁₋₁₀ alkyl, heteroaryl-C₁₋₁₀ alkyl, or C₃₋₁₀ cycloalkyl-C₁₋₁₀ alkyl;
R¹ is -Ra-R¹⁰ (wherein Ra is C₁₋₆ alkylene, and R¹⁰ is naphthyl, quinolinyl, isoquinolinyl, quinoxalinyl, benzothienyl, benzothiazolyl optionally substituted with - NH₂, benzothiadiazolyl, or thienopyridinyl optionally substituted with halogen,
R³ is C₁₋₄ alkyl, and
R² is -W²¹-W²²-Rb-R²⁰ (wherein W²¹ is - (CO) -, W²² is -NH-, Rb is C₁₋₆ alkylene, and R²⁰ is C₆₋₁₄ aryl optionally substituted with halogen or heteroaryl).

### [Item B12]

The composition of any one of the preceding items, wherein the compound is selected from
(6S,9S)-N-benzyl-6-(4-hydroxybenzyl)-2,9-dimethyl-8-(naphthalen-1-ylmethyl)-4,7-dioxooctahydro-1H-pyrazino[2,1-c][1,2,4]triazine-1-carboxamide,
(6S,9S)-2-allyl-N-benzyl-6-(4-hydroxybenzyl)-9-methyl-8-(naphthalen-1-ylmethyl)-4,7-dioxooctahydro-1H-pyrazino[2,1-c][1,2,4]triazine-1-carboxamide,
(6S,9S)-N-benzyl-6-(4-hydroxybenzyl)-9-methyl-8-(naphthalen-1-ylmethyl)-4,7-dioxohexahydropyrazino[2,1-c][1,2,4]oxadiazine-1(6H)-carboxamide,
(6S,9S)-8-((2-aminobenzo[d]thiazol-4-yl)methyl)-N-benzyl-6-(4-hydroxybenzyl)-2,9-dimethyl-4,7-dioxooctahydro-1H-pyrazino[2,1-c] [1,2,4]triazine-1-carboxamide,
(6S,9S)-N-benzyl-6-(4-hydroxybenzyl)-2,9-dimethyl-4,7-dioxo-8-(quinolin-8-ylmethyl)octahydro-1H-pyrazino[2,1-c] [1,2,4]triazine-1-carboxamide,
(6S,9S)-2-allyl-N-benzyl-6-(4-hydroxybenzyl)-9-methyl-4,7-dioxo-8-(quinolin-8-ylmethyl)octahydro-1H-pyrazino[2,1-c][1,2,4]triazine-1-carboxamide,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-8-(naphthalen-1-ylmethyl)-4,7-dioxooctahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl sodium phosphate,
4-(((6S,9S)-1-(benzylcarbamoyl)-2,9-dimethyl-4,7-dioxo-8-(naphthalen-8-ylmethyl)octahydro-1H-pyrazino[2,1-c][1,2,4]triazin-6-yl)methyl)phenyl sodium phosphate,
(6S,9S)-2-allyl-6-(4-hydroxybenzyl)-9-methyl-4,7-dioxo-N-((R)-1-phenylethyl)-8-(quinolin-8-ylmethyl)octahydro-1H-pyrazino[2,1-c] [1,2,4]triazine-1-carboxamide,
(6S,9S)-2-allyl-6-(4-hydroxybenzyl)-9-methyl-4,7-dioxo-N-((S)-1-phenylethyl)-8-(quinolin-8-ylmethyl)octahydro-1H-pyrazino[2,1-c] [1,2,4]triazine-1-carboxamide,
(6S,9S)-N-benzyl-6-(4-hydroxy-2,6-dimethylbenzyl)-2,9-dimethyl-4,7-dioxo-8-(quinolin-8-ylmethyl)octahydro-1H-pyrazino[2,1-c] [1,2,4]triazine-1-carboxamide,
(6S,9S)-8-(benzo[b]thiophen-3-ylmethyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-8-(benzo[c][1,2,5]thiadiazol-4-ylmethyl)-N-benzyl-6-(4-hydroxybenzyl)-2,9-dimethyl-4,7-dioxooctahydro-1H-pyrazino[2,1-c] [1,2,4]triazine-1-carboxamide,
(6S,9S)-N-benzyl-6-(4-hydroxybenzyl)-8-(isoquinolin-5-ylmethyl)-2,9-dimethyl-4,7-dioxooctahydro-1H-pyrazino[2,1-c] [1,2,4]triazine-1-carboxamide,
(6S,9S)-N-benzyl-8-((5-chlorothieno[3,2-b]pyridin-3-yl)methyl)-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-2,9-dimethyl-4,7-dioxo-8-(quinoxalin-5-ylmethyl)octahydro-1H-pyrazino[2,1-c] [1,2,4]triazine-1-carboxamide,
(6S,9S)-6-(4-hydroxybenzyl)-2,9-dimethyl-4,7-dioxo-8-(quinolin-8-ylmethyl)-N-(thiophen-2-ylmethyl)octahydro-1H-pyrazino[2,1-c] [1,2,4]triazine-1-carboxamide,
(6S,9S)-N-benzyl-6-(4-hydroxybenzyl)-2,9-dimethyl-4,7-dioxo-8-(quinolin-8-ylmethyl)hexahydro-2H-pyrazino[2,1-c] [1,2,4]triazine-1(6H)-carboxamide (C-82), and
4-{[(6S,9S)-1-(benzylcarbamoyl)-2,9-dimethyl-4,7-dioxo-8-(quinolin-8-ylmethyl)-octahydro-1H-pyrazino[2,1-c] [1,2,4]triazin-6-yl]methyl}phenoxy)phosphoric acid (OK-1).

### [Item B13]

The composition of any one of the preceding items, wherein the compound is (6S,9S)-N-benzyl-6-(4-hydroxybenzyl)-2,9-dimethyl-4,7-dioxo-8-(quinolin-8-ylmethyl)octahydro-1H-pyrazino[2,1-c] [1,2,4]triazine-1-carboxamide.

### [Item B14]

The composition of any one of the preceding items, wherein the compound is 4-(((6S,9S)-1-(benzylcarbamoyl)-2,9-dimethyl-4,7-dioxo-8-(quinolin-8-ylmethyl)octahydro-1H-pyrazino[2,1-c] [1,2,4]triazin-6-yl)methyl)phenyl dihydrogen phosphate.

### [Item B15]

The composition of any one of the preceding items, wherein
R⁷ is C₆₋₁₄ aryl-C₁₋₁₀ alkyl optionally substituted with a substituent selected from -OH, -OP (=O) (OH)₂, -OP (=O) (ONa)₂, and C₁₋₁₀ alkyl,
G is -NR⁶- or -O- (wherein R⁶ is independently selected from C₁₋₆ alkyl and C₁₋₆ alkenyl),
R¹ is -Ra-R¹⁰ (wherein Ra is C₁₋₆ alkylene, and R¹⁰ is naphthyl or a fused bicyclic heteroaryl optionally substituted with a substituent selected from -NH₂ and halogen),
R² is -W²¹-W²²-Rb-R²⁰ (wherein W²¹ is - (CO) -, W²² is -NH-, Rb is C₁₋₆ alkylene optionally substituted with C₁₋₁₀ alkyl, and R²⁰ is C₆₋₁₄ aryl optionally substituted with halogen or heteroaryl), and
R³ is C₁₋₆ alkyl.

### [Item BA1]

Use in the manufacture of a medicament for modulating, slowing the progression of, preventing, or treating a sarcopenia related disease, disorder, or symptom, comprising a compound or a pharmaceutically acceptable salt thereof, or a solvate thereof, wherein the compound is a compound represented by formula (I) 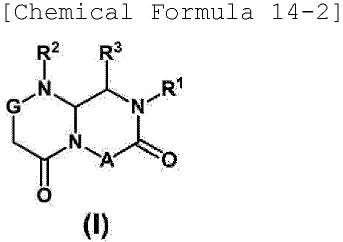 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O) (OH)₂, -OP(=O) (ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen),
R² is -W²¹-W²²-Rb-R²⁰ (wherein W²¹ is - (CO) -, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon.

### [Item BA2]

The use of item BA1, comprising one or more features of any one or more of the preceding items.

### [Item BB1]

A method for modulating, slowing the progression of, preventing, or treating a sarcopenia related disease, disorder, or symptom in a subject, comprising administering to the subject an effective amount of a compound or a pharmaceutically acceptable salt thereof, or a solvate thereof, wherein the compound is a compound represented by formula (I) 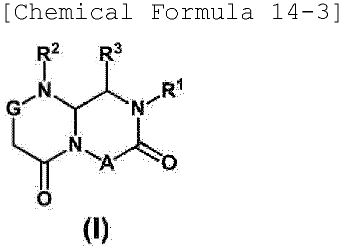 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen,
R² is -W²¹-W²²-Rb-R²⁰ (wherein W²¹ is - (CO) -, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon.

### [Item BB2]

The method of item BB1, comprising one or more features of any one or more of the preceding items.

### [Item BC1]

A compound or a pharmaceutically acceptable salt thereof, or a solvate thereof, for modulating, slowing the progression of, preventing, or treating a sarcopenia related disease, disorder, or symptom, wherein the compound is a compound represented by formula (I) 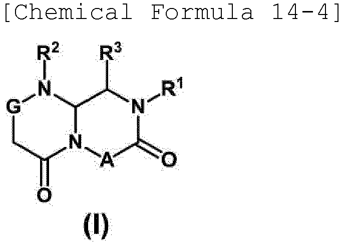 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen),
R² is -W²¹-W²²-Rb-R²⁰ (wherein W²¹ is -(CO)-, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon.

### [Item BC2]

The compound or pharmaceutically acceptable salt thereof, or solvate thereof of item BC1, comprising one or more features of any one or more of the preceding items.

### [Item ZZ]

The composition, method, use, medicament, compound or pharmaceutically acceptable salt thereof, or solvate thereof of any one of the preceding items, wherein the compound, substance, or agent comprises
OK-1 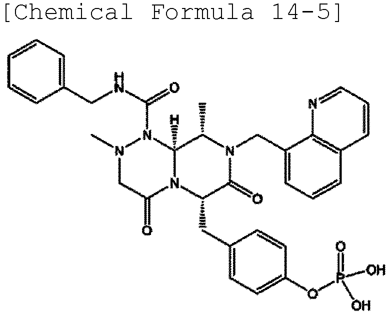 or
C-82 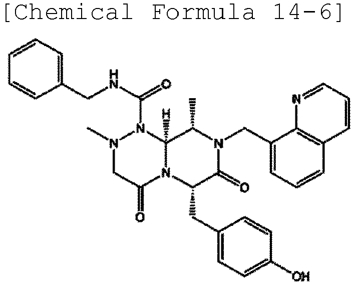 or a pharmaceutically acceptable salt thereof, or a solvate thereof.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

The present disclosure provides a technology that can treat or prevent sarcopenia and related diseases, disorders, and symptoms for the first time. The present disclosure also provides a technology that can treat or prevent senescence related diseases, disorders, and symptoms for the first time.

### [Brief Description of Drawings]

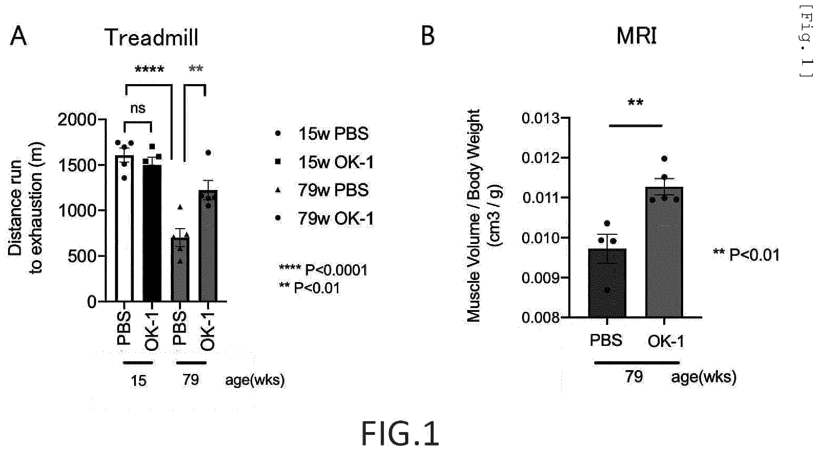

[Figure 1] Figure 1 is a diagram showing improvement in sarcopenia from administration of OK-1. A measures endurance (muscle strength) of young mice (15-week old) and old mice (79-week old) by using a treadmill. The effect of administering PBS (control) or OK-1 for three months is also studied. B is a diagram quantifying the muscle mass using MRI after having old mice administered with PBS (control) or OK-1 to exercise for 3 weeks on a treadmill.

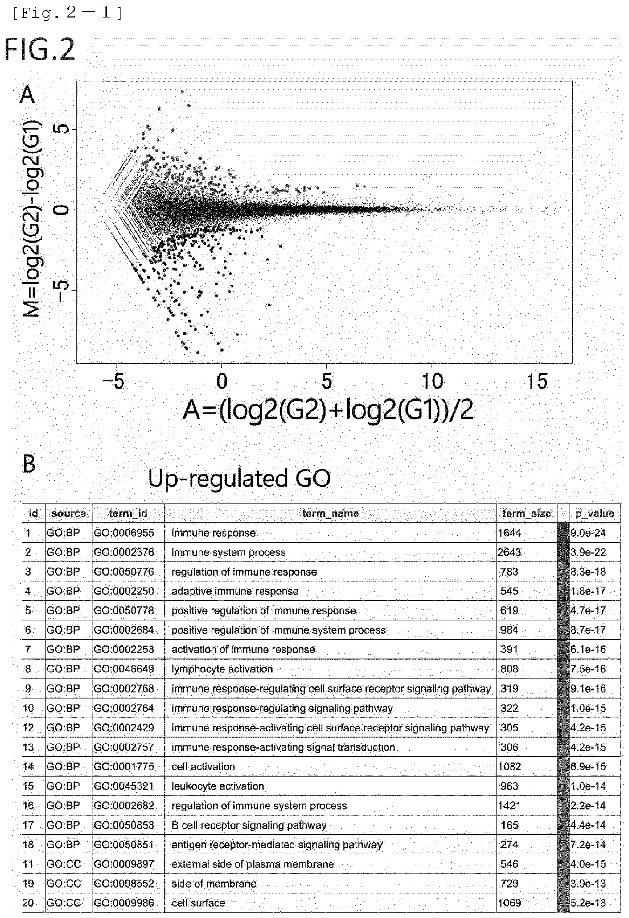

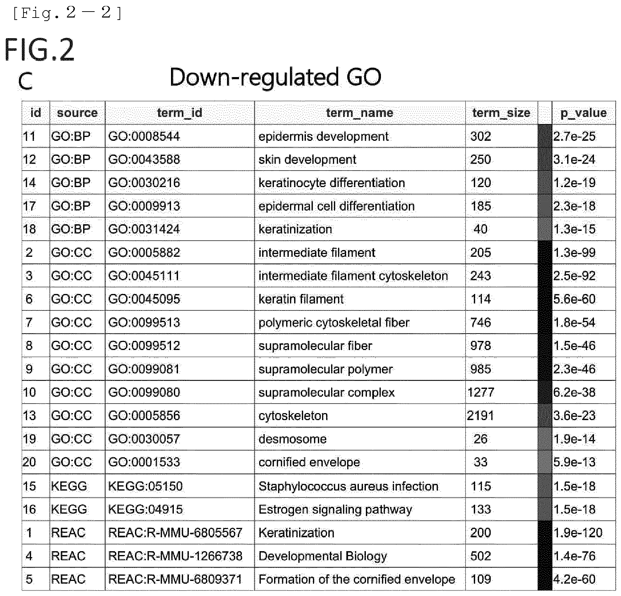

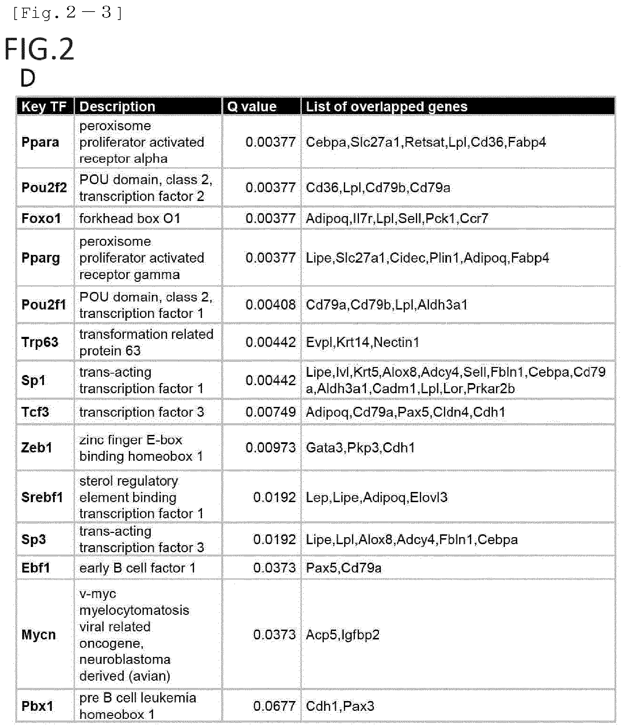

[Figure 2-1] Figure **2** is a diagram showing gene modulation in the muscle of mice administered with OK-1 with no exercise. (A) shows genes (206 genes) with upregulated expression and genes (445 genes) with downregulated expression due to OK-1 administration (p < 0.05). (B) shows a pathway analysis diagram of genes with upregulated expression.
[Figure 2-2] Figure **2** is a diagram showing gene modulation in the muscle of mice administered with OK-1 without exercise. (C) shows a pathway analysis diagram of genes with downregulated expression.
[Figure 2-3] Figure **2** is a diagram showing gene modulation in the muscle of mice administered with OK-1 without exercise. (D) shows an upstream transcription factor analysis diagram of genes observed to have a change in gene expression.
[Figure 3] Figure **3** is a diagram analyzing the change in gene expression of muscles in mice administered with OK-1 without exercise by GSEA analysis. (A) shows pathways with upregulated gene expression in the muscle by OK-1 administration. (B) shows pathways with downregulated gene expression in the muscle by OK-1 administration.
[Figure 4-1] Figure **4** is a diagram showing the top 50 genes with a change in gene expression in the muscle of mice administered with OK-1 without exercise.
[Figure 4-2] Figure **4** is a diagram showing the top 50 genes with a change in gene expression in the muscle of mice administered with OK-1 without exercise.
[Figure 5-1] Figure **5** is a diagram showing gene regulation in the muscle of mice administered with OK-1 with exercise. (A) shows genes (145 genes) with upregulated expression and genes (631 genes) with downregulated expression due to OK-1 administration (p < 0.05). (B) shows a pathway analysis diagram of genes with upregulated expression.
[Figure 5-2] Figure **5** is a diagram showing gene regulation in the muscle of mice administered with OK-1 with exercise. (C) shows a pathway analysis diagram of genes with downregulated expression. (D) shows an upstream transcription factor analysis diagram of genes observed to have a change in gene expression.
[Figure 6] Figure **6** is a diagram from analyzing the change in gene expression in muscles of mice administered with OK-1 with exercise by GSEA analysis. (A) shows pathways with upregulated gene expression in the muscle by OK-1 administration. (B) shows pathways with downregulated gene expression in the muscle by OK-1 administration.
[Figure 7-1] Figure **7** is a diagram showing the top 50 genes with a change in gene expression in the muscle of mice administered with OK-1 with exercise.
[Figure 7-2] Figure **7** is a diagram showing the top 50 genes with a change in gene expression in the muscle of mice administered with OK-1 with exercise.
[Figure 8-1] Figure **8** is a diagram showing a list of related diseases registered in MedlinePlus correlated with a gene with a change in expression by OK-1 treatment.
[Figure 8-2] Figure **8** is a diagram showing a list of related diseases registered in MedlinePlus correlated with a gene with a change in expression by OK-1 treatment.
[Figure 9] Figure **7** is a diagram showing the top 50 genes with a change in gene expression in the muscle of mice administered with OK-1 with exercise.
[Figure 10A] Figure **10A** is a diagram showing that 581 genes increase and 611 genes decrease significantly in myoblasts by treatment with C-82.
[Figure 10B] Figure **10B** is a diagram showing the top 30 genes with a change in gene expression by treatment with C-82.
[Figure 10C] Figure **10C** is a diagram showing categorization of biological functions (GO) with which a gene with upregulated gene expression by treatment with C-82 is involved.
[Figure 10D] Figure **10D** is a diagram showing categorization of biological functions (GO) with which a gene with downregulated gene expression by treatment with C-82 is involved.
[Figure 10E] Figure **10E** is a diagram showing the ranking of upstream transcription factors of genes with a change in expression by treatment with C-82.
[Figure 11] Figure **11** shows the change in muscle strength (endurance) and grip strength of mice administered with C-82. The muscle strength (endurance) increased significantly in mice administered with C-82 compared to mice without administration thereof. While a significant difference was not observed in grip strength, a tendency of a concentration dependent increase was observed.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", etc. in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence

The definitions of the terms and/or the detailed basic technology that are particularly used herein are described hereinafter as appropriate.

### (Definition of the terms)

As used herein, "sarcopenia" refers to a disease that is diagnosed when satisfying, in addition to 1. evidence supporting low muscle mass, 2. low muscle strength, or 3. low physical function (European Working Group on Sarcopenia in Older People (EWGSOP)). Since the skeletal structures of Americans/Europeans differ from those of Asians, a diagnostic standard unique to Asians that is also compatible with the physique of Japanese was established in 2014 by AWGS (ASIAN working Group FOR SARCOPENIA) (Sportsmedicine 27(9), 2-11, 2015-11, Book House HD). Sarcopenia is classified into "primary sarcopenia" caused by aging and "secondary sarcopenia" caused by factors other than aging. Besides aging, sarcopenia is induced by daily activity, disease, or nutritional state, including those due to disuse caused by bedridden life or reduced activity, those due to diseases such as cancer, ischemic heart failure, terminal renal failure, or endocrine disease, those due to malabsorption, anorexia from a gastrointestinal disease or side effect of a drug, or insufficient intake of energy/protein, neurodegeneration induced decrease or loss of activity (e.g., ALS), etc. Sarcopenia refers to "reduced muscle strength" of the entire body, such as grip strength or leg muscle/trunk muscle from reduced muscle mass due to aging or a disease. This results in reduced physical function such as slower gait speed or need for a cane or handrail.

Muscles have a function of "muscle strength" as well as an element of "energy metabolism" and "secretory organ". If the muscle mass decreases or muscular function deteriorates, the function of an organ such as the heart or respiratory organ decreases, from the viewpoint of "muscle strength", so that a load is placed on bones at a joint, resulting in arthritis. From the viewpoint of "energy metabolism", fat and sugar in blood would not be usable by reduced muscles, resulting in obesity. Further, the inability to metabolize fat leads to a metabolic disease such as myocardial infarction. In addition, NAMPT synthesizes NAD from NMN in muscles. A decrease in NAD results in reduced brain function, metabolic function, etc. From the viewpoint of "secretory organ", muscles secrete inflammatory substances such as IL-6 and growth factors such as insulin-like growth factor 1 (IGF-1) and BDNF, which have an effect on cancer or bone, nerve, or blood cell growth (see Hoffmann C, Weigert C. Skeletal Muscle as an Endocrine Organ: The Role of Myokines in Exercise Adaptations. Cold Spring Harb Perspect Med. 2017; 7(11): a029793. Published 2017 Nov 1. doi:10.1101/cshperspect.a029793) The importance of sarcopenia treatment is not only in recovery of muscle strength. It is understood that the treatment can be administered from the viewpoint of metabolism or systemic senescence and recovery of muscle a secretory organ. For this reason, sarcopenia patients can be considered as having broadly developed diseases.

The muscle mass is maintained by repeatedly synthesizing and decomposing muscle proteins. The muscle mass decreases when a decrease in agents required for the synthesis of muscle proteins or decomposition of muscle proteins exceeds the synthesis of muscle proteins. Sarcopenia develops by aging, together with factors such as decrease in sex hormone related to increase in muscles/death of cells required for functioning of muscles (apoptosis)/mitochondrial dysfunction, or atrophy (cachexia) due to a wasting disease such as diabetes or disuse/undernutrition/ cancer. Sarcopenia also results from loss of a motor nerve that serves the role of transmitting an instruction from the brain to a muscle or an effect of hormones related to the increase in muscles such as corticosteroid/growth hormone (GH)/insulin-like growth factor 1 (IGF-1)/thyroid dysfunction/insulin resistance. It is understood that inflammatory cytokines increase by developing each disease and decomposition of muscle proteins progresses, which leads to the development of sarcopenia.

As used herein, "sarcopenia related disease, disorder, or symptom" refers to sarcopenia or a disease, disorder, or symptom associated with one or more of the features of sarcopenia, i.e., muscle strength disorder, energy metabolism disorder, and secretory organ disorder. A sarcopenia related disease, disorder, or symptom can be discussed separately for a disease, disorder, or symptom due to a muscle strength disorder; a disease, disorder, or symptom due to an energy metabolism disorder; and a disease, disorder, or symptom due to a secretory disorder (although there are diseases, disorders, or symptoms that fall under two or more thereof), which are considered to a comprehensively expressed. A sarcopenia related disease, disorder, or symptom can also be discussed as primary sarcopenia, secondary sarcopenia, and a disease, disorder, or symptom accompanied therewith. Examples of a sarcopenia related disease, disorder, or symptom include, but are not limited to, sarcopenia related lifestyle diseases (non-wasting diseases), wasting diseases, tumor associated diseases (including cancer cachexia), motor diseases, neurodegenerative diseases (including ALS), cognitive dysfunction, other associated disorders (defined as including undernutrition, frailty, spinal cord injury, inactivity, reduced activity, disuse syndrome, trauma, and invasive surgery, etc.), etc.

As used herein, "senescence related disease, disorder, or symptom" and "aging related disease, disorder, or symptom" are interchangeably used, referring to a disease, disorder, or symptom that develops in connection with aging. Examples of a senescence related disease, disorder, or symptom include sarcopenia, a lifestyle disease (non-wasting disease), a wasting disease, a motor disease, a neurodegenerative disease, cognitive dysfunction, and other associated disorders. More specific examples include sarcopenia, dementia, a metabolic disease, rheumatoid arthritis, osteoporosis, reduced muscle mass, etc.

As used herein, "disease, disorder, or symptom due to a muscle strength disorder" refers to any disease, disorder, or symptom associated with a decrease in the quantity or quality (muscle function) of muscle. Examples thereof from the viewpoint of "muscle" include a disease, disorder, or symptom from a decrease in the function of an organ such as the heart or respiratory organ, so that a load is placed on bones at a joint, resulting in arthritis, etc. Examples of a disease, disorder, or symptom due to a muscle strength disorder include neurogenic muscular atrophy, myogenic muscular atrophy (muscular dystrophy, congenital myopathy, mitochondrial encephalomyopathy, myopathy due to congenital metabolic disorder, etc.), dysphagia, chronic respiratory failure, excretory disorder (urinary incontinence, fecal incontinence, etc.), essential hypotension, farsightedness, ptosis, etc.

As used herein, "disease, disorder, or symptom due to an energy metabolism disorder" refers to any disease, disorder, or symptom due to an energy metabolism disorder. Fat and sugar in blood would not be usable due to reduced muscles, resulting in obesity, or the inability to metabolize fat leads to a metabolic disease such as myocardial infarction. Meanwhile, NAMPT synthesizes NAD from NMN in muscles. A decrease in NAD results in reduced brain function, metabolic function, etc. Thus, any disease due to such a function can be a disease, disorder, or symptom due to an energy metabolism disorder in relation to sarcopenia. Examples of a disease, disorder, or symptom due to an energy metabolism disorder include diabetes, dyslipidemia, obesity, metabolic syndrome, osteoporosis, fatty liver, hyperuricemia, hypertension, etc.

As used herein, "disease, disorder, or symptom due to a secretory disorder" refers to any disease, disorder, or symptom associated with a disorder of a muscle as a secretory organ secreted or produced from the muscle. Muscles secrete inflammatory substances such as IL-6 and growth factors such as insulin-like growth factor 1 (IGF-1) and BDNF, which have an effect on cancer or bone, nerve, or blood cell growth (Hoffmann C, Weigert C. Skeletal Muscle as an Endocrine Organ: The Role of Myokines in Exercise Adaptations. Cold Spring Harb Perspect Med. 2017; 7(11):a029793. Published 2017 Nov 1. doi:10.1101/cshperspect.a029793 = https://www.ncbi.nlm.nih.gov/pmc/articles/PMC5666622/) . Such an impairment of a function as a secretory organ results in a disease, disorder, or symptom. A disease, disorder, or symptom due to a secretory disorder encompasses diseases, disorders, or symptoms associated therewith. Examples of a disease, disorder, or symptom due to a secretory disorder include diabetes, pancreatitis, ulcerative colitis, diarrhea, etc.

As used herein, "disease, disorder, or symptom due to a nervous system disorder" refers to any disease, disorder, or symptom associated with a disorder of a nerve associated with a muscle. Muscles contract by a signal of a nervous system. Thus, muscles and nerves have a close relationship. Impairment of such a function as a nervous system results in a disease, disorder, or symptom. A disease, disorder, or symptom due to a secretory disorder encompasses diseases, disorders, or symptoms associated therewith. Examples of a disease, disorder, or symptom due to a nervous system disorder include a neurodegenerative disease (including ALS), cognitive dysfunction, etc.

As used herein, "primary sarcopenia" refers to sarcopenia with no clear cause other than aging.

As used herein, "secondary sarcopenia" refers to sarcopenia with a cause other than aging. Examples thereof include sarcopenia associated with activity (can be caused by bedridden, inactive life style or zero-gravity state), sarcopenia associated with a disease (accompanying severe organ failure (heart, lung, liver, kidney, or brain), inflammatory disease, malignant tumor, or endocrine disease), sarcopenia associated with nutrition (due to insufficient ability to ingest energy and/or protein accompanying malabsorption, digestive tract disease, and use of a drug causing anorexia, use of a drug resulting in anorexia), etc.

As used herein, "OK-1" is 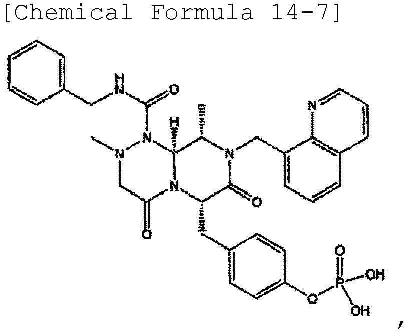 and
the chemical name thereof is 4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid, and is also known as 4-(((6S,9S)-1-(benzylcarbamoyl)-2,9-dimethyl-4,7-dioxo-8-(quinolin-8-ylmethyl)octahydro-2H-pyrazino[2,1-c][1,2,4]triazin-6-yl)methyl)phenyl dihydrogen phosphate.

As used herein, "C-82" is 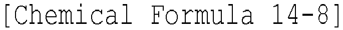 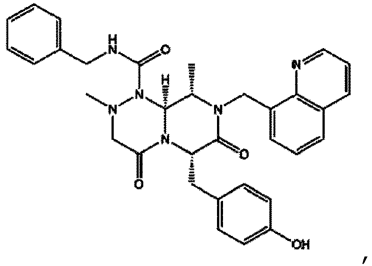 and
the chemical name thereof is (6S,9S)-N-benzyl-6-(4-hydroxybenzyl)-2,9-dimethyl-4,7-dioxo-8-(quinolin-8-ylmethyl)hexahydro-2H-pyrazino[2,1-c] [1,2,4]triazine-1(6H)-carboxamide.

As used herein, "OK-1 related compound group" refers to compounds associated with compound groups [1] to [9], pharmaceutically acceptable salts thereof, or solvates thereof, and is defined elsewhere herein.

It is understood that a compound mentioned herein also encompasses enantiomers thereof.

As used herein, "pharmaceutically acceptable salt" refers to an acid addition salt or base addition salt which is pharmaceutically acceptable for use. Specific examples of "pharmaceutically acceptable salts" include, but are not limited to, acid addition salts such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, para-toluenesulfonate (tosylate), laurylsulfate, malate, ascorbate, mandelate, saccharinate, xinafoate, pamoate, cinnamate, adipate, cysteine salt, N-acetyl cysteine salt, hydrochloride, hydrobromide, phosphate, sulfate, hydroiodide, nicotinate, oxalate, picrate, thiocyanate, undecanoate, acrylic acid polymer salt, and carboxyvinyl polymer; inorganic base addition salts such as lithium salt, sodium salt, potassium salt, and calcium salt; organic base addition salts such as morpholine and piperidine; amino acid addition salts such as aspartic acid and glutamic acid; etc.

As used herein, the term "solvate" refers to a compound further comprising a stoichiometric or non-stoichiometric amount of compound bound by a non-covalent intramolecular force. If the solvent is water, the solvate is hydrate.

As used herein, the term "stereoisomer" encompasses any enantiomerically/stereoisomerically pure compound and enantiomerically/stereoisomerically concentrated compound of the compounds provided herein. An enantiomer refers to a stereoisomer of a compound having one chiral center. It is understood that the compound of the present disclosure can encompass any corresponding stereoisomer.

Prodrugs of the compounds of the present disclosure are also encompassed within the scope of the present disclosure. As used herein, a prodrug refers to a derivative that provides a compound described herein in vivo by, for example, acid hydrolysis or enzymatic degradation. For example, if a compound described herein has a hydroxyl group, amino group, or carboxyl group, these groups can be modified in accordance with a conventional method to manufacture a prodrug. Prodrug technologies are described in, for example, C. G. Wermuth, "The Practice of Medicinal Chemistry", 4th Ed., Academic Press, (2015), Chapter 28. Thus, the compound of the present disclosure, etc. can be provided as a prodrug, and the composition of the present disclosure can be in a form comprising a prodrug of the compound of the present disclosure, etc.

The term "prodrug" is intended to encompass compounds that are converted into the therapy activation agent (e.g., compound of formula I) of the present disclosure under physiological conditions. A general method for preparing a prodrug is a method of including one or more selected portions for exposing a desired molecule by hydrolysis under physiological conditions. In another embodiment, a prodrug is converted by enzymatic activity of a host animal. For example, esters and carbonate (e.g., alcohol or carboxylic acid ester or carbonate) are preferred prodrugs of the present disclosure. In a specific embodiment, a portion of the formulations presented above or the compounds of the present disclosure can be replaced with a suitable corresponding prodrug, wherein, for example, hydroxyl in the parent compound is given as an ester or carbonate, or carboxylic acid in the parent compound is given as an ester.

As used herein, "physical therapy" refers to therapy through exercise. Intervention in sarcopenia other than through drugs are considered to be exercise and dietary therapy using Vitamin D, omega 3, etc.

As used herein, "dietary therapy" refers to therapy through diet. Intervention in sarcopenia other than through drugs are considered to be exercise and dietary therapy using Vitamin D, omega 3, etc.

As used herein, "other drug therapy" refers to drug therapy using a compound other than the "OK-1 related compound" of the present disclosure. Use of a plurality of OK-1 related compound groups is not referred to as other drug therapy and falls under use of a plurality of the compounds, etc. of the present disclosure.

As used herein, "prevention" is an act of administering an active ingredient of the present disclosure to a healthy individual who has not developed a disease in order to, for example, inhibit the onset of the disease.

As used herein, "treatment" is an act of administering an active ingredient of the present disclosure to a person (patient) diagnosed as having developed a disease by a physician.

As used herein, "modulation" refers to a change, preferably improvement, in the condition of a disease, etc. of a subject who has developed the disease (e.g., sarcopenia related disease, disorder, or symptom) when the compound of the present disclosure is administered compared to when the compound is not administered.

As used herein, "slowing of progression" refers to no exacerbation, preferably improvement in the degree of a disease, etc. of a subject who has developed the disease (e.g., sarcopenia related disease, disorder, or symptom).

As used herein, "kit" refers to a unit which are generally separated into two or more segments for providing parts to be provided (e.g., compound, pharmaceutical product, composition, etc., and user manual, etc.). Such a kit form is preferred when providing a composition, which should not be provided in a mixed state for stability, etc. and is preferably used by mixing immediately prior to use. Alternatively, such a kit is preferable when provided as a combination with an agent for identifying a patient in advance as a companion drug. Such a kit advantageously comprises an instruction or user manual describing how provided portions are used, or how a reagent or waste fluid after use should be processed. When a kit is used as a pharmaceutical product kit herein, the kit generally comprises an instruction, etc. describing the method of administering the pharmaceutical product, etc.

As used herein, "instruction" is a document with an explanation of the method of use of the present disclosure for users. The instruction has a description instructing the method of use (e.g., administration) of the compound, etc. of the present disclosure. The instruction is prepared in accordance with a format specified by the regulatory agency of the country in which the present disclosure is practiced (e.g., the Ministry of Health, Labour and Welfare, Ministry of Agriculture, Forestry and Fisheries, etc. in Japan, Food and Drug Administration (FDA) or U.S. Department of Agriculture (USDA) in the U.S., etc.), with an explicit description showing approval by the regulatory agency as needed. The instruction can be provided in, but not limited to, paper media. The instructions can also be provided in a form such as electronic media (e.g., web sites provided on the Internet or emails).

### (Explanation of compounds)

The OK-1 related compound groups used in the present disclosure include compound group [1] to [9] described below. Although not wishing to be bound by any theory, compound groups [2] to [9] are compounds developed to have the same function as compound group [1]. Those skilled in the art understand that said compound groups have the same function as compound group [1] represented by OK-1 in the present disclosure.

### Compound group [1]

A compound or a pharmaceutically acceptable salt thereof, or a solvate thereof, used in one embodiment of the present disclosure can be compound group [1]. Compound group [1] is described in Japanese Patent No. 5530427, and the content thereof is incorporated herein by reference. For example, the compound can be a compound represented by formula (I) 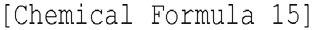 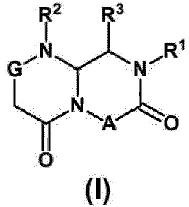 wherein
A is -CHR⁷- (wherein R⁷ is hydrogen, optionally substituted alkyl, optionally substituted alkenyl, optionally substituted alkynyl, optionally substituted arylalkyl, optionally substituted heteroarylalkyl, optionally substituted cycloalkylalkyl, optionally substituted heterocycloalkylalkyl, optionally substituted aryl, optionally substituted heteroaryl, optionally substituted cycloalkyl, or optionally substituted heterocycloalkyl),
G is -NH-, -NR⁶-, -O-, -CHR⁶-, or -C(R⁶)₂- (wherein R⁶ is independently selected from optionally substituted alkyl, optionally substituted alkenyl, and optionally substituted alkynyl),
R¹ is optionally substituted arylalkyl, optionally substituted heteroarylalkyl, optionally substituted cycloalkylalkyl, or optionally substituted heterocycloalkylalkyl,
R² is -W²¹-W²²-Rb-R²⁰ (wherein
   W²¹ is -(CO)- or -(SO₂)-,
   W²² is a bond, -O-, -NH-, or optionally substituted lower alkylene,
   Rb is a bond or optionally substituted lower alkylene, and
   R²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R³ is optionally substituted alkyl, optionally substituted alkenyl, or optionally substituted alkynyl, or a pharmaceutically acceptable salt thereof, or a solvate thereof.

In one embodiment, A is -CHR⁷- (wherein R⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl).

Examples of an optionally substituted alkyl group include methyl, ethyl, propyl, isopropyl, butyl, isobutyl, sec-butyl, tert-butyl, aminomethyl, aminoethyl, aminopropyl, aminobutyl, carboxymethyl, carboxyethyl, carboxypropyl, carboxybutyl, carbamoylmethyl, carbamoylethyl, carbamoylpropyl, carbamoylbutyl, methoxymethyl, methoxyethyl, methoxypropyl, methoxybutyl, methylthiomethyl, methylthioethyl, methylthiopropyl, methylthiobutyl, hydroxymethyl, hydroxyethyl, hydroxypropyl, hydroxybutyl, etc.

Examples of alkenyl group include ethenyl, allyl, 1-propenyl, 2-methylallyl, etc.

Examples of alkynyl group include 1-propynyl, ethynyl, etc.

Examples of aryl and heteroaryl include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Examples of cycloalkyl and optionally substituted heterocycloalkyl include cyclopropyl, cyclobutyl, cyclopentyl, cyclohexyl, cycloheptyl, adamantyl, etc.

In another embodiment, A is -CHR⁷- (wherein R⁷ is optionally substituted arylalkyl, optionally substituted heteroarylalkyl, optionally substituted cycloalkylalkyl, optionally substituted heterocycloalkylalkyl, optionally substituted aryl, optionally substituted heteroaryl, optionally substituted cycloalkyl, or optionally substituted heterocycloalkyl, each of which is represented by formula - Rc-R⁷⁰ (wherein Rc is a bond or optionally substituted lower alkylene, and R⁷⁰ is optionally substituted aryl, optionally substituted heteroaryl, optionally substituted cycloalkyl, or optionally substituted heterocycloalkyl)).

Examples of a lower alkylene group include methylene, ethylene, methylmethylene, 1,2-propylene, 1,3-propylene, 1,2-butylene, 1,3-butylene, 1,4-butylene, 1,2,3-propanetriyl, 1,3,3-propanetriyl, etc.

Examples of aryl group and heteroaryl group include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Examples of cycloalkyl group and optionally substituted heterocycloalkyl group include cyclopropyl, cyclobutyl, cyclopentyl, cyclohexyl, cycloheptyl, adamantyl, etc.

In a specific embodiment of formula (I), R⁷⁰ in the aforementioned embodiment is optionally substituted aryl or optionally substituted heteroaryl.

Examples of aryl group and heteroaryl group include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Preferred examples of aryl group and heteroaryl group include phenyl, pyridyl, pyrimidyl, pyridazinyl, pyrazinyl, triazinyl, pyrrolyl, thienyl, furyl, thiazolyl, oxazolyl, imidazolyl, benzothienyl, etc.

The most preferred examples of allyl group include phenyl, etc.

Examples of substituents for R⁷ include -R⁸, -OH, -OR⁸, -OC(O)R⁸, -OC(O)OR⁸, -COOH, -COOR⁸, -CONH₂, -CONHR⁸, -CONR⁸R⁴, -NH₂, -NHR⁸, -NR⁸R⁴, -SH, -SR⁸, -SO₂R⁸, -SO₂NH₂, -SO₂NHR⁸, - SO₂NR⁸R⁴, -SO₃H, -SOR⁸, -NHC(NH₂)(=NH), -NHC(NHR⁸)(=NR⁴), - OP(=O)(OH)₂, -OP(=O)(ONa) ₂, -OP(=O)(OR⁸)₂, -OP(=O)(OR⁸)(OH), -OP(=O)(OH)-O-P(=O)(OH) ₂, -OP(=O)(ONa)-O-OP(=O)(ONa)₂, -CN,-NO₂, and halogen (wherein R⁸ and R⁴ are independently selected from linear or branched, cyclic or non-cyclic, substituted or unsubstituted alkyl chain, aryl, and arylalkyl moiety).

Preferred examples of such substituents include -OH, - COOH, -OC(O)R⁸, -OC(O)OR⁸, -NH₂, -SH, -SO₃H, -SOR⁸, - OP(=O)(OH) ₂, -OP(=O)(OR⁸)₂, -OP(=O)(OR⁸)(OH), -OP(=O)(ONa) ₂, - OP(=O)(OH)-O-P(=O)(OH)₂, -OP(=O)(ONa)-O-OP(=O)(ONa)₂, and halogen.

The most preferred examples of such substituents include -OH, -OP(=O)(OH)₂, -OP(=O)(ONa)₂, and halogen.

In one embodiment, G is -NH-, -NR⁶-, -O-, -CHR⁶-, or - C(R⁶)₂- (wherein R⁶ is independently selected from optionally substituted alkyl, optionally substituted alkenyl, and optionally substituted alkynyl).

G is preferably -NH-, -NR⁶-, or -O-, more preferably - NR⁶- .

Examples of alkyl group include C₁₋₄ alkyl such as methyl, ethyl, propyl, isopropyl, butyl, isobutyl, sec-butyl, and tert-butyl.

Examples of alkenyl group include ethenyl, allyl, 1-propenyl, 2-methylallyl, etc.

Examples of alkynyl group include 1-propynyl, ethynyl, etc.

R⁶ is preferably optionally substituted alkyl or optionally substituted alkenyl, more preferably lower alkyl (e.g., methyl) or lower alkenyl (e.g., allyl).

In one embodiment, R¹ is optionally substituted arylalkyl, optionally substituted heteroarylalkyl, optionally substituted cycloalkylalkyl, or optionally substituted heterocycloalkylalkyl, each of which is formula -Ra-R¹⁰ (wherein Ra is a bond or optionally substituted lower alkylene, and R¹⁰ is optionally substituted aryl, optionally substituted heteroaryl, optionally substituted cycloalkyl, or optionally substituted heterocycloalkyl).

In another embodiment, R¹ is optionally substituted arylalkyl, optionally substituted heteroarylalkyl, optionally substituted cycloalkylalkyl, or optionally substituted heterocycloalkylalkyl, each of which is formula -Ra-R¹⁰ (wherein Ra is a bond or optionally substituted lower alkylene, and R¹⁰ is optionally substituted fused bicyclic aryl or optionally substituted fused bicyclic heteroaryl).

Examples of lower alkylene group include methylene, ethylene, methylmethylene, 1,2-propylene, 1,3-propylene, 1,2-butylene, 1,3-butylene, 1,4-butylene, 1,2,3-propanetriyl, 1,3,3-propanetriyl, etc.

Examples of aryl group and heteroaryl group include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, and imidazopyridinyl.

Examples of cycloalkyl group and heterocycloalkyl group include cyclopropyl, cyclobutyl, cyclopentyl, cyclohexyl, cycloheptyl, adamantyl, etc.

In a specific embodiment of formula (I), Ra in the aforementioned embodiment is optionally substituted lower alkylene, and R¹⁰ is optionally substituted aryl or optionally substituted heteroaryl.

Examples of lower alkylene group include methylene, ethylene, methylmethylene, 1,2-propylene, 1,3-propylene, 1,2-butylene, 1,3-butylene, 1,4-butylene, 1,2,3-propanetriyl, 1,3,3-propanetriyl, etc.

Examples of aryl group and heteroaryl group include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Preferred examples of lower alkylene group include methylene, ethylene, etc.

Preferred examples of aryl group and heteroaryl group include naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, and imidazopyridinyl, and other fused bicyclic aryl groups and fused bicyclic heteroaryl groups.

Examples of substituents for R¹ include -R⁸, -OH, -OR⁸, -COOH, -COOR⁸, -CONH₂, -CONHR⁸, -CONR⁸R⁴, -NH₂, -NHR⁸, -NR⁸R⁴, -SH, -SR⁸, -SO₂R⁸, -SO₂NH₂, -SO₂NHR⁸, -SO₂NR⁸R⁴, -SO₃H, -SOR⁸, - NHC(NH₂)(=NH) , -NHC(NHR⁸)NR⁴, -OP(=O)(OH)₂, -OP(=O)(ONa)₂, - CN, -NO₂, and halogen (wherein R⁸ and R⁴ are independently selected from linear or branched, cyclic or non-cyclic, substituted or unsubstituted alkyl chain, aryl, and arylalkyl moiety).

Preferred examples of the substituents include -NH₂, - OH, -OR⁸, -COOH, -CONH₂, -CONHR⁸, -CONR⁸R⁴, -NHR⁸, -NR⁸R⁴, and halogen. More preferred examples of the substituents include -NH₂, -OH, -COOH, -CONH₂, and halogen.

In one embodiment, R² is -W²¹-W²²-Rb-R²⁰ (wherein W²¹ is - (CO) - or - (SO₂)-; W²² is a bond, -O-, -NH-, or optionally substituted lower alkylene; Rb is a bond or optionally substituted lower alkylene; and R²⁰ is optionally substituted alkyl, optionally substituted alkenyl, optionally substituted alkynyl, optionally substituted aryl, optionally substituted heteroaryl, optionally substituted cycloalkyl, optionally substituted heterocycloalkyl, etc.).

Examples of a lower alkylene group for W²² include methylene, ethylene, propylene, butylene, etc.

Examples of a lower alkylene group for Rb include methylene, ethylene, methylmethylene, 1,2-propylene, 1,3-propylene, 1,2-butylene, 1,3-butylene, 1,4-butylene, 1,2,3-propanetriyl, 1,3,3-propanetriyl, etc.

Examples of optionally substituted alkyl group include methyl, ethyl, propyl, isopropyl, butyl, isobutyl, sec-butyl, tert-butyl, aminomethyl, aminoethyl, aminopropyl, aminobutyl, carboxymethyl, carboxyethyl, carboxypropyl, carboxybutyl, carbamoylmethyl, carbamoylethyl, carbamoylpropyl, carbamoylbutyl, methoxymethyl, methoxyethyl, methoxypropyl, methoxybutyl, methylthiomethyl, methylthioethyl, methylthiopropyl, methylthiobutyl, hydroxymethyl, hydroxyethyl, hydroxypropyl, hydroxybutyl, etc.

Examples of alkenyl group include ethenyl, allyl, 1-propenyl, 2-methylallyl, etc.

Examples of alkynyl group include 1-propynyl, ethynyl, etc.

Examples of aryl group and heteroaryl group include biphenyl, phenyl, pyridyl, pyrimidyl, pyridazinyl, pyrazinyl, triazinyl, pyrrolyl, thienyl (thinyl), furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Examples of cycloalkyl group and heterocycloalkyl group include cyclopropyl, cyclobutyl, cyclopentyl, cyclohexyl, cycloheptyl, adamantyl, etc.

In a specific embodiment of formula (I), R² in the aforementioned embodiments is -W²¹-W²²-Rb-R²⁰, wherein W²¹ is -(CO)-; W²² is -NH-; Rb is optionally substituted lower alkylene, and R²⁰ is optionally substituted aryl or optionally substituted heteroaryl.

Examples of a lower alkylene group for Rb include methylene, ethylene, methylmethylene, 1,2-propylene, 1,3-propylene, 1,2-butylene, 1,3-butylene, 1,4-butylene, 1,2,3-propanetriyl, 1,3,3-propanetriyl, etc.

Examples of aryl group and heteroaryl group include biphenyl, phenyl, pyridyl, pyrimidyl, pyridazinyl, pyrazinyl, triazinyl, pyrrolyl, thienyl, furyl, thiazolyl, oxazolyl, imidazolyl, tetrahydronaphthyl, naphthyl, quinolinyl, isoquinolinyl, quinazolinyl, quinoxalinyl, cinnolinyl, naphthyridinyl, benzotriazinyl, indenyl, pyridopyrimidinyl, pyridopyrazinyl, pyridopyridazinyl, pyridotriazinyl, benzofuryl, benzothienyl, indolyl, indazolyl, benzoxazolyl, benzimidazolyl, benzothiazolyl, benzothiadiazolyl, furopyridinyl, thienopyridinyl, pyropyridinyl, oxazolopyridinyl, thiazolopyridinyl, imidazopyridinyl, etc.

Preferred examples of aryl group and heteroaryl group include phenyl, naphthyl, pyridyl, pyrimidyl, pyridazinyl, pyrazinyl, triazinyl, pyrrolyl, thienyl, furyl, thiazolyl, oxazolyl, and imidazolyl, and other monocyclic aryl groups and monocyclic heteroaryl group.

Examples of substituents for R²⁰ include -R⁸, -OH, -OR⁸, -COOH, -COOR⁸, -CONH₂, -CONHR⁸, -CONR⁸R⁴, -NH₂, -NHR⁸, -NR⁸R⁴, -SH, -SR⁸, -SO₂R⁸, -SO₂NH₂, -SO₂NHR⁸, -SO₂NR⁸R⁴, -SO₃H, -SOR⁸, - NHC(NH₂)(=NH), -NHC(NHR⁸)NR⁴, -OP(=O)(OH)₂, -OP(=O)(ONa)₂, - CN, -NO₂, and halogen (wherein R⁸ and R⁴ are independently selected from linear or branched, cyclic or non-cyclic, substituted or unsubstituted alkyl chain, aryl, and arylalkyl moiety).

Preferred examples of the substituents include -NH₂, - OH, -OR⁸, -COOH, -CONH₂, -CONHR⁸, -CONR⁸R⁴, -NHR⁸, -NR⁸R⁴, and halogen.

In one embodiment, R³ is optionally substituted alkyl, optionally substituted alkenyl, or optionally substituted alkynyl.

Examples of alkyl group include C₁₋₄ alkyl such as methyl, ethyl, propyl, isopropyl, butyl, isobutyl, sec-butyl, and tert-butyl.

Preferred examples of alkyl group include methyl, ethyl, etc.

Examples of alkenyl group include ethenyl, allyl, 1-propenyl, 2-methylallyl, etc.

Examples of alkynyl group include 1-propynyl, ethynyl, etc.

R³ is preferably C₁₋₄ alkyl such as methyl, ethyl, propyl, isopropyl, butyl, isobutyl, sec-butyl, or tert-butyl, more preferably methyl or ethyl.

A compound used in the present disclosure or a pharmaceutically acceptable salt thereof, or a solvate thereof, can be a compound represented by formula (I): 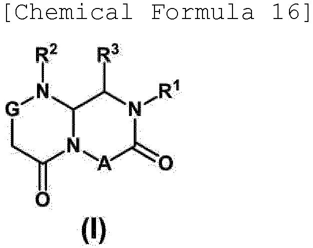 or a pharmaceutically acceptable salt thereof, or a solvate thereof, wherein
A is -CHR⁷-, wherein
   R⁷ is C₁₋₁₀ alkyl optionally substituted with a substituent selected from -OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O-, wherein
   R⁶ is independently selected from C₁₋₁₀ alkyl and C₂₋₁₀ alkenyl,
R¹ is -Ra-R¹⁰, wherein
   Ra is C₁₋₆ alkylene, and
   R¹⁰ is naphthyl or fused bicyclic heteroaryl optionally substituted with a substituent selected from -NH₂ and halogen,
R² is -W²¹-W²²-Rb-R²⁰, wherein
   W²¹ is -(CO)-,
   W²² is -O- or -NH-,
   Rb is a bond or C₁₋₆ alkylene optionally substituted with C₁₋₁₀ alkyl, and
   R²⁰ is C₁₋₁₀ alkyl, C₆₋₁₄ aryl optionally substituted with halogen, heteroaryl, or C₃₋₁₀ cycloalkyl, and
R³ is C₁₋₁₀ alkyl,
wherein heteroaryl refers to a 5- to 14-membered monocyclic or polycyclic aromatic group wherein at least one ring atom is a heteroatom selected from nitrogen, oxygen, and sulfur, and the rest of the ring atoms is carbon.

In one embodiment, A is -CHR⁷-, wherein R⁷ is C₁₋₁₀ alkyl optionally substituted with a substituent selected from -OH, -OR⁸ (wherein R⁸ represents C₁₋₁₀ alkyl), -NH₂, -COOH, and - CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl.

In one embodiment, R⁷ is C₆₋₁₄ aryl-C₁₋₁₀ alkyl optionally substituted with a substituent selected from -OH,-OP(=O)(OH)₂, -OP(=O)(ONa)₂, and C₁₋₁₀ alkyl, heteroaryl-C₁₋₁₀ alkyl, or C₃₋₁₀ cycloalkyl-C₁₋₁₀ alkyl.

In one embodiment, G is -NR⁶- or -O-, wherein R⁶ is independently selected from C₁₋₁₀ alkyl and C₂₋₁₀ alkenyl.

In one embodiment, R¹ is -Ra-R¹⁰, wherein Ra is C₁₋₆ alkylene, and R¹⁰ is naphthyl or fused bicyclic heteroaryl optionally substituted with a substituent selected from -NH₂ and halogen.

In one embodiment, Ra is C₁₋₆ alkylene, and R¹⁰ is naphthyl, quinolinyl, isoquinolinyl, quinoxalinyl, benzothienyl, benzothiazolyl optionally substituted with - NH₂, benzothiadiazolyl, or thienopyridinyl optionally substituted with halogen.

In one embodiment, R² is -W²¹-W²²-Rb-R²⁰, wherein W²¹ is -(CO)-, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl.

In one embodiment, R³ is C₁₋₁₀ alkyl.

In one embodiment, R² is -W²¹-W²²-Rb-R²⁰, wherein W²¹ is -(CO)-, W²² is -NH-, Rb is C₁₋₆ alkylene optionally substituted with C₁₋₁₀ alkyl; and R²⁰ is C₆₋₁₄ aryl optionally substituted with halogen, heteroaryl, or C₃₋₁₀ cycloalkyl.

In one embodiment, R² is -W²¹-W²²-Rb-R²⁰, wherein W²¹ is -(CO) -, W²² is -NH-, Rb is C₁₋₆ alkylene, and R²⁰ is C₆₋₁₄ aryl optionally substituted with halogen or heteroaryl.

In one embodiment, R³ is C₁₋₄ alkyl.

In one embodiment, R⁶ is C₁₋₆ alkyl or C₂₋₆ alkenyl.

In one embodiment, R⁷ is C₆₋₁₄ aryl-C₁₋₁₀ alkyl optionally substituted with a substituent selected from -OH, - OP(=O)(OH)₂, -OP(=O)(ONa)₂, and C₁₋₁₀ alkyl, heteroaryl-C₁₋₁₀ alkyl, or C₃₋₁₀ cycloalkyl-C₁₋₁₀ alkyl;

R¹ is -Ra-R¹⁰, wherein Ra is C₁₋₆ alkylene, and R¹⁰ is naphthyl, quinolinyl, isoquinolinyl, quinoxalinyl, benzothienyl, benzothiazolyl optionally substituted with - NH₂, benzothiadiazolyl, or thienopyridinyl optionally substituted with halogen, R³ is C₁₋₄ alkyl, and R² is -W²¹-W²²-Rb-R²⁰, wherein W²¹ is -(CO)-, W²² is -NH-, Rb is C₁₋₆ alkylene, and R²⁰ is C₆₋₁₄ aryl optionally substituted with halogen or heteroaryl.

In one embodiment, a compound represented by formula (I) is selected from (6S,9S)-N-benzyl-6-(4-hydroxybenzyl)-2,9-dimethyl-8-(naphthalen-1-ylmethyl)-4,7-dioxooctahydro-1H-pyrazino[2,1-c] [1,2,4]triazine-1-carboxamide,
(6S,9S)-2-allyl-N-benzyl-6-(4-hydroxybenzyl)-9-methyl-8-(naphthalen-1-ylmethyl)-4,7-dioxooctahydro-1H-pyrazino[2,1-c][1,2,4]triazine-1-carboxamide,
(6S,9S)-N-benzyl-6-(4-hydroxybenzyl)-9-methyl-8-(naphthalen-1-ylmethyl)-4,7-dioxohexahydropyrazino[2,1-c][1,2,4]oxadiazine-1(6H)-carboxamide,
(6S,9S)-8-((2-aminobenzo[d]thiazol-4-yl)methyl)-N-benzyl-6-(4-hydroxybenzyl)-2,9-dimethyl-4,7-dioxooctahydro-1H-pyrazino[2,1-c] [1,2,4]triazine-1-carboxamide,
(6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide,
(6S,9S)-2-allyl-N-benzyl-6-(4-hydroxybenzyl)-9-methyl-4,7-dioxo-8-(quinolin-8-ylmethyl)octahydro-1H-pyrazino[2,1-c][1,2,4]triazine-1-carboxamide,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-8-(naphthalen-1-ylmethyl)-4,7-dioxooctahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl sodium phosphate,
4-(((6S,9S)-1-(benzylcarbamoyl)-2,9-dimethyl-4,7-dioxo-8-(naphthalen-8-ylmethyl)octahydro-1H-pyrazino[2,1-c][1,2,4]triazin-6-yl)methyl)phenyl sodium phosphate,
(6S,9S)-2-allyl-6-(4-hydroxybenzyl)-9-methyl-4,7-dioxo-N-((R)-1-phenylethyl)-8-(quinolin-8-ylmethyl)octahydro-1H-pyrazino[2,1-c][1,2,4]triazine-1-carboxamide,
(6S,9S)-2-allyl-6-(4-hydroxybenzyl)-9-methyl-4,7-dioxo-N-((S)-1-phenylethyl)-8-(quinolin-8-ylmethyl)octahydro-1H-pyrazino[2,1-c][1,2,4]triazine-1-carboxamide,
(6S,9S)-N-benzyl-6-(4-hydroxy-2,6-dimethylbenzyl)-2,9-dimethyl-4,7-dioxo-8-(quinolin-8-ylmethyl)octahydro-1H-pyrazino[2,1-c][1,2,4]triazine-1-carboxamide,
(6S,9S)-8-(benzo[b]thiophen-3-ylmethyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-8-(benzo[c][1,2,5]thiadiazol-4-ylmethyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-8-(isoquinolin-5-ylmethyl)-2,9-dimethyl-4,7-dioxooctahydro-1H-pyrazino[2,1-c][1,2,4]triazine-1-carboxamide,
(6S,9S)-N-benzyl-8-((5-chlorothieno[3,2-b]pyridin-3-yl)methyl)-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-2,9-dimethyl-4,7-dioxo-8-(quinoxalin-5-ylmethyl)octahydro-1H-pyrazino[2,1-c][1,2,4]triazine-1-carboxamide,
(6S,9S)-6-(4-hydroxybenzyl)-2,9-dimethyl-4,7-dioxo-8-(quinolin-8-ylmethyl)-N-(thiophen-2-ylmethyl)octahydro-1H-pyrazino[2,1-c] [1,2,4]triazine-1-carboxamide,
(6S,9S)-N-benzyl-6-(4-hydroxybenzyl)-2,9-dimethyl-4,7-dioxo-8-(quinolin-8-ylmethyl)hexahydro-2H-pyrazino[2,1-c] [1,2,4]triazine-1(6H)-carboxamide (C-82), and
4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid (OK-1).

In one embodiment, a compound represented by formula (I) is (6S,9S)-N-benzyl-6-(4-hydroxybenzyl)-2,9-dimethyl-4,7-dioxo-8-(quinolin-8-ylmethyl)octahydro-1H-pyrazino[2,1-c] [1,2,4]triazine-1-carboxamide. In one embodiment, a compound represented by formula (I) is (6S,9S)-N-benzyl-6-(4-hydroxybenzyl)-2,9-dimethyl-4,7-dioxo-8-(quinolin-8-ylmethyl)hexahydro-2H-pyrazino[2,1-c] [1,2,4]triazine-1(6H)-carboxamide.

In one embodiment, a compound represented by formula (I) is 4-(((6S,9S)-1-(benzylcarbamoyl)-2,9-dimethyl-4,7-dioxo-8-(quinolin-8-ylmethyl)octahydro-1H-pyrazino[2,1-c] [1,2,4]triazin-6-yl)methyl)phenyl dihydrogen phosphate. In one embodiment, a compound represented by formula (I) is 4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid.

In one embodiment, R⁷ is C₆₋₁₄ aryl-C₁₋₁₀ alkyl optionally substituted with a substituent selected from -OH, - OP(=O)(OH)₂, -OP(=O)(ONa) ₂, and C₁₋₁₀ alkyl, G is -NR⁶- or - O-, R⁶ is independently selected from C₁₋₆ alkyl and C₂₋₆ alkenyl, R¹ is -Ra-R¹⁰, wherein Ra is C₁₋₆ alkylene, and R¹⁰ is naphthyl or fused bicyclic heteroaryl optionally substituted with a substituent selected from -NH₂ and halogen, R² is -W²¹-W²²-Rb-R²⁰, wherein W²¹ is -(CO)-, W²² is -NH-, Rb is C₁₋₆ alkylene optionally substituted with C₁₋₁₀ alkyl, and R²⁰ is C₆₋₁₄ aryl optionally substituted with halogen or heteroaryl, and R³ is C₁₋₆ alkyl.

A compound used in the present disclosure or a pharmaceutically acceptable salt thereof, or a solvate thereof, is exemplified in Japanese Patent No. 5530427, and can be, for example,
(6S,9S)-N-benzyl-6-(4-hydroxybenzyl)-2,9-dimethyl-8-(naphthalen-1-ylmethyl)-4,7-dioxooctahydro-1H-pyrazino[2,1-c][1,2,4]triazine-1-carboxamide,
(6S,9S)-2-allyl-N-benzyl-6-(4-hydroxybenzyl)-9-methyl-8-(naphthalen-1-ylmethyl)-4,7-dioxooctahydro-1H-pyrazino[2,1-c][1,2,4]triazine-1-carboxamide,
(6S,9S)-N-benzyl-6-(4-hydroxybenzyl)-9-methyl-8-(naphthalen-1-ylmethyl)-4,7-dioxohexahydropyrazino[2,1-c][1,2,4]oxadiazine-1(6H)-carboxamide,
(6S,9S)-8-((2-aminobenzo[d]thiazol-4-yl)methyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide,
(6S,9S)-2-allyl-N-benzyl-6-(4-hydroxybenzyl)-9-methyl-4,7-dioxo-8-(quinolin-8-ylmethyl)octahydro-1H-pyrazino[2,1-c][1,2,4]triazine-1-carboxamide,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-8-(naphthalen-1-ylmethyl)-4,7-dioxooctahydro-1H-pyrazino[2,1-c][1,2,4]triazin-6-yl)methyl)phenyl dihydrogen phosphate,
4-(((6S,9S)-1-(benzylcarbamoyl)-2,9-dimethyl-4,7-dioxo-8-(quinolin-8-ylmethyl)octahydro-1H-pyrazino[2,1-c][1,2,4]triazin-6-yl)methyl)phenyl sodium phosphate
4-(((6S,9S)-1-(benzylcarbamoyl)-2,9-dimethyl-4,7-dioxo-8-(naphthalen-8-ylmethyl)octahydro-1H-pyrazino[2,1-c][1,2,4]triazin-6-yl)methyl)phenyl sodium phosphate,
(6S,9S)-2-allyl-6-(4-hydroxybenzyl)-9-methyl-4,7-dioxo-N-((R)-1-phenylethyl)-8-(quinolin-8-ylmethyl)octahydro-1H-pyrazino[2,1-c][1,2,4]triazine-1-carboxamide,
(6S,9S)-2-allyl-6-(4-hydroxybenzyl)-9-methyl-4,7-dioxo-N-((S)-1-phenylethyl)-8-(quinolin-8-ylmethyl)octahydro-1H-pyrazino[2,1-c][1,2,4]triazine-1-carboxamide,
(6S,9S)-N-benzyl-6-(4-hydroxy-2,6-dimethylbenzyl)-2,9-dimethyl-4,7-dioxo-8-(quinolin-8-ylmethyl)octahydro-1H-pyrazino[2,1-c][1,2,4]triazine-1-carboxamide,
(6S,9S)-8-(benzo[b]thiophen-3-ylmethyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-8-(benzo[c][1,2,5]thiadiazol-4-ylmethyl)-N-benzyl-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-8-(isoquinolin-5-ylmethyl)-2,9-dimethyl-4,7-dioxooctahydro-1H-pyrazino[2,1-c][1,2,4]triazine-1-carboxamide,
(6S,9S)-N-benzyl-8-((5-chlorothieno[3,2-b]pyridin-3-yl)methyl)-6-(4-hydroxybenzyl)-2,9-dimethyl-4,7-dioxooctahydro-1H-pyrazino[2,1-c][1,2,4]triazine-1-carboxamide,
(6S,9S)-N-benzyl-6-(4-hydroxybenzyl)-2,9-dimethyl-4,7-dioxo-8-(quinoxalin-5-ylmethyl)octahydro-1H-pyrazino[2,1-c][1,2,4]triazine-1-carboxamide,
(6S,9S)-6-(4-hydroxybenzyl)-2,9-dimethyl-4,7-dioxo-8-(quinolin-8-ylmethyl)-N-(thiophen-2-ylmethyl)octahydro-1H-pyrazino[2,1-c][1,2,4]triazine-1-carboxamide,
(6S,9S)-N-benzyl-6-(4-hydroxybenzyl)-2,9-dimethyl-4,7-dioxo-8-(quinolin-8-ylmethyl)hexahydro-2H-pyrazino[2,1-c][1,2,4]triazine-1(6H)-carboxamide (C-82), or
4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid (OK-1) or a pharmaceutically acceptable salt thereof, or a solvate thereof.

### Compound group [2]

A compound used in the present disclosure or a pharmaceutically acceptable salt thereof, or a solvate thereof, can be compound group [2]. Compound group [2] is described in Japanese National Phase PCT Laid-open Publication No. 2012-505153, and the content thereof is incorporated herein by reference. The compound can be, for example, a compound represented by the following formula: 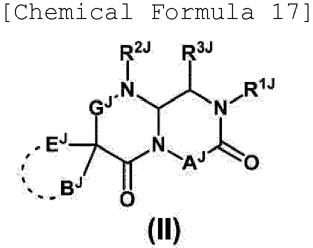 or a pharmaceutically acceptable salt thereof, or a solvate thereof, wherein
A^{J} is - (CHR^{7J})- (wherein R^{7J} is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
B^{J} and E^{J} are same or different, independently selected from hydrogen, optionally substituted alkyl, optionally substituted alkenyl, and optionally substituted alkynyl, or form an optionally substituted spiro ring represented by a dotted line,
G^{J} is -NH-, -NR^{J6}-, -O-, -CH₂-, -CHR^{6J}-, or -C(R^{6J})₂-(wherein R^{6J} is each the same or different, independently selected from optionally substituted alkyl, optionally substituted alkenyl, and optionally substituted alkynyl),
R^{1J} is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R^{2J} is -W^{21J}-W^{22J}-Rb^{J}-R^{20J} (wherein
   W^{21J} is -(CO)- or -(SO₂)-,
   W^{22J} is a bond, -O-, -NH-, or optionally substituted lower alkylene,
   Rb^{J} is a bond or optionally substituted lower alkylene, and
   R^{20J} is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R^{3J} is hydrogen, optionally substituted alkyl, optionally substituted alkenyl, or optionally substituted alkynyl,
with the proviso that
1) if Rb^{J} is optionally substituted lower alkylene, W^{22J} must be -O- or -NH-,
2) if E^{J} and B^{J} are hydrogen, R^{3J} must be hydrogen,
3) if G^{J} is -NH-, -CH₂-, -CHR^{6J}-, or -NR^{6J}-, B^{J} and E^{J} cannot be hydrogen, and,
4) if G^{J} is -O-, B^{J} and E^{J} are hydrogen, and R^{3J} is hydrogen, R^{1J} cannot be 8-quinolylmethyl.

In this regard, various substituents can have the same embodiment as compound group 1.

### Compound group [3]

A compound used in the present disclosure or a pharmaceutically acceptable salt thereof, or a solvate thereof, can be compound group [3]. Compound group [3] is described in Japanese National Phase PCT Laid-open Publication No. 2012-505153, and the content thereof is incorporated herein by reference. The compound can be, for example, a compound represented by 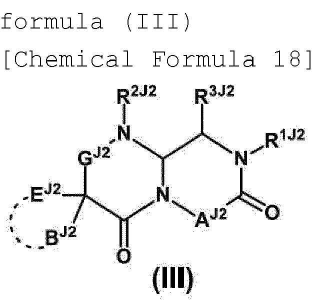 or a pharmaceutically acceptable salt thereof, or a solvate thereof, wherein
A^{J2} is -(CHR^{7J2})- (wherein R^{7J2} is C₁₋₁₀ alkyl optionally substituted with a substituent selected from OH, -OR^{8J2} (wherein R^{8J2} represents C₆₋₁₄ aryl-C₁₋₁₀ alkyl), -NH₂, -COOH, and -CONH₂, C₆₋₁₄ aryl, or C₆₋₁₄ aryl-C₁₋₁₀ alkyl optionally substituted with OH);
B^{J2} and E^{J2} are hydrogen;
G^{J2} is -O-;
R^{1J2} is C₁₋₁₀ alkyl optionally substituted with a substituent selected from OH, -COOH, and -COOR^{8J2'} (wherein R^{8J2'} represents C₁₋₁₀ alkyl), C₆₋₁₄ aryl-C₁₋₁₀ alkyl, heteroaryl-C₁₋₁₀ alkyl optionally substituted with -NH₂, or C₃₋₁₀ cycloalkyl-C₁₋₁₀ alkyl;
R^{2J2} is -W^{21J2}-W^{22J2}-Rb^{J2}-R^{20J2} (wherein
   W^{21J2} is -(CO)- or -(SO₂)-;
   W^{22J2} is a bond, -O-, or C₁₋₆ alkylene;
   Rb^{J2} is a bond or C₁₋₆ alkylene; and
   R^{20J2} is C₁₋₁₀ alkyl, C₆₋₁₄ aryl optionally substituted with halogen, or heteroaryl); and
R^{3J2} is hydrogen;
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon,
with the proviso that

1) if Rb^{J2} is C₁₋₆ alkylene, W^{22J2} must be -O-, and
2) R^{1J2} cannot be 8-quinolylmethyl.

### Compound group [4]

A compound used in the present disclosure or a pharmaceutically acceptable salt thereof, or a solvate thereof, can be compound group [4]. Compound group [4] is described in Japanese National Phase PCT Laid-open Publication No. 2012-526042, and the content thereof is incorporated herein by reference. The compound can be, for example, a compound represented by formula (IV): 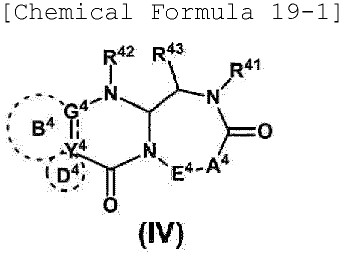 or a pharmaceutically acceptable salt thereof, or a solvate thereof, wherein 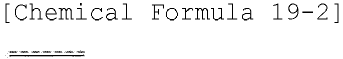 is a single bond or a double bond,
A⁴ is -CHR⁴⁷- (wherein R⁴⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
E⁴ is a bond, -CHR⁴⁵-, -O-, or -NR⁴⁸-
(wherein
   R⁴⁵ is hydrogen, optionally substituted alkyl, optionally substituted alkenyl, optionally substituted alkynyl, optionally substituted arylalkyl, or optionally substituted heteroarylalkyl, and
   R⁴⁸ is hydrogen, optionally substituted alkyl, optionally substituted alkenyl, or optionally substituted alkynyl),
B⁴ is absent, or an optionally substituted monocyclic ring formed together with G⁴ and Y⁴,
D⁴ is absent, or an optionally substituted spiro ring formed together with Y⁴, with the proviso that not both of B⁴ and D⁴ are present,
if B⁴ is present, G⁴ and Y⁴ are independently a carbon atom or a nitrogen atom,
if D⁴ is present, Y⁴ is a carbon atom, and G⁴ is -NR⁴⁶-, -O-, -CHR⁴⁶-, or -C(R⁴⁶)₂-,
if both B⁴ and D⁴ are absent, G⁴ and Y⁴ are the same or different, each -NR⁴⁶-, -O-, -CHR⁴⁶-, or -C(R⁴⁶)₂- (wherein R⁴⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl), and
if E⁴ is a bond, D⁴ is absent, B is an optionally substituted monocyclic ring, and B⁴ and Y⁴ are independently a carbon atom or a nitrogen atom,
R⁴¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁴² is -W⁴²¹-W⁴²²-Rb⁴-R⁴²⁰
(wherein
   W⁴²¹ is -(CO)- or -(SO₂)-,
   W⁴²² is a bond, -O-, -NH-, or optionally substituted lower alkylene,
   Rb⁴ is a bond or optionally substituted alkylene, and
   R⁴²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁴³ is hydrogen, optionally substituted alkyl, optionally substituted alkenyl, or optionally substituted alkynyl,
   with the proviso that
if D⁴ is absent, E⁴ is a bond, B is benzene, and R⁴² is -W⁴²¹-W⁴²²-Rb⁴-R⁴²⁰ (wherein W⁴²¹ is -(CO)-, W⁴²² is -NH-, and Rb⁴ is a bond), R⁴²⁰ cannot be optionally substituted phenyl.

### Compound group [5]

A compound used in the present disclosure or a pharmaceutically acceptable salt thereof, or a solvate thereof, can be compound group [5]. Compound group [5] is described in Japanese Patent No. 5768239, and the content thereof is incorporated herein by reference. The compound can be, for example, a compound represented by formula (V): 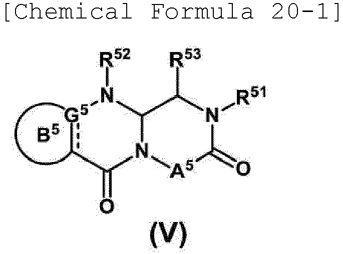 or a pharmaceutically acceptable salt thereof, or a solvate thereof, wherein 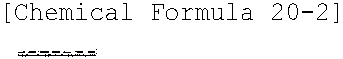 is a single bond or a double bond,
A⁵ is - (CHR⁵⁷) - (wherein R⁵⁷ is C₁₋₁₀ alkyl optionally substituted with a substituent selected from -OH, -OR⁵⁹ (wherein R⁵⁹ represents C₁₋₁₀ alkyl), -NH₂, -COOH, and -CONH₂, C₆₋₁₄ aryl, or C₆₋₁₄ aryl-C₁₋₁₀ alkyl optionally substituted with -OH),
B⁵ is a 5- or 6-membered monocyclic saturated carbon ring, or a saturated or unsaturated 4-, 5-, 6-, or 7-membered heterocyclic ring optionally substituted with a formyl group, formed together with G⁵, wherein a heteroatom is selected from S, N, and O, and the number of heteroatoms is an integer from 1 to 3,
G⁵ is a carbon atom or a nitrogen atom,
R⁵¹ is C₁₋₁₀ alkyl, C₆₋₁₄ aryl-C₁₋₁₀ alkyl optionally substituted with a substituent selected from -R^{59'} and halogen, or 5- to 14-membered heteroaryl-C₁₋₁₀ alkyl optionally substituted with -NHCOOR^{59'}, wherein R^{59'} is C₁₋₁₀ alkyl or C₆₋₁₄ aryl,
R⁵² is -W⁵²¹-W⁵²²-Rb⁵-R⁵²⁰ (wherein
   W⁵²¹ is -(CO)-,
   W⁵²² is -NH-,
   Rb⁵ is a bond, or C₁₋₆ alkylene optionally substituted with C₆₋₁₄ aryl, and
   R⁵²⁰ is C₁₋₁₀ alkyl optionally substituted with a substituent selected from -OH and -COOR^{59"} (wherein R^{59"} represents a hydrogen atom or C₁₋₁₀ alkyl), C₆₋₁₄ aryl optionally substituted with a substituent selected from halogen, methylenedioxy, and C₁₋₁₀ alkyl, 5- to 14-membered heteroaryl, or C₃₋₁₀ cycloalkyl), and
R⁵³ is hydrogen or C₁₋₁₀ alkyl.

### Compound group [6]

A compound used in the present disclosure or a pharmaceutically acceptable salt thereof, or a solvate thereof, can be compound group [6]. Compound group [6] is described in Japanese Patent No. 6040344, and the content thereof is incorporated herein by reference. The compound can be, for example, a compound represented by formula (VI): 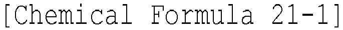 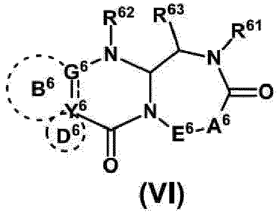 or a pharmaceutically acceptable salt thereof, or a solvate thereof, wherein 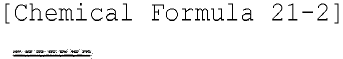 is a single bond or a double bond,
A⁶ is -CHR⁶⁷-, wherein R⁶⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl,
E⁶ is -CHR⁶⁵-, -O-, or -NR⁶⁸- (wherein
   R⁶⁵ is hydrogen, optionally substituted alkyl, optionally substituted alkenyl, optionally substituted alkynyl, optionally substituted arylalkyl, or optionally substituted heteroarylalkyl, and
   R⁶⁸ is hydrogen, optionally substituted alkyl, optionally substituted alkenyl, or optionally substituted alkynyl),
B⁶ is absent, or an optionally substituted monocyclic ring formed together with G⁶ and Y⁶,
D⁶ is absent, or an optionally substituted spiro ring formed together with Y⁶, with the proviso that
not both B⁶ and D⁶ are present,
if B⁶ is present, G⁶ and Y⁶ are independently a carbon atom or a nitrogen atom,
if D⁶ is present, Y⁶ is a carbon atom, and G⁶ is -NR⁶⁶-, -O-, -CHR⁶⁶-, or -C(R⁶⁶)₂-,
if B⁶ and D⁶ are both absent, G⁶ and Y⁶ are the same or different, each -NR⁶⁶-, -O-, -CHR⁶⁶-, or -C(R⁶⁶)₂ (wherein R⁶⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl),
R⁶¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁶² is -W⁶²¹-W⁶²²-Rb⁶-R⁶²⁰ (wherein
   W⁶²¹ is -(CO)- or -(SO₂)-,
   W⁶²² is a bond, -O-, -NH-, or optionally substituted lower alkylene,
   Rb⁶ is a bond or optionally substituted alkylene, and
   R⁶²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁶³ is hydrogen, optionally substituted alkyl, optionally substituted alkenyl, or optionally substituted alkynyl.

### Compound group [7]

A compound used in the present disclosure or a pharmaceutically acceptable salt thereof, or a solvate thereof, can be compound group [7]. Compound group [7] is described in Japanese National Phase PCT Laid-open Publication No. 2008-533155, and the content thereof is incorporated herein by reference. The compound can be, for example, a compound represented by formula (VII) 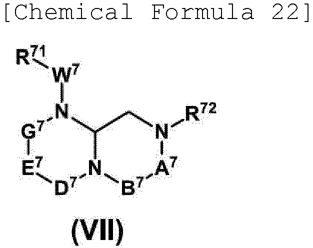 or a pharmaceutically acceptable salt thereof, or a solvate thereof, wherein
A⁷ is -(CHR⁷³)-(C=O)-, B⁷ is -(NR⁷⁴)-, D⁷ is -(CHR⁷⁵)- or -(C=O)-, E⁷ is -(ZR⁷⁶)- or -(C=O)-, G⁷ is -(X⁷R⁷⁷)ₙ₇-, -(CHR⁷⁷)-(NR⁷⁸)-, -(C=O)-(X⁷R⁷⁹)-, or -(C=O)-, W⁷ is -y⁷(C=O)-, - (C=O)NH-, -(SO₂)-, or absent, Y⁷ is oxygen or sulfur, X⁷ and Z⁷ are independently nitrogen or CH, n7 = 0 or 1, and R⁷¹, R⁷², R⁷³, R⁷⁴, R⁷⁵, R⁷⁶, R⁷⁷, R⁷⁸, and R⁷⁹ are the same or different, independently selected from an amino acid side chain, a derivative of an amino acid side chain, a linker facilitating the linkage of the compound to another moiety or compound, a linker attaching the compound to a solid support, and a solid support, and stereoisomers thereof.

R⁷¹, R⁷², R⁷³, R⁷⁴, R⁷⁵, R⁷⁶, R⁷⁷, R⁷⁸, and R⁷⁹ in formula (VII) are independently selected from the group consisting of amino C₂₋₅ alkyl, guanidino C₂₋₅ alkyl, C₁₋₄ alkylguanidino C₂₋₅ alkyl, di-C₁₋₄ alkylguanidino-C₂₋₅ alkyl, amidino C₂₋₅ alkyl, C₁₋₄ alkylamidino C₂₋₅ alkyl, di-C₁₋₄ alkylamidino C₂₋₅ alkyl, C₁₋₃ alkoxyl, phenyl, substituted phenyl (wherein substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), benzyl, substituted benzyl (wherein substituents on the benzyl are independently selected from one or more of amino, amidino, guanidino, hydrazino, amidrazonyl, C₁₋₄ alkylamino, C₁₋₄ di-alkylamino, halogen, perfluoro C₁₋₄ alkyl, C₁₋₄ alkyl, C₁₋₃ alkoxyl, nitro, carboxy, cyano, sulfuryl, and hydroxyl), naphthyl, substituted naphthyl (wherein substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), bis-phenylmethyl, substituted bis-phenylmethyl (wherein substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), pyridyl, substituted pyridyl, (wherein substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), pyridyl C₁₋₄ alkyl, substituted pyridyl C₁₋₄ alkyl (wherein pyridine substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), pyrimidyl C₁₋₄ alkyl, substituted pyrimidyl C₁₋₄ alkyl (wherein pyrimidine substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), triazin-2-yl-C₁₋₄ alkyl, substituted triazin-2-yl-C₁₋₄ alkyl (wherein triazine substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), imidazo C₁₋₄ alkyl, substituted imidazole C₁₋₄ alkyl (wherein imidazole substituents are independently selected from one or more of amino, amidino, guanidino, hydrazino, amidrazonyl, C₁₋₄ alkylamino, C₁₋₄ dialkylamino, halogen, perfluoro C₁₋₄ alkyl, C₁₋₄ alkyl, C₁₋₃ alkoxyl, nitro, carboxy, cyano, sulfuryl, and hydroxyl), imidazolinyl C₁₋₄ alkyl, N-amidinopiperazinyl)-N-C₀₋₄ alkyl, hydroxy C₂₋₅ alkyl, C₁₋₅ alkylamino C₂₋₅ alkyl, hydroxy C₂₋₅ alkyl, C₁₋₅ alkylamino C₂₋₅ alkyl, C₁₋₅ dialkylamino C₂₋₅ alkyl, N-amidinopiperidinyl C₁₋₄ alkyl, and 4-aminocyclohexyl C₀₋₂ alkyl.

### Compound group [8]

A compound used in the present disclosure or a pharmaceutically acceptable salt thereof, or a solvate thereof, can be compound group [8]. Compound group [8] is described in US Patent No. 5929237, and the content thereof is incorporated herein by reference. The compound can be, for example, a compound represented by formula (VIII) 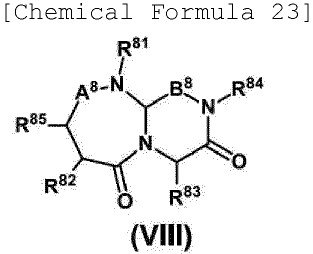 or a pharmaceutically acceptable salt thereof, or a solvate thereof, wherein
A⁸ is -(CHR^{8'})ₙ₈-, B⁸ is -(CHR^{B"})ₘ₈-, wherein n8 is 0, 1, or 2, m8 is 1, 2, or 3, and R^{8'} , R^{8"}, R⁸¹, R⁸², R⁸³, R⁸⁴, and R⁸⁵ are the same as defined below.

Instruction with solid lines for attachment of R⁸², R⁸³, and R⁸⁵ means that these R groups can be either above or below the plane of this page in formula (VIII). If a compound of formula (VIII) is intended to be similar to the opposite turn of a naturally-occurring amino acid (i.e., "L-amino acid"), the R groups are generally below the plane of this page in formula (VIII) (i.e., 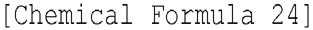 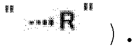 However, if a compound of formula (VIII) is intended to be similar to the opposite turn including one or more D-amino acids, the corresponding R groups are above the plane of this page in formula (VIII) (i.e.,

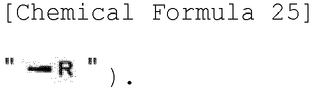

In one embodiment, R⁸¹ and R⁸⁴ are the same or different, representing the remaining portions of the compound, and R^{8'}, R^{8"}, R⁸², R⁸³, and R⁸⁵ are the same or different, independently selected from an amino acid side chain moiety or a derivative thereof. R^{8'} and R^{8"} should be understood, so that each presence of R^{8'} and R^{8"} is independently selected from an amino acid side chain moiety and a derivative thereof. If, for example, m8 = 2, B⁸ is a -CHR^{8"}CHR^{8"}- moiety. In such a case, the presence of both R^{8"} is independently selected and can be the same or different. Thus, if the first R^{8"} is hydrogen and the second R^{8"} is methyl, B⁸ has a structure of -CH₂CH(CH₃)-.

As used herein, the term "remaining portion of the compound" refers to a moiety, a reagent, a compound, a support, a molecule, a linker, an amino acid, a peptide, or a protein to be covalently attached to the basic backbone moiety of a compound of formula (VIII) at the position of R⁸¹ and/or R⁸⁴. The term also encompasses amino acid side chain moieties and derivatives thereof.

As used herein, the term "amino acid side chain moiety" represents one of the amino acid side chain moieties that is present in a naturally-occurring protein (which include, but are not limited to side chain moieties of naturally-occurring amino acids confirmed in Table 1). Other examples of naturally occurring amino acid side chain moieties of the present disclosure include, but are not limited to, 3,5-dibromotyrosine, 3,5-diiodotyrosine, hydroxylysine, γ-carboxyglutamate, phosphotyrosine, and phosphoserine. Glycosylated amino acid side chains can also be used for the practice of the present disclosure, which include, but are not limited to, glycosylated threonine, serine, and asparagine.

### [Table 1]

**Table 1**

| | Amino acid side chain moieties | |
|---|---|---|
| Amino acid side chain moieties | | Amino acids |
| -H | | Glycine |
| -CH₃ | | Alanine |
| -CH(CH₃)₂ | | Valine |
| -CH₂CH(CH₃)₂ | | Leucine |
| -CH(CH₃)CH₂CH₃ | | Isoleucine |
| -(CH₂)₄NH₃ | | Lysine |
| -CH₂)₃NHC(NH₂)NH₃ | | Arginine |
| 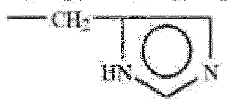 | | Histidine |
| -CH₂COO⁻ | | Aspartic acid |
| -CH₂CH₂COO⁻ | | Glutamic acid |
| -CH₂CONH₂ | | Asparagine |
| -CH₂CH₂CONH₂ | | Glutamine |
| 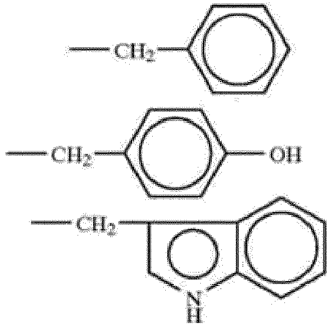 | | Phenylalanine |
| | | Tyrosine |
| | | Tryptophan |
| -CH₂SH | | Cysteine |
| -CH₂CH₂SCH₃ | | Methionine |
| -CH₂OH | | Serine |
| -CH(OH)CH₃ | | Threonine |
| 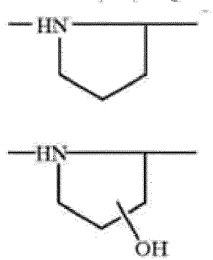 | | Proline |
| | | Hydroxy proline |

In addition to naturally-occurring amino acid side chain moieties, the amino acid side chain moieties of the present disclosure encompass various derivatives thereof. As used herein, a "derivative" of an amino acid side chain moiety comprises an alternation and/or change to a naturally-occurring amino acid side chain moiety. For example, an amino acid side chain moiety of alanine, valine, leucine, isoleucine, and phenylalanine can be generally classified as a lower chain alkyl, aryl, or aralkyl moiety. Derivatives of an amino acid side chain moiety include other linear or branched, cyclic or non-cyclic, substituted or unsubstituted, saturated or unsaturated lower chain alkyl, aryl, or arylalkyl moieties.

The term "lower chain alkyl moiety" comprises 1 to 12 carbon atoms, "lower chain aryl moiety" comprises 6 to 12 carbon atoms, and "lower chain aralkyl moiety" comprises 7 to 12 carbon atoms. Thus, in one embodiment, an amino acid side chain derivative is selected from C₁₋₁₂ alkyl, C₆₋₁₂ aryl, and C₇₋₁₂ aralkyl, and selected from C₁₋₇ alkyl, C₆₋₁₀ aryl, and C₇₋₁₁ aralkyl in a more preferred embodiment.

The amino acid side chain derivative of the present disclosure further includes derivatives with a substitution at a lower chain alkyl, aryl, or aralkyl moiety, wherein the substituent is selected from one or more of the following chemical moieties (but not limited thereto): -OH, -OR⁸⁸, - COOH, -COOR⁸⁸, -CONH₂, -NH₂, -NHR⁸⁸, -NR⁸⁸R⁸⁸, -SH, -SR⁸⁸, - SO₂R⁸⁸, -SO₂H, -SOR⁸⁸, and halogen (including F, Cl, Br, and I), wherein each presence of R⁸⁸ is independently selected from lower chain alkyl, aryl, and aralkyl moieties. The cyclic lower chain alkyl, aryl, and aralkyl moieties of the present disclosure further encompass naphthalene and heterocyclic compounds (e.g., thiophene, pyrrole, furan, imidazole, oxazole, thiazole, pyrazole, 3-pyrroline, pyrrolidine, pyridine, pyrimidine, purine, quinoline, isoquinoline, and carbazole). An amino acid side chain derivative includes (but are not limited to) heteroalkyl derivatives of lower chain alkyl and aralkyl moieties and phosphonate and silane of alkyl and aralkyl.

In another embodiment, R⁸¹, R⁸², R⁸³, R⁸⁴, or R⁸⁵ can be, in addition to an amino acid side chain moiety or a derivative thereof, or a remaining portion of the compound for R⁸¹ and R⁸⁴, a linker facilitating the linkage of the compound to another moiety or compound. For example, the compound of the present disclosure can attach to one or more known compounds (e.g., biotin) for use in a diagnostic assay or screening assay. Furthermore, R⁸¹, R⁸², R⁸³, R⁸⁴, or R⁸⁵ can be a linker that attaches this compound to a solid support (e.g., support used in solid phase peptide synthesis), or alternatively can be the support itself. In this embodiment, attachment to another moiety or a compound or attachment to a solid support is preferably at the position of R⁸¹ or R⁸⁴, more preferably at the position of R⁸⁴.

In a preferred embodiment, R⁸⁵ is hydrogen.

In one embodiment of the present disclosure, n8 = 0, R^{8"} is hydrogen, R⁸⁵ is hydrogen, and the compound of formula (VIII) is represented by formula (VIIIa): 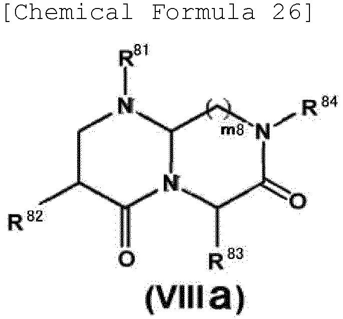 wherein m8 = 1, 2, or 3, and R⁸¹, R⁸², R⁸³, and R⁸⁴ are defined the same as above. In a preferred embodiment, m8 = 1 or 2, R⁸¹ and R⁸⁴ are remaining portions of the compound, and R⁸² and R⁸³ are independently selected from an amino acid side chain moiety.

In another embodiment, n8 = 0, m8 = 1, R^{8"} is hydrogen, R⁸⁵ is hydrogen, and a compound of formula (VIII) is represented by formula (VIIIb): 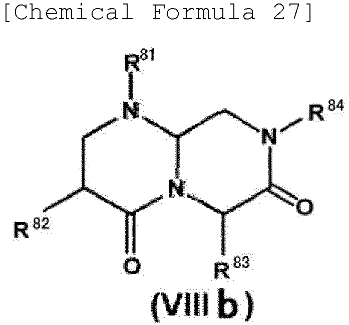 wherein R⁸¹, R⁸², R⁸³, and R⁸⁴ are the same as defined above. In a preferred embodiment, R⁸¹ and R⁸⁴ are remaining portions of the compound, and R⁸² and R⁸³ are independently selected from an amino acid side chain moiety.

A compound used in the present disclosure can be a compound represented by formula (VIII) 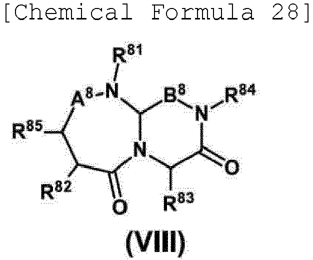 wherein
A⁸ is -(CHR^{8'})ₙ₈-,
B⁸ is -(CHR^{8"})ₘ₈-, n8 is 0, 1, or 2, m8 is 1, 2, or 3, R^{8'}, R^{8"}, R⁸², R⁸³, and R⁸⁵ are the same or different, each of R^{8'}, R^{8"}, R⁸², R⁸³, and R⁸⁵ that are present is independently selected from the group consisting of an amino acid side chain moiety, an amino acid side chain derivative, a linker, and a solid support,
R⁸¹ and R⁸⁴ represent the remaining portions of the compound, wherein R⁸¹ and R⁸⁴ are the same or different, independently selected from the group consisting of a certain moiety, an active substance, a compound, a support, a molecule, a linker, an amino acid, a peptide, and a protein.

In one embodiment, one or less of R^{8'}, R^{8"}, R⁸¹, R⁸², R⁸³, R⁸⁴, and R⁸⁵ is a solid support.

In one embodiment, R^{8'} is hydrogen.

In one embodiment, R^{8"} is hydrogen.

In one embodiment, n8 = 0.

In one embodiment, m8 = 1 or 2.

In one embodiment, R⁸⁵ is hydrogen.

In a preferred embodiment, n8 = 0, R^{8"} and R⁸⁵ are hydrogen, and the compound is represented by structural formula (VIIIa) 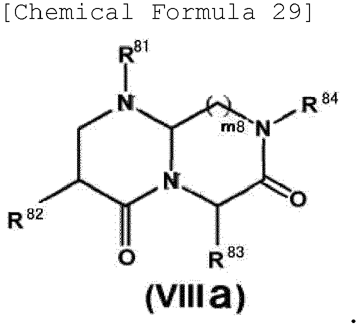 In an embodiment of a compound of this formula,
in a preferred embodiment, R⁸² and R⁸³ are the same or different, independently selected from an amino acid moiety and a derivative thereof.

In a preferred embodiment, R⁸⁴ is linked to a solid support.

In a more preferred embodiment, n8 = 0, R^{8"} and R⁸⁵ are hydrogen, m8 = 1, and the compound is represented by structural formula (VIIIb) 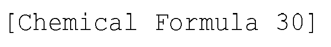 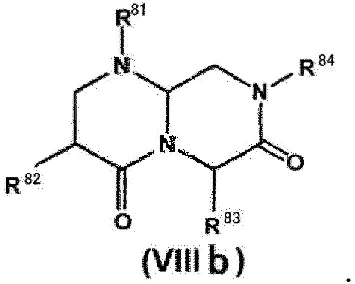 In an embodiment of a compound of this formula, R⁸² and R⁸³ are the same or different, independently selected from an amino acid moiety and a derivative thereof, and R⁸⁴ is linked to a solid support.

A compound of formula (VIII) used in the present disclosure or a pharmaceutically acceptable salt thereof, or a solvate thereof, encompasses ICG-001 represented by 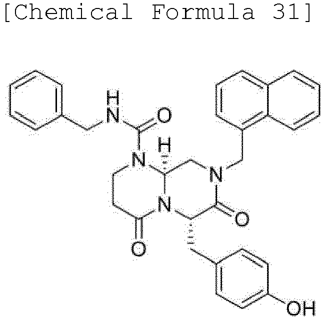 and a derivative thereof and a pharmaceutically acceptable salt thereof, and a solvate thereof.

### Compound group [9]

A compound used in the present disclosure or a pharmaceutically acceptable salt thereof, or a solvate thereof, can be compound group [9]. Compound group [9] is described in Japanese Patent No. 6085040 (WO 2015/098853), and the content thereof is incorporated herein by reference. The compound can be, for example, a compound represented by formula (IX) 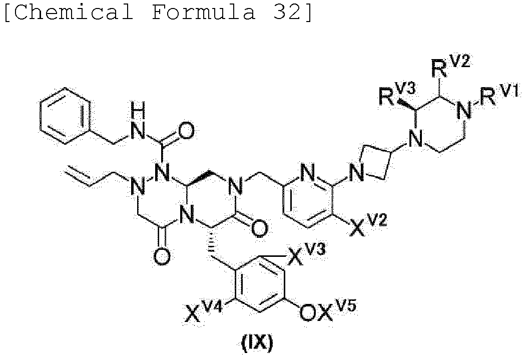 or a pharmaceutically acceptable salt thereof, or a solvate thereof, wherein R^{V1} refers to a C₁₋₆ alkyl group, R^{V2} and R^{V3} are each the same or different, referring to a hydrogen atom or a C₁₋₆ alkyl group, X^{V2}, X^{V3}, and X^{V4} are each the same or different, referring to a hydrogen atom or a halogen atom, and X^{V5} refers to a hydrogen atom or -P(=O)(OH)₂.

A compound used in the present disclosure or a pharmaceutically acceptable salt thereof, or a solvate thereof, can be
(6S,9aS)-N-benzyl-8-((6-(3-(4-ethylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-(4-ethylpiperazin-1-yl)azetidin-1-yl)pyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2-fluoro-4-hydroxyphenyl)methyl)-8-((6-(3-(4-methylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3S)-3,4-dimethylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3R)-3,4-dimethylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((2S)-2,4-dimethylpiperazin-1-yl)azetidin-1-yl)pyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-(4-ethylpiperazin-1-yl)azetidin-1-yl)-5-fluoropyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((5-fluoro-6-(3-(4-methylpiperazin-1-yl)azetidin-1-yl)pyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((4-hydroxyphenyl)methyl)-4,7-dioxo-2-(prop-2-en-1-yl)-8-((6-(3-(4-(propan-2-yl)piperazin-1-yl)azetidin-1-yl)pyridin-2-yl)methyl)-octahydro-1H-pyrazino[2,1-c][1,2,4]triazine-1-carboxamide,
(6S,9aS)-N-benzyl-6-((2-fluoro-4-hydroxyphenyl)methyl)-8-((5-fluoro-6-(3-(4-methylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2-fluoro-4-hydroxyphenyl)methyl)-4,7-dioxo-2-(prop-2-en-1-yl)-8-((6-(3-(4-(propan-2-yl)piperazin-1-yl)azetidin-1-yl)pyridin-2-yl)methyl)-octahydro-1H-pyrazino[2,1-c][1,2,4]triazine-1-carboxamide,
(6S,9aS)-N-benzyl-6-((2,6-difluoro-4-hydroxyphenyl)methyl)-4,7-dioxo-2-(prop-2-en-1-yl)-8-((6-(3-(4-(propan-2-yl)piperazin-1-yl)azetidin-1-yl)pyridin-2-yl)methyl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-(4-ethylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-(4-methylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((2S)-2,4-dimethylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-((3S)-3,4-dimethylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-((2S)-2,4-dimethylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3S)-4-ethyl-3-methylpiperazin-1-yl)azetidin-1-yl)pyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-3-((3R)-4-ethyl-3-methylpiperazin-1-yl)azetidin-1-yl)pyridin-2-yl)methyl-6-((4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3S)-4-ethyl-3-methylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3R)-4-ethyl-3-methylpiperazin-1-yl)azetidin-1-yl)pyridin-2-yl)methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-((3S)-4-ethyl-3-methylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-6-((2,6-difluoro-4-hydroxyphenyl)methyl)-8-((6-(3-((3R)-4-ethyl-3-methylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazine-1-carboxamide,
(6S,9aS)-N-benzyl-8-((6-(3-((3R)-3,4-dimethylpiperazin-1-yl)azetidin-1-yl)-5-fluoropyridin-2-yl)methyl)-6-((4-hydroxyphenyl)methyl)-4,7-dioxo-2-(prop-2-en-1-yl)-octahydro-1H-pyrazino[2,1-c][1,2,4]triazine-1-carboxamide,
4-(((6S,9aS)-1-(benzylcarbamoyl)-8-((6-(3-(4-ethylpiperazin-1-yl)azetidin-1-yl)pyridin-2-yl)methyl)-4,7-dioxo-2-(prop-2-en-1-yl)-octahydro-1H-pyrazino[2,1-c][1,2,4]triazin-6-yl)methyl)-3-fluorophenyl dihydrogen phosphate, or
4-(((6S,9aS)-1-(benzylcarbamoyl)-8-((6-(3-(4-ethylpiperazin-1-yl)azetidin-1-yl)pyridin-2-yl)methyl-4,7-dioxo-2-(prop-2-en-1-yl)-octahydro-1H-pyrazino[2,1-c][1,2,4]triazin-6-yl)methyl)phenyl dihydrogen phosphate, or a pharmaceutically acceptable salt thereof, or a solvate thereof.

### (Manufacturing method of a compound)

The manufacturing method of the compound of the present disclosure is described below, but the manufacturing method of the compound of the present disclosure is not limited thereto.

For example, the compound of the present disclosure can be manufactured by using the manufacturing method, condition, material, etc. described in Japanese Patent No. 5530427, Japanese National Phase PCT Laid-open Publication No. 2012-505153, Japanese Patent No. 5545573, Japanese National Phase PCT Laid-open Publication No. 2012-526042, Japanese Patent No. 5768239, Japanese Patent No. 6040344, Japanese Patent No. 6085040 (WO 2015/098853), US Patent No. 5929237, Japanese National Phase PCT Laid-open Publication No. 2008-533155, etc. The descriptions thereof are incorporated herein by reference in their entirety.

The compound of the present disclosure can be manufactured by, for example, the following manufacturing methods, but the methods are not limited to such methods. These manufacturing methods can be appropriately improved upon based on the expertise of those skilled in the art of organic synthetic chemistry. Salts of the compounds used as a raw material can be used in the following manufacturing method, as long as the reaction is not affected.

In the manufacturing methods of the present disclosure, even if use of a specific protecting group is not explicitly described, a functional group other than those at the reaction point can be protected as needed and deprotected after the completion of a reaction or after a series of reactions to obtain a compound of interest if one of the functional groups other than those at the reaction point is altered under the reaction condition or if it is unsuitable for post-reaction processing. Common protecting groups described in documents (T. W. Greene and P. G. M. Wuts, "Protective Group in Organic Synthesis", 3rd Ed., John Wiley and Sons, Inc., New York (1999)), etc. can be used as the protecting groups used in these processes. A protecting group can be introduced or removed by a method that is commonly used in organic synthetic chemistry (e.g., method described in the aforementioned document, etc.) or a method in accordance thereto.

The starting material and intermediate in the manufacturing methods of the present disclosure can be purchased as a commercially available product or are available by synthesis in accordance with a method described in a known document or a known method from a known compound. Salts of the starting materials and intermediates can also be used, as long as the reaction is not affected.

The intermediate and compound of interest in the manufacturing methods of the present disclosure can also be converted into another compound encompassed by the present disclosure by appropriately converting their functional groups. A functional group can be converted in doing so by a method that is commonly used in organic synthetic chemistry (e.g., method described in R. C. Larock, "Comprehensive Organic Transformations", 2nd Ed., John Wiley and Sons, Inc., New York (1999), etc.) or a method in accordance therewith.

The compounds used in the present disclosure can be generally synthesized by a technique described in the following Synthesis Schemes 1, 2, and 3.

### Synthesis Scheme 1

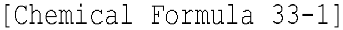

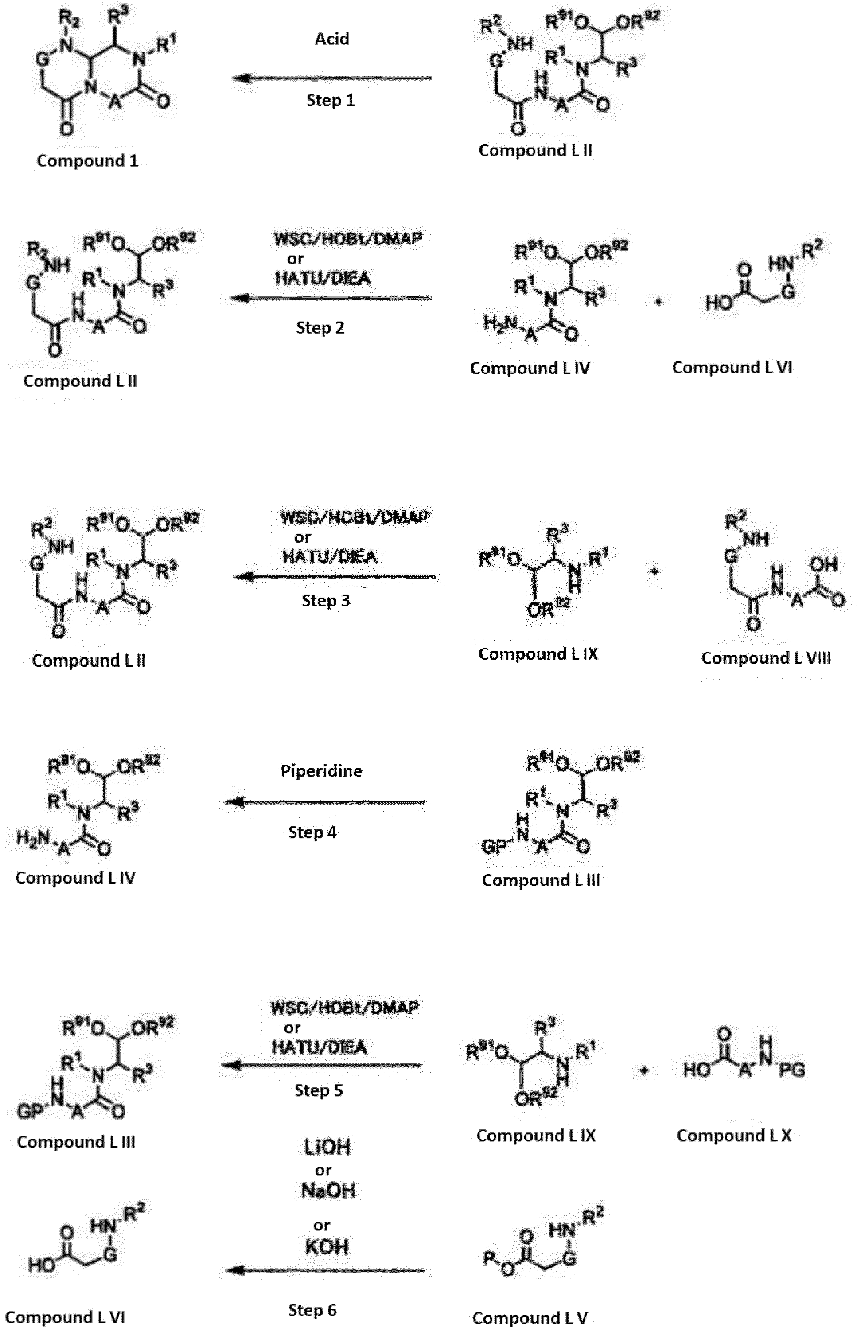

### Synthesis Scheme 2

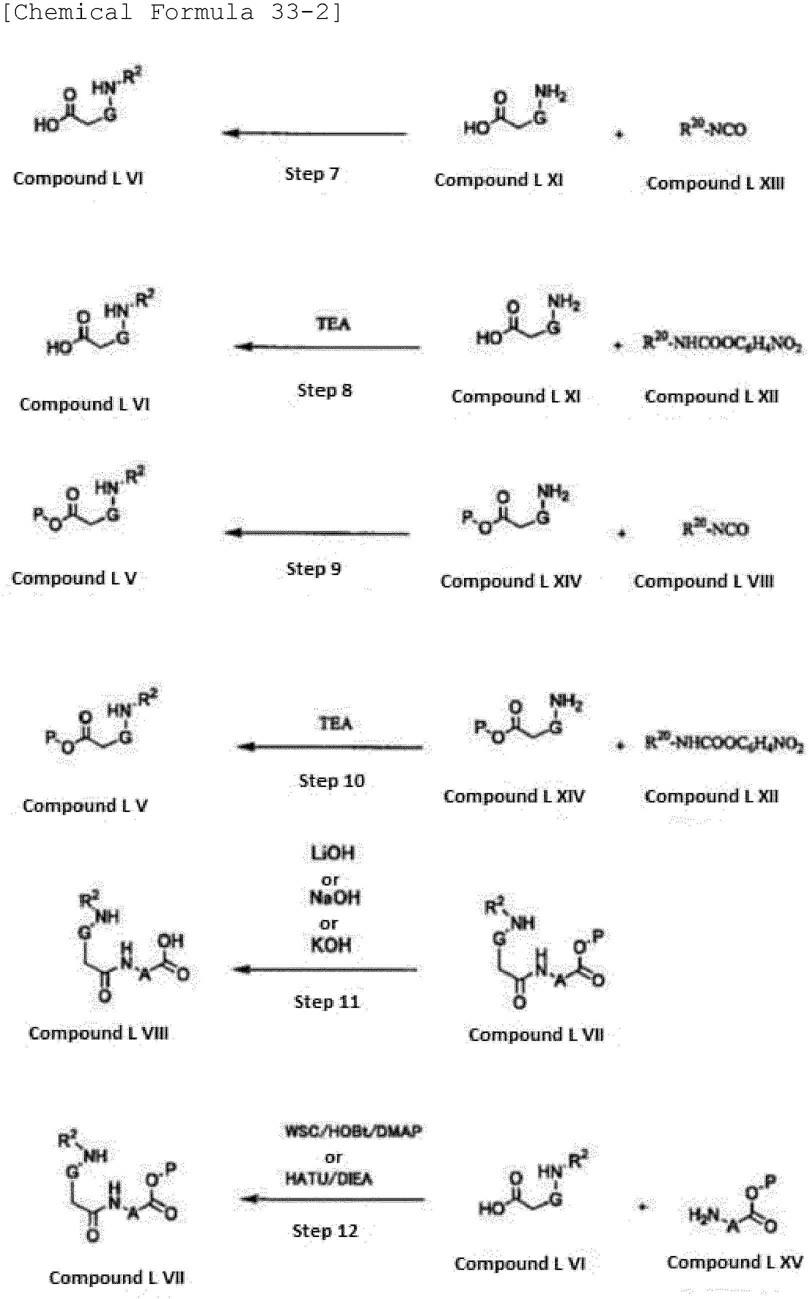

### Synthesis Scheme 3

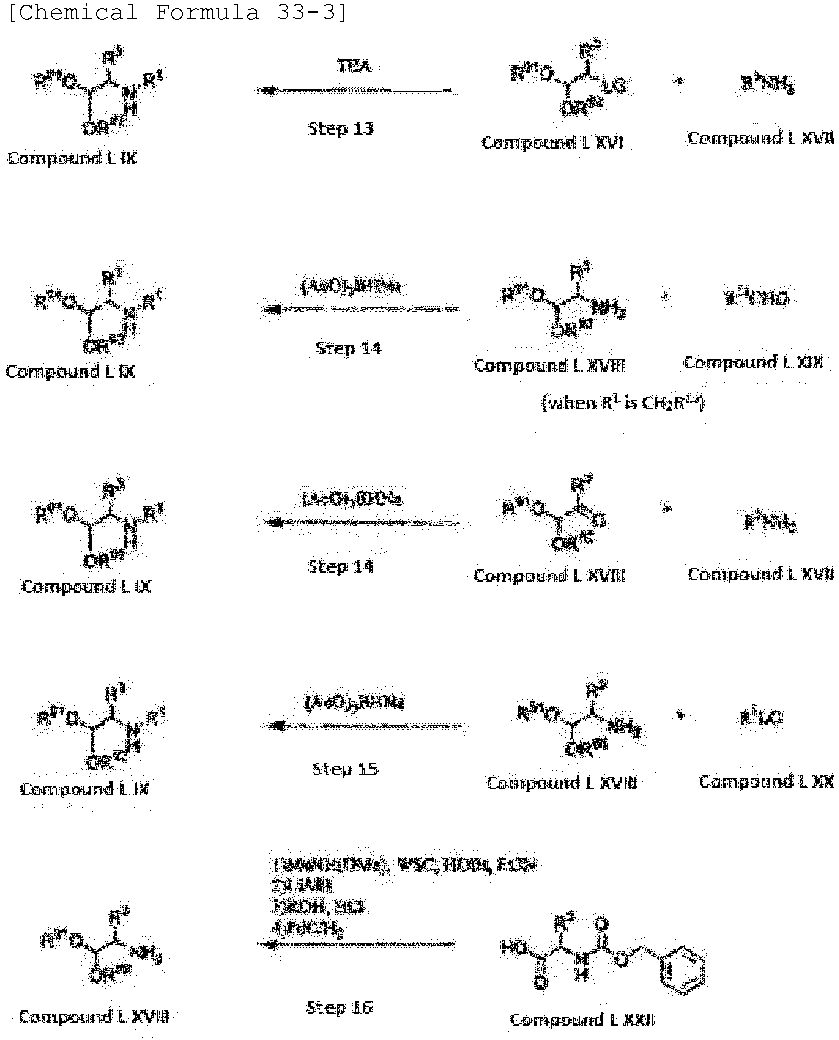

In view of for example Schemes 1, 2, and 3, compound LIX can have the disclosed structure (wherein R¹ and R³ are defined above, R⁹¹ and R⁹² are protecting groups that are suitable for use in synthesis, where the protecting groups may be attached to a solid phase macromolecular support or linker to enable solid phase synthesis) . Examples of suitable R⁹¹ group and R⁹² group include optionally substituted alkyl groups. In a preferred embodiment, R⁹¹ and R⁹² are both a methyl group or ethyl group. Such a compound IX can be readily synthesized by reductive amination of H₂N-R¹ with CH(OR⁹¹)(OR⁹²)-C(=O)R3, reductive amination of R^{1a}-CHO (wherein R¹ is equivalent to CH₂-R^{1a}) with CH(OR⁹¹)(OR⁹²)-CHR³NH₂, a substitution reaction of H₂N-R¹ with CH(OR⁹¹)(OR⁹²)-CHR³-LG (wherein LG refers to a leaving group, e.g., halogen (Hal) group), or a substitution reaction of LG-R¹ with CH(OR⁹¹)(OR⁹²)-CHR³-NH₂ (wherein LG refers to a leaving group, e.g., halogen (Hal) group).

Compound LIII can have the disclosed structure, wherein PG is an amino protecting group that is suitable for use in peptide synthesis, and A is defined the same as above. Examples of a preferred protecting group include 9H-fluorenylmethyloxycarbonyl (FMOC), t-butyldimethylsilyl (TBDMS), t-butyloxycarbonyl (BOC), methyloxycarbonyl (MOC), and allyl-oxycarbonyl (Alloc). Commercial N-protected amino acids are available. For example, when an azide derivative of an amino acid that is available from various sources is provided as compound LIII, FMOC amino acid can be manufactured from a corresponding amino acid through a reaction disclosed in Zaloom et al. (J. Org. Chem. 46: 5173-76, 1981).

Compound LVI of the present disclosure can have the disclosed structure (wherein PG and R² are as defined above). Other suitable compound LVI are available as commercial products from various sources, or can be prepared by a method that is well known in organic chemistry.

Compounds LX, LXI, LXIII, LXIV, LXV, LXVI, LXVII, LXVIII, LXIX, LXX, and LXXI are available as commercial products from various sources, or can be prepared by a method that is well known in organic chemistry.

As described in Figures **1**, **2**, and **3**, a compound of formula (I) (compound I) can be synthesized by reacting compound LIX with compound LX to obtain a bound compound LIII, then treating the bound compound LIII with piperidine to provide compound LIV. Compound LIV is sequentially reacted with compound LVI to provide bound compound LII, then the intermediate is cyclized to obtain a compound of formula (I) . Alternatively, as described in Synthesis Schemes 1, 2, and 3, a compound of formula (I) can be synthesized by reacting compound LVI with compound LXV to obtain bound compound LVII, then treating the bound compound LVII with lithium hydroxide, sodium hydroxide, or potassium hydroxide to provide compound LVIII. Compound LVIII is sequentially reacted with compound LIX to provide bound compound LII, then the intermediate is cyclized to obtain a compound of formula (I).

The preparation method of the compound of formula (I) is not limited to the methods described herein. For example, the compound of the present disclosure can be manufactured by modifying or converting a substituent of a compound that is useful as a precursor of the compound in accordance with a method or combination of methods described in a general publication in the field of chemistry.

Examples of recrystallization solvents that can be used include alcohol solvents such as methanol, ethanol, and 2-propanol, ether solvents such as diethyl ether, ester solvents such as ethyl acetate, aromatic hydrocarbon solvents such as benzene and toluene, ketone solvents such as acetone, halogen solvents such as dichloromethane and chloroform, hydrocarbon solvents such as hexane, aprotic solvents such as dimethylformamide and acetonitrile, water, mixtures thereof, etc. The methods described in Jikken Kagaku Koza [Experimental Chemistry] (Ed. by The Chemical Society of Japan, Maruzen) Vol. 1, etc. can be used as other purification methods. The molecular structure of the compound of the present disclosure can be readily determined by a spectroscopic method such as nuclear magnetic resonance, infrared spectroscopy, or circular dichroism spectroscopy, or mass spectrometry by referring to the structure derived from each raw material compound.

The intermediate or final product in the manufacturing method described above can lead to another compound encompassed by the present disclosure by appropriately converting a functional group thereof, extending various side changes from especially an amino, hydroxyl group, carbonyl, halogen, etc, and, in doing so, applying protection and deprotection described below as needed. Conversion of a functional group and extension of a side chain can be performed using a common method that is routinely used (see, for example, Comprehensive Organic Transformations, R. C. Larock, John Wiley & Sons Inc. (1999) or the like).

Examples of protecting groups of amino that can be used include alkylcarbonyl (e.g., acetyl and propionyl), formyl, phenylcarbonyl, alkyloxycarbonyl (e.g., methoxycarbonyl, ethoxycarbonyl, and tert-butoxycarbonyl), phenyloxycarbonyl, arylalkyloxycarbonyl (e.g., benzyloxycarbonyl), trityl, phthaloyl, tocyl, and benzyl.

Examples of protecting groups of carboxyl that can be used include alkyl (e.g., methyl, ethyl, propyl, isopropyl, butyl, and tert-butyl), phenyl, benzyl, trityl, and silyl (e.g., trimethylsilyl and tert-butyldimethylsilyl).

Examples of protecting groups of hydroxy that can be used include methyl, tert-butyl, allyl, substituted methyl (e.g., methoxymethyl and methoxyethoxymethyl), ethoxyethyl, tetrahydropyranyl, tetrahydrofuranyl, trityl, arylalkyl (e.g., benzyl), alkylcarbonyl (e.g., acetyl and propionyl), formyl, benzoyl, arylalkyloxycarbonyl (e.g., benzyloxycarbonyl), and silyl (e.g., trimethylsilyl and tert-butyldimethylsilyl).

Carbonyl can be protected by converting carbonyl into acyclic ketal (dimethyl ketal, diethyl ketal, etc.) or cyclic ketal (1,3-dioxolane, 1,3-dioxane, etc.).

The compound of the present disclosure represented by formula (I) 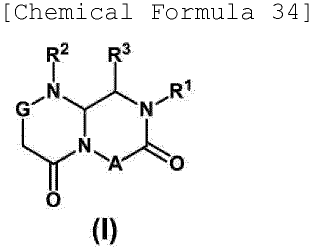 or pharmaceutically acceptable salt thereof can have asymmetry or a substituent having an asymmetric carbon. Such a compound has an enantiomer. The compound of the present disclosure also encompasses mixtures of each isomer and isolated isomers, which can be manufactured in accordance with a conventional method.

Examples of the manufacturing method include a method using a raw material having an asymmetric point and a method of introducing asymmetry during the process. Enantiomers for example can be obtained by using an optically active raw material, or performing optical resolution, etc. at a suitable stage of a manufacturing step. Examples of optical resolution methods include a diastereomer method of forming a salt, when the compound represented by formula (I) 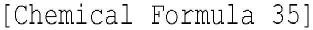 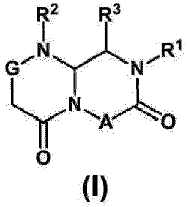 or an intermediate thereof has a basic functional group, in an inert solvent (e.g., an alcohol solvent such as methanol, ethanol, or 2-propanol; an ether solvent such as diethyl ether; an ester solvent such as ethyl acetate; a hydrocarbon solvent such as toluene; an aprotic solvent such as acetonitrile; or a mixture of two or more of said solvents) using an optically active acid (e. g., monocarboxylic acid such as mandelic acid, N-benzyloxyalanine, or lactic acid, dicarboxylic acid such as tartaric acid, ortho-diisopropylidene tartaric acid, or malic acid, or sulfonic acid such as camphorsulfonic acid or bromocamphorsulfonic acid).

When the compound of the present disclosure represented by formula (I) 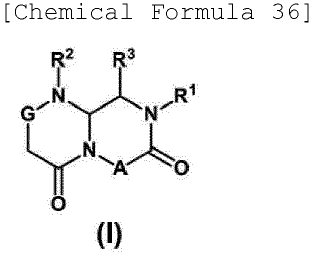 or an intermediate thereof has an acidic functional group such as a carboxyl group, optical resolution can be performed by forming a salt using an optically active amine (e.g., organic amines such as 1-phenylethylamine, quinine, quinidine, cinchonidine, cinchonine, or strychnine).

A temperature for the formation of a salt is selected from the range from -50°C to the boiling point of a solvent, preferably from the range from -0°C to the boiling point, and more preferably from the range from room temperature to the boiling point of a solvent. To improve the optical purity, it is desirable to first increase the temperature to a temperature near the boiling point of a solvent. When filtering out a precipitated salt, the temperature can be cooled as needed to improve the yield. The amount of an optically active acid or amine used is suitably in the range from about 0.5 to about 2.0 equivalents and preferably approximately 1 equivalent relative to a substrate. A crystal can be recrystallized in an inert solvent (e.g., an alcohol solvent such as methanol, ethanol, or 2-propanol; an ether solvent such as diethyl ether; an ester solvent such as ethyl acetate; a hydrocarbon solvent such as toluene; an aprotic solvent such as acetonitrile; or a mixture said solvents) as needed to obtain an optically active salt with high purity. An optically resolved salt can also be treated with an acid or a base by a conventional method to obtain its free form as needed.

Raw materials and intermediates in each of the manufacturing methods described above without a specific description of the manufacturing method are commercially available compounds, or compounds that can be synthesized from a commercially available compound by a method known to those skilled in the art or a method in accordance thereto.

### (Description of preferred embodiments)

Preferred embodiments of the present disclosure are described below. Embodiments described below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is also understood that the following embodiments of the present disclosure can be used independently or as a combination thereof.

### (Treatment, prevention, slowing of progression, or modulation of sarcopenia related disease, disorder, or symptom)

In one aspect, the present disclosure provides a composition, medicament, method, compound, use, etc. for modulating, slowing the progression of, preventing, or treating a sarcopenia related disease, disorder, or symptom, comprising or utilizing at least one selected from the group of OK-1 related compound groups [1] to [9]. The present disclosure has been completed and is provided by unexpectedly discovering that an OK-1 related compound group can modulate, slow the progression of, prevent, or treat a sarcopenia related disease, disorder, or symptom.

The OK-1 related compound group used in the present disclosure can be any compound included in compound groups [1] to [9], a pharmaceutically acceptable salt, or a solvate thereof (including, for example, hydrate). Compound groups [1] to [9] can also be referred to as OK-1 related compound groups [1] to [9].

Thus, in a preferred embodiment, the compound group of the present disclosure is compound group [1]. Compound group [1] is described in Japanese Patent No. 5530427, and the content thereof is incorporated herein by reference. The compound is, for example, a compound represented by formula (I) : 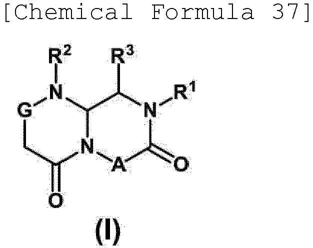 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen),
R² is -W²¹-W²²-Rb-R²⁰ (wherein W²¹ is -(CO)-, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon, or a pharmaceutically acceptable salts thereof, or a solvate thereof.

Examples of various embodiments of the compounds used in the present disclosure include any embodiment described in the section of (Explanation of compounds) or elsewhere herein or in the cited references.

In this manner, the present disclosure can modulate, slow the progression of, prevent, or treat not only sarcopenia, but also sarcopenia related diseases, disorders, and symptoms.

In one embodiment, a sarcopenia related disease, disorder, or symptom that can be modulated, slowed in terms of the progression of, prevented, or treated in the present disclosure comprises at least one selected from the group consisting of a disease, disorder, or symptom due to a muscle strength disorder; a disease, disorder, or symptom due to an energy metabolism disorder; and a disease, disorder, or symptom due to a secretory disorder.

In a specific embodiment, examples of a sarcopenia related disease, disorder, or symptom include a sarcopenia associated life-style disease (non-wasting disease), a wasting disease, a tumor associated disease (including cancer cachexia), a motor disease, a neurodegenerative disease (including ALS), cognitive dysfunction, and other associated disorders (defined as including undernutrition, frailty, spinal cord injury, inactivity, reduced activity, disuse syndrome, trauma, invasive surgery, etc.), etc. The examples thereof include, but are not limited to, respiratory diseases (respiratory system dysfunction, etc.), visceral diseases (or urinary system diseases) (chronic kidney disease (CKD), etc.), bone diseases (osteoporosis, etc.), muscular diseases (dystrophy, myogenic fibrotic myopathy, ALS, etc.), etc.

Examples of disease, disorder, or symptom targeted by the present disclosure include, but are not limited to, diseases, disorders, or symptoms associated with a digestive system (digestive tract), a circulatory system, a respiratory system (include vocal organ), a urinary system, a reproductive system, an endocrine system, a sensory nervous system, a nervous system, a locomotor system (bone, joint, ligament, muscle, etc.), and neoplasm (cancer, tumor, etc.) system, which can be directly or indirectly associated with sarcopenia through muscle strength, energy metabolism, or secretory system.

In another aspect, the present disclosure provides a composition, method, compound, or use for modulating, slowing the progression of, preventing, or treating a senescence related disease, disorder, or symptom, comprising at least one of an OK-1 related compound group.

In one embodiment, a senescence related disease, disorder, or symptom is selected from sarcopenia, a lifestyle disease (non-wasting disease), a wasting disease, a motor disease, a neurodegenerative disease, cognitive dysfunction, and other associated disorders (same as those described above). In yet another embodiment, a senescence related disease, disorder, or symptom is selected from sarcopenia, dementia, a metabolic disease, rheumatoid arthritis, osteoporosis, and reduced muscle mass.

In one embodiment, the OK-1 related compound group used in the present disclosure is compound group [1].

In another embodiment, the OK-1 related compound group used in the present disclosure can be orally administrated.

### (Sarcopenia related gene, screening, and companion diagnosis/treatment)

In some embodiments, modulation, slowing of progression, prevention, or treatment may be confirmed by modulation of at least one gene selected from the group consisting of Ppara, Pou2f2, Foxo1, Pparg, Pou2f1, Trp63, Sp1, Tcf3, Zebl, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkbl, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

In one aspect, the present disclosure provides a composition for modulating, slowing the progression of, preventing, or treating a senescence related disease, disorder, or symptom, comprising at least one of an OK-1 related compound group. In one embodiment, a senescence related disease, disorder, or symptom is selected from sarcopenia, a lifestyle disease (non-wasting disease), a wasting disease, a motor disease, a neurodegenerative disease, cognitive dysfunction, and other associated disorders (any disorder defined as including undernutrition, frailty, spinal cord injury, inactivity, reduced activity, disuse syndrome, trauma, invasive surgery, etc.). In a specific embodiment, a senescence related disease, disorder, or symptom is selected from sarcopenia, dementia, a metabolic disease, rheumatoid arthritis, osteoporosis, and reduced muscle mass.

In one aspect, the present disclosure provides a composition for use in diagnosis or testing for a sarcopenia related disease, disorder, or symptom of a subject, wherein the composition comprises means or a reagent for identifying the presence/absence or level of expression of at least one gene selected from the group consisting of a treatment gene group. In some embodiments, a treatment gene group comprises Ppara, Pou2f2, Foxo1, Pparg, Pou2f1, Trp63, Sp1, Tcf3, Zebl, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtpl, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosll, Nfkbl, Smad3, Srf, Junb, Runx2, Ets2, Etv4, Tgfbl, Tgfblil, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, Il33, Nbll, Gm26910, Pzp, 4933406L23Rik, Fblnl, Mfap5, Cnnl, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

In one embodiment, a treatment gene group can be classified in the following manner.
Immune response related gene group
   Lax1, Prg4, Ifi213, Rasgrp1, Thbs1, B2m, Sla2, Nfatc2, Tnfsf10, Cd5l, S100a8, S100a9, Gbp7, Gbp3, Gbp2, Lck, Cd36, Jchain, Cxcl9, Sh2b2, Lep, Trbc1, Trbc2, Igkv14-111, Igkv4-53, Igkv5-39, Igkv8-28, Igkv8-27, Igkv3-7, Cd8b1, Cd8a, Pparg, Clec4d, Ercc1, Cd79a, Ffar2, Cd22, Cd19, Ifitm1, Nlrc5, Cd3d, Cadm1, Eomes, Raetle, H60b, Vsir, Cfd, Itk, Irf1, Irgm2, Ccl5, Skap1, Ccr7, Cd79b, Rsad2, Ighg3, Ighd, Ighm, Ighv5-4, Ighv2-3, Ighv11-1, Ighv9-3, Ighv10-3, Ighv1-76, H2bc12, Cxcl14, Ptk2b, Il7r, Tnfrsf13c, Mx2, Fpr2, H2-K1, Tap2, H2-Eb2, Ltb, H2-D1, H2-Q6, H2-Q7, H2-T23, Cnpy3, Aqp4, Gpr17, Iigp1, Ms4a1, Cd274, and Ifit3
Outer plasma membrane related gene group
   Sell, Thbs1, B2m, Cd2, Lepr, Cd36, Cxcl9, Trbc1, Trbc2, Cd8b1, Cd8a, Cd79a, Cd19, Itgax, Cd3d, Raetle, H60b, Vsir, Atp1b2, Ccr7, Cd79b, Ighg3, Ighd, Ighv5-4, Ighv2-3, Ighv11-1, Ighv9-3, Ighv10-3, Ighv1-76, Ctsb, Il7r, Tnfrsf13c, Abcg1, H2-K1, H2-D1, H2-Q6, H2-Q7, H2-T23, Aqp4, Ms4a1, and Cd274
External organism stimulation response related gene group Fmol, Ifi213, Rasgrp1, B2m, Pck1, Fabp4, S100a8, S100a9, Gbp7, Gbp3, Gbp2, Cd36, Jchain, Cxcl9, Lep, Trbc1, Trbc2, Cd8a, Pparg, Clec4d, Mgst1, Ffar2, Thrsp, Itgax, Cyp2e1, Ifitml, Lpl, Nlrc5, Acp5, Cadm1, Raetle, H60b, Gja1, Cfd, Irf1, Irgm2, Ccl5, Ccr7, Cd79b, Rsad2, Dgkb, Tmem229b, Ifi27l2a, Ighg3, Ighd, Ighm, Ighv5-4, Ighv2-3, Ighv11-1, Ighv9-3, Ighv10-3, Ighv1-76, H2bc12, Cxcl14, Clu, Adipoq, Mx2, Fpr2, H2-K1, Tap2, H2-Q7, H2-T23, Cnpy3, Lrg1, Aqp4, Iigp1, Ms4a1, Cd274, Ifit3, and Car5b
Positive intracellular modulation related gene group
   Hdac4, En1, Rgs2, Glul, Sell, Lpgat1, G0s2, Ccdc3, Tnfaip6, Creb3l1, Fmn1, Rasgrp1, Thbs1, B2m, Tpx2, Sla2, Nnat, Fam83d, Chd6, Nfatc2, Pck1, Cdh4, Fabp4, Car2, Tnfsf10, Sh3d19, Cd5l, S100a8, S100a9, Cd2, Casq2, Lhx8, Pax5, Klf4, Epb41l4b, Lepr, Rab3b, Lck, Sfn, Slc30a2, Pla2g5, Pax7, Cd36, Cytl1, Klb, Rhoh, Cxcl9, Tbx3, Kdm2b, Sh2b2, Asns, Lep, Ptn, Trbc1, Trbc2, Epha1, Cd8a, Ghrl, Pparg, Clec4d, Ercc1, Ffar2, Cebpa, Ccne1, Atf5, Tenm4, Dgat2, Stard10, Mical2, Spon1, Cd19, Itgax, Ifitm1, Pnpla2, Retn, Adrb3, Lpl, Comp, Slc27a1, Nlrc5, Nqo1, Zfp821, Mmp3, Panx1, Dnmt1, Nrgn, Cd3d, Cadm1, Pou2af1, Slc51b, Zic1, Eomes, Cited2, Raetle, Tpd52l1, Vgll2, Gja1, Vsir, Aifm2, Lrrtm3, Shc2, Spic, Socs2, Wif1, Cdk2, Il9r, Irfl, Irgm2, Atp1b2, Inpp5k, Cryba1, Ccl5, Hoxb3, Skap1, Ccr7, Aoc3, Rsad2, Egln3, Wars, Ighg3, Ighd, Ighv5-4, Ighv2-3, Ighv11-1, Ighv9-3, Ighv10-3, Ighv1-76, Rnf144b, Cxcl14, Thbs4, Pik3r1, Tspan14, Clu, Ptk2b, Htr2a, Slc1a3, Il7r, Ptp4a3, Gpt, Mrtfa, Tnfrsf13c, Fbln1, Glis2, Adipoq, Trp63, Robo1, Tiam1, Smoc2, Fpr2, Abcg1, H2-K1, Tap2, Ltb, H2-D1, H2-Q6, H2-Q7, H2-T23, Lrg1, Mib1, Gpr17, Cd274, Fgfbp3, Pitx3, Gsto1, Nutf2-ps1, Hdac8, and Gpr174
Leucocyte activation related gene group
   Lax1, Cr2, Rasgrp1, Thbs1, B2m, Sla2, Nfatc2, Cd2, Lepr, Lck, Rhoh, Lep, Trbc1, Trbc2, Cd8a, Clec4d, Ercc1, Cd79a, Cd22, Cebpa, Cd19, Cd3d, Eomes, Gjal, Vsir, Itk, Irf1, Ccl5, Ccr7, Cd79b, Rsad2, Ighg3, Ighd, Ighv5-4, Ighv2-3, Ighv11-1, Ighv9-3, Ighv10-3, Ighv1-76, Pik3r1, Clu, Ptk2b, Il7r, Tnfrsf13c, Fpr2, H2-T23, Ms4a1, and Cd274
Keratinization related gene group
   Pkpl, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, and Lipm
Developmental biology related gene group
   Pkpl, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Cacng4, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, and Lipm Estrogen signaling pathway related gene group Krt25, Krt26, Krt27, Krt28, Krt10, Krt23, Krt39, Krt40, Krt33a, Krt33b, Krt34, Krt31, Krt32, Krt35, Krt36, Krt15, Krt14, Krt17, Calml3, Calm4, and Calm5 Desmosome related gene group Pkpl, Pkp3, Perp, Evpl, Dsp, Dsc3, Dsc2, Dsc1, Dsg1a, Dsg1b, Dsg4, and Pof1b Extracellular structure tissue related gene group Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, and Lox Collagen bond related gene group Ddr2, Dpp4, Thbs1, Ctsk, Ctss, Pcolce, Antxr1, Serpinh1, Comp, Mmp12, Pcolce2, Col6a2, Col6a1, Dcn, Lum, Aebp1, Sparc, C1qtnf1, Nid1, Ecm2, Aspn, Thbs4, Abi3bp, Tnxb, and Lox Vascular system development related gene group Col3a1, Fn1, Cfh, Cd34, Thbs1, Rin2, Ptgis, Svep1, Lepr, Angptl7, Emilin1, Pdgfra, Pf4, Col1a2, Antxr1, C3ar1, Gpr4, Tgfbl, Nr2f2, Anpep, Itgax, Comp, Hmox1, Mmp2, Dnmt1, Loxl1, Aldh1a2, Tgfbr2, Cx3cr1, Dcn, Sparc, Pik3r6, Arhgef15, Serpinf1, Ccl2, Colla1, Grn, Itgb3, Ace, Apob, Meox2, Thbs4, Plau, Stab1, Mmp14, Loxl2, Klf5, Spry2, Itga5, Col8a1, Smoc2, Thbs2, Angptl4, Lox, Acta2, and Tnmd
Fibronectin bond related gene group
   Myoc, Thbs1, Ctsk, Ctss, Tnc, Loxl3, Ssc5d, Comp, Mmp2, Itgb3, Thbs4, Fblnl, and Igfbp6
Blood vessel development related gene group
   Col3a1, Fn1, Cfh, Cd34, Thbs1, Rin2, Ptgis, Lepr, Emilin1, Pdgfra, Pf4, Col1a2, Antxr1, C3ar1, Gpr4, Tgfb1, Nr2f2, Anpep, Itgax, Comp, Hmox1, Mmp2, Loxl1, Aldh1a2, Tgfbr2, Cx3cr1, Dcn, Sparc, Pik3r6, Serpinf1, Ccl2, Collal, Grn, Itgb3, Ace, Apob, Meox2, Thbs4, Plau, Stab1, Mmp14, Loxl2, Klf5, Spry2, Itga5, Col8a1, Smoc2, Thbs2, Angptl4, Lox, Acta2, and Tnmd
Extracellular region related gene group
   Adamts13, Cel, Cela2a, Cxcl9, Lep, Try4, Igkv1-122, Igkv15-103, Igkv10-96, Igkv4-70, Igkv6-20, Igkv3-5, Dmbt1, Ctrb1, Ighv5-6, Ighv5-9, Ighv5-15, Ighv1-19, Ighv1-34, Ighv1-80, Tnfsf11, Slurp1, Apol7e, Cdsn, Dsc3, and Crtac1
Plasma heme removal related gene group
   Igkv1-122, Igkv10-96, Ighv5-6, Ighv5-9, Ighv5-15, and Apol7e

In a preferred embodiment, a keratinization related gene group and an extracellular structure tissue related gene group can be used as a treatment gene group.

In one aspect, the present disclosure provides a composition for modulating, slowing the progression of, preventing, or treating a sarcopenia related disease, disorder, or symptom, comprising a substance or agent for modulating at least one gene selected from the group consisting of Ppara, Pou2f2, Foxol, Pparg, Pou2f1, Trp63, Sp1, Tcf3, Zebl, Srebfl, Sp3, Ebfl, Mycn, Pbxl, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebfl, Jun, Stat3, Sp3, Myf6, Nr3c1, Foxo3, Nfe2l2, Myf5, Ahr, Ep300, Myog, and Usf2.

In another aspect, the present disclosure provides a method for screening a substance or agent for modulating, slowing the progression of, preventing, or treating a sarcopenia related disease, disorder, or symptom, the method comprising:
A) administering a candidate substance or agent to a subject;
B) checking modulation of at least one gene selected from the group consisting of Ppara, Pou2f2, Foxol, Pparg, Pou2f1, Trp63, Sp1, Tcf3, Zeb1, Srebfl, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkpl, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfbl, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject; and
C) identifying the candidate substance or agent as a substance or agent that can have an ability to modulate, slow the progression of, prevent, or treat a sarcopenia related disease, disorder, or symptom based on a result of B) .

### (General description of a medicament, therapeutic method, etc.)

The compound group, compound, medicament, composition, etc. of the present disclosure can be administered via any dosing form, which is preferably, but not limited to, oral administration.

Anticipated "subject" of administration includes, but are not limited to, humans (i.e., male or female in any age group such as pediatric subjects (e.g., infant, child, and adolescent) and adult subjects (young, middle-aged, and elderly adults)) and/or other non-human animals such as mammals (e.g., primates (e.g., cynomolgus monkey and rhesus monkey), commercially utilized mammals such as cows, pigs, horses, sheep, goats, cats, and/or dogs) and avians (e.g., commercially utilized avians such as chickens, ducks, geese, and/or turkeys), reptiles, amphibians, and fish. In a certain embodiment, a non-human animal is a mammal. A non-human animal can be a male or female at any period of development. A non-human animal can be a transgenic animal.

As used herein, unless noted otherwise, the term "treat", "treatment", and "therapy" refers to an action performed while a subject is suffering from a disease, disorder, or symptom. As used herein, an action for reducing the severity of a disease, disorder, or symptom, or delaying or slowing the progression of a disorder is "slowing the progression", and an action for inhibiting a disease, disorder, or symptom or reducing the severity of a disorder before a subject starts suffering from a disease, disorder, or symptom is "prevention".

"Effective amount" of compound generally refers to an amount that is sufficient to elicit a desired biological response, i.e., to treat a disorder. As is obvious to those skilled in the art, the effective amount of the compound of the present disclosure can vary depending on factors such as the desired biological endpoint, pharmacokinetics of the compound, disorder to be treated, dosing form, age, health, and subject. An effective amount includes therapeutic and prophylactic treatment.

As used herein, unless specifically noted otherwise, the term "therapeutically effective amount" of a compound refers to an amount of compound that is sufficient upon administration to prevent, or alleviate to a certain extent, expression of one or more symptoms of a disease targeted for treatment. The term also refers to an amount of compound that is sufficient to elicit a biological or medical response of a biological molecule (e.g., protein, enzyme, RNA, or DNA), cell, tissue, system, animal, or human, required by a researcher, veterinarian, physician, or clinician. Furthermore, a therapeutically effective amount of a compound refers to an amount of a therapeutic drug alone or an amount combined with another therapy, which affords a therapeutic benefit in the treatment or management of a disease. The term encompasses an amount that improves the overall therapy, an amount that alleviates or evades the symptom or cause of a disease, and an amount that enhances the therapeutic efficacy of another therapeutic drug.

As used herein, unless specifically noted otherwise, a "prophylactically effective amount" of a compound is an amount sufficient for inhibiting or reducing a symptom of a disease, or for preventing recurrence of a disease. A prophylactically effective amount of a compound refers to an amount of a therapeutic drug alone or an amount combined with another agent, which affords a prophylactic benefit in the inhibition or reduction in a symptom of a disease or recurrence of a disease. The term "prophylactically effective amount" can encompass an amount that improves the overall prevention and an amount that enhances the prophylactic efficacy of another prophylactic drug.

As used herein, unless noted otherwise, "therapeutically effective amount" of a compound refers to an amount that is sufficient to provide a therapeutic effect upon treatment of a disorder or to delay or minimize one or more symptoms linked to a disorder. A therapeutically effective amount of a compound refers to an amount of therapeutic agent providing a therapeutic effect upon treatment of a disorder, individually or in combination with another therapy. The term "therapeutically effective amount" can encompass an amount that improves the overall therapy, reduces or prevents the symptom or cause of a disorder, or enhances the therapeutic efficacy of another therapeutic agent.

As used herein, unless noted otherwise, "prophylactically effective amount" of a compound is an amount that is sufficient to prevent a disorder or one or more symptoms linked to a disorder, or to prevent the recurrence thereof. A therapeutically effective amount of a compound refers to an amount of a therapeutic agent that provides a prophylactic effect upon prevention of a disorder, individually or in combination with another agent. The term "prophylactically effective amount" can encompass an amount that improves the overall prevention or enhances the prophylactic efficacy of another prophylactic agent.

Exemplary diseases, disorders, or symptoms are described elsewhere herein.

Since sarcopenia is associated with a muscle, sarcopenia is also associated with muscle strength, energy metabolism, nervous system, and secretory organs associated with a muscle. In one embodiment, sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder. In some embodiments, a sarcopenia related disease, disorder, or symptom comprises at least one selected from the group consisting of primary sarcopenia, secondary sarcopenia, and a disease, disorder, or symptom accompanied therewith. In another embodiment, a sarcopenia related disease, disorder, or symptom comprises at least one selected from the group consisting of a sarcopenia associated life-style disease (non-wasting disease), a wasting disease, a tumor associated disease (including cancer cachexia), a motor disease, a neurodegenerative disease (including ALS), cognitive dysfunction, and other associated disorders (any disorder defined as including undernutrition, frailty, spinal cord injury, inactivity, reduced activity, disuse syndrome, trauma, invasive surgery, etc.). In another embodiment, a sarcopenia related disease, disorder, or symptom is of a system selected from the group consisting of a digestive system (digestive tract, etc.), a circulatory system, a respiratory system (vocal organ, etc.), a urinary system, a reproductive system, an endocrine system, a sensory nervous system, a cranial nervous system, and a locomotor system (bone, joint, ligament, muscle, etc.).

Examples of a disease, disorder, or symptom due to a muscle strength disorder that can be modulated, slowed in terms of the progression, prevented, or treated in a specific embodiment include neurogenic muscular atrophy, myogenic muscular atrophy (muscular dystrophy, congenital myopathy, mitochondrial encephalomyopathy, myopathy due to congenital metabolic disorder, etc.), etc.

Examples of a disease, disorder, or symptom due to an energy metabolism disorder that can be modulated, slowed in terms of the progression, prevented, or treated in a specific embodiment include diabetes, dyslipidemia, obesity, metabolic syndrome, osteoporosis, fatty liver, hyperuricemia, hypertension, etc.

Examples of a disease, disorder, or symptom due to a secretory disorder that can be modulated, slowed in terms of the progression, prevented, or treated in a specific embodiment include diabetes, pancreatitis, ulcerative colitis, diarrhea, etc.

Examples of a disease, disorder, or symptom due to a nervous system disorder that can be modulated, slowed in terms of the progression, prevented, or treated in a specific embodiment include a neurodegenerative disease (including ALS), cognitive dysfunction, etc.

In another embodiment, sarcopenia related disease, disorder, or symptom can also be divided into primary and secondary forms.

The sarcopenia related disease, disorder, or symptom comprises at least one selected from the group consisting of primary sarcopenia, secondary sarcopenia, and a disease, disorder, or symptom accompanied therewith.

Examples of a disease, disorder, or symptom accompanied with primary sarcopenia and/or secondary sarcopenia that can be modulated, slowed in terms of the progression, prevented, or treated in a specific embodiment include bedridden, inactive life style, poor physical condition, severe organ failure, inflammatory disease, malabsorption, digestive tract disease, anorexia, etc.

In a specific embodiment, the compound of the present disclosure may be selected from compound group [1]. In a preferred embodiment, the compound of the present disclosure or a pharmaceutically acceptable salt thereof or a solvate thereof can be orally administrated. In another embodiment, the composition of the present disclosure may be administered in combination with at least one of physical therapy, dietary therapy, and other drug therapy. In a specific embodiment, the composition of the present disclosure may be administered in combination with physical therapy. In a specific embodiment, modulation, slowing of progression, prevention, or treatment can comprise modulation, slowing of progression, prevention, or treatment in at least one of reduced muscle mass, reduced (muscular) endurance, reduced short-term memory, reduced bone density, and osteoporosis. In a specific embodiment, modulation, slowing of progression, prevention, or treatment can comprise modulation, slowing of progression, prevention, or treatment in at least one of reduced muscle mass and reduced (muscular) endurance.

The route of administration of the compound, medicament, composition, etc. of the present disclosure can be oral administration, parenteral administration, or rectal administration. The daily dosage thereof varies by the type of compound, administration method, patient's symptom or age, etc. For oral administration, generally about 0.01 to 1000 mg and still more preferably about 0.1 to 500 mg per 1 kg of body weight of a human or mammal can be administered in one to several doses. For parenteral administration such as intravenous administration, generally about 0.01 mg to 300 mg and still more preferably about 1 mg to 100 mg per 1 kg of body weight of a human or mammal can be administered.

While the descriptions of pharmaceutical compositions provided herein are primarily directed to pharmaceutical compositions for administration to humans, said compositions are generally suitable for administration to any type of animals, as is obvious to those skilled in the art. Alteration of a pharmaceutical composition for administration to various animals is sufficiently understood. Generally, veterinary pharmacologists can design and/or practice such modification by at most a simple general experiment.

The compound, medicament, composition, etc. of the present disclosure can be administered directly, or as a formulation using a suitable dosage form, by oral or parenteral administration. Examples of the dosage form include, but are not limited to, tablets, capsules, powder, granules, liquid agents, suspension, injections, patches, poultice, etc. The formulations are manufactured by a known method using a pharmaceutically acceptable additive. As the additive, an excipient, disintegrant, binding agent, fluidizer, lubricating agent, coating agent, solubilizing agent, solubilization promotor, thickener, dispersant, stabilizer, sweetener, flavoring agent, etc. can be used depending on the objective. Specific examples thereof include lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, etc.

As used herein, a pharmaceutically acceptable additive includes any or all solvents, dispersants, diluents, or other liquid base agents, dispersant or solubilization promotors, surfactants, isotonizing agents, thickening or emulsifying agents, preservatives, solid binding agents, lubricating agents, and similar in a manner that is suitable for a specific desired dosage form. Remington's The Science and Practice of Pharmacy, 21st Edition, A. R. Gennaro, (Lippincott, Williams & Wilkins, Baltimore, MD, 2006) discloses various additives used in formulating a pharmaceutical composition and a known technology for the preparation thereof. Use of any conventional carrier is understood to be within the scope of the present disclosure, except when incompatible with a certain substance or a derivative thereof (e.g., due to generation of any undesirable biological effect, or alternatively, due to an adverse interaction with any other component(s) of a pharmaceutical composition).

In some embodiments, a pharmaceutically acceptable additive is at least 95%, 96%, 97%, 98%, 99%, or 100% pure. In some embodiments, an additive is approved for use in humans and veterinary use. In some embodiments, an additive is approved by the US Food and Drug Administration. In some embodiments, an additive is of a pharmaceutical grade. In some embodiments, an additive meets the standards of the US Pharmacopoeia (USP), European Pharmacopoeia (EP), British Pharmacopoeia, Japanese Pharmacopoeia (JP) and/or International Pharmacopoeia.

Examples of a pharmaceutically acceptable additive used in the manufacture of a pharmaceutical composition include, but are not limited to, inert diluents, dispersants and/or granulating agents, surfactants and/or emulsifiers, disintegrants, binding agents, preservatives, buffer, lubricating agents, and/or oils. Such an additive can be optionally contained in the formulation of the present disclosure. An additive such as cocoa butter, suppository wax, coloring agent, coating agent, sweetener, flavoring agent, or fragrance can be in a composition in accordance with the judgment of the manufacturer.

Exemplary diluents include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dried starch, corn starch, powdered sugar, and combination thereof.

Exemplary granulating agents and/or dispersants include, but are not limited to, potato starch, corn starch, tapioca starch, sodium starch glycolate, clay, alginic acid, guar gum, citrus pulp, agar, bentonite, cellulose and wood products, natural sponge, cation exchange resin, calcium carbonate, silicate, sodium carbonate, cross-linked polyvinyl pyrrolidone (crospovidone), sodium carboxymethyl starch (sodium starch glycolate), carboxymethyl cellulose, internally crosslinked sodium carboxymethyl cellulose (croscarmellose), methylcellulose, gelatinized starch (starch 1500), microcrystalline starch, water-insoluble starch, calcium carboxymethyl cellulose, aluminum magnesium silicate (Veegum), sodium lauryl sulfate, quaternary ammonium compound, and combinations thereof.

Exemplary surfactants and/or emulsifiers include, but are not limited to, natural emulsifiers (e.g., acacia, agar, alginic acid, sodium alginate, tragacanth, chondrux, cholesterol, xanthan, pectin, gelatin, egg yolk, casein, wool fat, cholesterol, wax, and lecithin), colloidal clay (e.g., bentonite [aluminum silicate] and Veegum [magnesium aluminum silicate]), long-chain amino acid derivatives, high molecular weight alcohol (e.g., stearyl alcohol, cetyl alcohol, oleyl alcohol, triacetin monostearate, ethylene glycol distearate, glyceryl monostearate, propylene glycol monostearate, and polyvinyl alcohol), carbomers (carboxypolymethylene, polyacrylic acid, acrylic acid polymers, and carboxyvinyl polymers), carrageenan, cellulose derivatives (e.g., sodium carboxymethyl cellulose, powdered cellulose, hydroxymethyl cellulose, hydroxypropyl cellulose, hydroxypropyl methylcellulose, and methylcellulose), sorbitan fatty acid esters (e.g., polyoxyethylene sorbitan monolaurate [Tween-20], polyoxyethylene sorbitan [Tween-60], polyoxyethylene sorbitan monooleate [Tween-80], sorbitan monopalmitate [Span-40], sorbitan monostearate [Span-60], sorbitan tristearate [Span-65], glyceryl monooleate, and sorbitan monooleate [Span-80]), polyoxyethylene esters (e.g., polyoxyethylene monostearate [Myrj-45], polyoxyethylene hydrogenated castor oil, polyethoxylated castor oil, polyoxymethylene stearate, and Solutol), sucrose fatty acid esters, polyethylene glycol fatty acid esters (e.g., Cremophor), polyoxyethylene ethers (e.g., polyoxyethylene lauryl ether [Brij-30]), polyvinylpyrrolidone, diethylene glycol monolaurate, triethanolamine oleate, sodium oleate, potassium oleate, ethyl oleate, oleic acid, ethyl laurate, sodium lauryl sulfate, Pluronic^{®} F68, poloxamer 188, cetrimonium bromide, cetylpyridinium chloride, benzalkonium chloride, docusate sodium, and/or combinations thereof.

Exemplary binding agents include, but are not limited to, starch (e.g., corn starch and starch paste), gelatin, saccharide (e.g., sucrose, glucose, dextrin, molasses, lactose, lactitol, and mannitol), natural and synthetic gum (e.g., acacia, sodium alginate, Irish moss extract, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone, magnesium aluminum silicate (Veegum), and larch arabogalactan), alginates, polyethylene oxide, polyethylene glycol, inorganic calcium salts, silicic acid, polymethacrylates, waxes, water, alcohol, and mixtures thereof.

Exemplary preservatives can include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium pyrosulfite, propionic acid, propyl gallate, sodium ascorbate, sodium hydrogen sulfite, sodium pyrosulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium hydrogen sulfite, sodium pyrosulfite, potassium sulfite, Glydant Plus, Phenonip, methylparaben, Germall 115, Germaben II, Neolone, Kathon, and Euxyl. In one embodiment a preservative is an antioxidant. In another embodiment, a preservative is a chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium glucoheptonate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

Exemplary oils include, but are not limited to, almond, apricot kernel, avocado, babassu, bergamot, black currant seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary synthetic oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and mixtures thereof.

Liquid dosage forms for oral and parenteral administration include, but are not limited to, pharmaceutically acceptable emulsions, microemulsions, solutions, suspensions, syrups, and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (especially cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring agents, and fragrances. In certain embodiments for parenteral administration, the conjugates of the present disclosure are mixed with solubilizing agents such as Cremophor, alcohols, oils, modified oils, glycols, polysorbates, cyclodextrins, polymers, and mixtures thereof.

Injectable preparations, for example, sterile injectable aqueous or oleaginous suspensions can be formulated according to the known art using suitable dispersing or wetting agents and suspending agents. The sterile injectable preparation can be a sterile injectable solution, suspension, or emulsion in a nontoxic parenterally acceptable diluent or solvent, such as a solution in 1,3-butanediol. Among the acceptable vehicles and solvents that can be employed are water, Ringer's solution (USP), and isotonic sodium chloride solution. In addition, sterile, nonvolatile oils are conventionally employed as a solvent or suspending medium. For this purpose, any non-stimulating nonvolatile oil can be employed including synthetic mono- or di-glycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions (which can be dissolved or dispersed in sterile water or other sterile injectable medium prior to use) .

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution, which, in turn, may depend upon crystal size and crystalline form. Alternatively, delayed absorption of a parenterally administered drug form may be accomplished by dissolving or suspending the drug in an oil vehicle.

Compositions for rectal or vaginal administration are typically suppositories which can be prepared by mixing the conjugates of the present disclosure with non-irritating additives or carriers (e.g., cocoa butter, polyethylene glycol, or a suppository wax) which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable additive or carrier such as sodium citrate or dicalcium phosphate and/or (a) fillers or extenders such as starches, lactose, sucrose, glucose, mannitol, and silicic acid, (b) binding agents such as carboxymethylcellulose, alginic acid, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as cetyl alcohol and glycerol monostearate, (h) absorbents such as kaolin and bentonite clay, and (i) lubricating agents such as talc, calcium stearate, magnesium stearate, solid polyethylene glycols, sodium lauryl sulfate, and mixtures thereof. In the case of capsules, tablets, and pills, the dosage form may include a buffering agent.

Solid compositions of a similar type can be employed as fillers in soft and hard-filled gelatin capsules using additives such as lactose or milk sugar as well as high molecular weight polyethylene glycols and the like. The solid dosage forms of tablets, dragees, capsules, pills, and granules can be prepared with coatings and shells such as enteric coatings and other coatings well known in the art of pharmacology. They may optionally comprise opacifying agents and can be a composition that releases the active ingredient(s) only, or preferentially, in a certain part of the intestinal tract, optionally, in a delayed manner. Examples of encapsulating compositions which can be used include polymeric substances and waxes. Solid compositions of a similar type can be employed as fillers in soft and hard-filled gelatin capsules using additives such as lactose or milk sugar as well as high molecular weight polyethylene glycols and the like.

The active ingredient can be in a micro-encapsulated form with one or more additives described above. The solid dosage forms of tablets, dragees, capsules, pills, and granules can be prepared with coatings and shells such as enteric coatings, release controlling coatings, and other coatings well known in the pharmaceutical formulating art. In such solid dosage forms, the active ingredient can be admixed with at least one inert diluent such as sucrose, lactose, or starch. Such dosage forms can comprise, as is normal practice, additional substances other than inert diluents, such as tableting lubricants and other tableting aids, magnesium stearate and microcrystalline cellulose. In the case of capsules, tablets, and pills, the dosage forms can comprise buffering agents. They can optionally comprise opacifying agents and can be a composition that releases the active ingredient(s) only, or preferentially, in a certain part of the intestinal tract, optionally, in a delayed manner. Examples of encapsulating agents which can be used include polymeric substances and waxes.

Dosage forms for topical and/or transdermal administration of a compound group, salt, solvate, or composition of the present disclosure can include ointments, pastes, creams, lotions, gels, powders, solutions, sprays, inhalants, and/or patches. Generally, the active ingredient can be admixed under sterile conditions with a pharmaceutically acceptable carrier and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively or additionally, the rate can be controlled by providing a rate controlling membrane and/or by dispersing the active ingredient in a polymer matrix and/or gel.

Suitable devices for use in delivering intradermal pharmaceutical compositions described herein include short needle devices such as those described in US Patent Nos. 4,886,499, 5,190,521, 5,328,483, 5,527,288, 4,270,537, 5,015,235, 5,141,496, and 5,417,662. Intradermal compositions can be administered by devices which limit the effective penetration length of a needle into the skin, such as those described in PCT publication WO 99/34850 and functional equivalents thereof. Jet injection devices are described, for example, in US Patent Nos. 5,480,381, 5,599,302, 5,334,144, 5,993,412, 5, 649, 912, 5,569,189, 5,704,911, 5,383,851, 5,893,397, 5,466,220, 5,339,163, 5,312,335, 5,503,627, 5,064,413, 5,520,639, 4,596,556, 4,790,824, 4,941,880, and 4,940,460, and PCT publications WO 97/37705 and WO 97/13537. Alternatively or additionally, conventional syringes can be used in the classical mantoux method of intradermal administration.

Formulations for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations can, for example, comprise from about 1% to about 10% (w/w) active ingredient, although the concentration of the active ingredient can be as high as the solubility limit of the active ingredient in the solvent. Formulations for topical administration can further comprise one or more of the additional ingredients described herein.

A pharmaceutical composition of the present disclosure can be prepared, packaged, and/or sold in a formulation for pulmonary administration via the oral cavity. Such a formulation can comprise dry particles which comprise the active ingredient and have a diameter in the range from about 0.5 to about 7 nanometers, or from about 1 to about 6 nanometers. Such compositions are conveniently in the form of dry powders for administration using a device comprising a dry powder reservoir (to which a stream of propellant can be directed to disperse the powder) and/or using a self-propelling solvent/powder dispensing container (such as a device comprising the active ingredient dissolved and/or suspended in a low-boiling propellant in a sealed container) . Such powders comprise particles wherein at least 98% of the particles by weight have a diameter greater than 0.5 nanometers and at least 95% of the particles by number have a diameter less than 7 nanometers. Alternatively, at least 95% of the particles by weight have a diameter greater than 1 nanometer and at least 90% of the particles by number have a diameter less than 6 nanometers. Dry powder compositions can include a solid fine powder diluent such as sugar and are conveniently provided in a unit dose form.

Low boiling propellants generally include liquid propellants having a boiling point of below 65°F at atmospheric pressure. Generally, the propellant can constitute 50 to 99.9% (w/w) of the composition, and the active ingredient can constitute 0.1 to 20% (w/w) of the composition. The propellant can further comprise additional ingredients such as a liquid non-ionic and/or solid anionic surfactant and/or a solid diluent (which can have a particle size of the same order as particles comprising the active ingredient).

Pharmaceutical compositions of the present disclosure formulated for pulmonary delivery can provide the active ingredient in the form of droplets of a solution and/or suspension. Such formulations can be prepared, packaged, and/or sold as aqueous and/or dilute alcoholic solutions and/or suspensions, optionally sterile, comprising the active ingredient, and can conveniently be administered using any nebulization and/or atomization device. Such formulations can further comprise one or more additional ingredients including, but not limited to, a flavoring agent such as saccharin sodium, a volatile oil, a buffering agent, a surfactant, and/or a preservative such as methylhydroxybenzoate. The droplets provided by this route of administration can have an average diameter in the range from about 0.1 to about 200 nanometers.

Formulations described herein as being useful for pulmonary delivery are useful for intranasal delivery of a pharmaceutical composition of the present disclosure. Another formulation for intranasal administration is a coarse powder comprising the active ingredient and having an average particle from about 0.2 to 500 micrometers. Such a formulation is administered by rapid inhalation through the nasal passage from a container of the powder held close to the nares in the same manner as inhaling snuff.

Formulations for nasal administration may, for example, comprise from about as little as 0.1% (w/w) to as much as 100% (w/w) of the active ingredient, and may comprise one or more of the additional ingredients described herein. A pharmaceutical composition of the present disclosure can be prepared, packaged, and/or sold in a formulation for buccal administration. Such formulations may, for example, be in the form of tablets and/or lozenges made using conventional methods, and may contain, for example, 0.1 to 20% (w/w) active ingredient, the balance comprising an orally dissolvable and/or degradable composition and, optionally, one or more of the additional ingredients described herein. Alternately, formulations for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 to about 200 nanometers, and may further comprise one or more of the additional ingredients described herein.

A pharmaceutical composition of the present disclosure can be prepared, packaged, and/or sold in a formulation for ophthalmic administration. Such formulations may, for example, be in the form of eye drops including, for example, a 0.1/1.0% (w/w) solution and/or suspension of the active ingredient in an aqueous or oily liquid carrier. Such drops may further comprise buffering agents, salts, and/or one or more other of the additional ingredients described herein. Other opthalmically-administrable formulations which are useful include those which comprise the active ingredient in microcrystalline form and/or in a liposomal preparation. Ear drops and/or eye drops are also contemplated as being within the scope of the present disclosure.

General considerations in the formulation and/or manufacture of a pharmaceutical agent can be found in, for example, Remington: The Science and Practice of Pharmacy 21st ed., Lippincott Williams & Wilkins, 2005.

In some embodiments, the composition of the present disclosure can be administered to a patient for at least 2 weeks, at least 3 weeks, at least 4 weeks, at least 5 weeks, at least 6 weeks, at least 7 weeks, or at least 8 weeks. In a specific embodiment, the composition of the present disclosure can be administered to a patient for at least 4 weeks. Typically, an effect of sarcopenia can be evaluated in about 4 weeks.

The pharmaceutical composition described herein can be prepared by any method that is known in the art of pharmacology or any method that may be developed in the future. In general, such a preparation method comprises steps of combining the active ingredient with an additive and/or one or more other supplementary components, and if needed and/or desired, molding and/or packaging the product in a desired single or multiple dose units.

The pharmaceutical composition of the present disclosure can be prepared, packaged, and/or sold in bulk, as a single unit dose and/or a plurality of units of single doses. As used herein, "unit dose" is an individual amount of a pharmaceutical composition containing a given amount of an active ingredient. The amount of active ingredient is generally equivalent to the dosage of active ingredient administered to a subject and/or a convenient fraction of said dosage (e.g., 1/2 or 1/3 of said dosage). The relative amounts of the active ingredient, pharmaceutically acceptable additive, and/or any additional component in the pharmaceutical composition of the present disclosure vary depending on the background, size, and/or disorder of the subject of treatment, as well as on the route of administration of the composition. For example, a composition can comprise 0.1% to 100% (w/w) of active ingredient.

The compound group of the present disclosure is typically formulated in a dosage unit form for ease of administration and uniformity of dosage. It will be understood, however, that the total daily usage of the compositions of the present invention will be decided by the attending physician within the scope of sound medical judgment. The specific therapeutically effective dose level for any particular subject will depend upon a variety of factors including the disease or disorder being treated and the severity of the disorder; the activity of the specific active ingredient employed; the age, body weight, general health, sex, and diet of the subject; the time of administration, route of administration, and rate of excretion of the specific active ingredient employed; the duration of the treatment; drugs used in combination or coincidental with the specific active ingredient employed; and like factors well known in the medical arts.

The compound group of the present disclosure, a pharmaceutically acceptable salt thereof, a solvate thereof, medicament, etc. can be administered through any route. In some embodiments, the polypeptide of formula (II), a salt thereof, or a pharmaceutical composition thereof is administered through various routes (including oral, intravenous, intramuscular, intra-arterial, intramedullary, intrathecal, subcutaneous, intraventricular, transdermal, intradermal, rectal, intravaginal, intraperitoneal, topical (as by powders, ointments, creams, and/or drops), mucosal, nasal, bucal, sublingual; by intratracheal instillation, bronchial instillation, and/or inhalation; and/or as an oral spray, nasal spray, and/or aerosol). Specifically contemplated routes are systemic intravenous injection, regional administration via blood and/or lymph supply, and/or direct administration to an affected site. In general, the most appropriate route of administration will depend upon a variety of factors including the nature of the agent (e.g., its stability in the environment of the gastrointestinal tract), and/or the disorder of the subject (e.g., whether the subject is able to tolerate oral administration). Currently, oral and/or nasal spray and/or aerosol routes are most commonly used for direct delivery of a therapeutic agent to the lung and/or respiratory system. However, the present disclosure encompasses delivery of the pharmaceutical composition of the present disclosure through any suitable route by considering advancement that could occur in the science of drug delivery.

The compound group, salt, solvate, and pharmaceutical composition of the present disclosure can be administered at a dosage level that is sufficient to achieve a desired therapeutic effect by delivering about 0.001 mg/kg to about 100 mg/kg, about 0.01 mg/kg to about 50 mg/kg, about 0.1 mg/kg to about 40 mg/kg, about 0.5 mg/kg to about 30 mg/kg, about 0.01 mg/kg to about 10 mg/kg, about 0.1 mg/kg to about 10 mg/kg, or about 1 mg/kg to about 25 mg/kg of the subject's body weight/day at once daily or twice or more daily. The desired dosage can be delivered three times a day, twice a day, once a day, every two days, every three days, weekly, biweekly, every three weeks, or every four weeks. In a certain embodiment, the desired dosage can be delivered through multiple administrations (e.g., 2, 3, 4, 5, 6, 7, 8, 9, 10, 11, 12, 13, 14, 15 or more administrations).

Obviously, dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult. The exact amount of a compound, salt, solvate, or composition of the present disclosure required to achieve an effective amount will vary from subject to subject, depending, for example, on species, age, and general condition of a subject, severity of the side effects or disorder, identity of the particular compound(s), mode of administration, and the like.

The present disclosure encompasses a "therapeutic cocktail" comprising the compound, salt, solvate, or pharmaceutical composition of the present disclosure. In some embodiments, the compound, salt, solvate, or pharmaceutical composition of the present disclosure comprises one type that can attach to a plurality of targets. In some embodiments, different compounds, salts, solvates, or pharmaceutical compositions of the present disclosure can comprise different types of targeting moieties, which can all attach to the same target. In some embodiments, different compounds, salts, solvates, or pharmaceutical compositions of the present disclosure can comprise different types of targeting moieties, which can all attach to different target. In some embodiments, the different targets can be associated with the same cell type. In some embodiments, the different targets can be associated with different cell types.

Obviously, the compound, salt, solvate, or pharmaceutical composition of the present disclosure can be used in combination therapy. The specific combination of therapies (therapeutic drug or treatment) used in a combined regimen will take into account the compatibility with a desired therapeutic drug and/or treatment and a desired therapeutic effect to be achieved. Obviously, the therapeutic methods used can achieve a desired effect for the same purpose (e.g., the complex of the present disclosure useful for the detection of tumor can be concomitantly administered with another agent that is useful for the detection of tumor) or can achieve different effects (e.g., controlling any one of side effects).

The pharmaceutical composition of the present disclosure can be administered alone or in combination with one or more therapeutically active agents. While "in combination with" is not intended so that agents must be administered concomitantly and/or formulated for delivery together, such methods of delivery are within the scope of the present disclosure. A composition can be administered simultaneously with, prior to, or subsequent to, one or more other desired therapeutic drugs or medical treatment. Generally, each agent can be administered at a dosage and/or time schedule determined for said agent. In addition, the present disclosure encompasses delivery of the pharmaceutical composition of the present disclosure in combination with an agent that can improve the bioavailability thereof, an agent that can reduce and/or alter the metabolism thereof, an agent that can inhibit the excretion thereof, and/or an agent that can alter the biodistribution thereof. Obviously, the compound, salt, solvate, or pharmaceutical composition of the present disclosure and a therapeutically active agent used in such a combination can be administered together as a single composition or administered separately as different compositions.

The specific combination used in a combined regimen will take into account the compatibility of a therapeutically active agent and/or treatment with the compound, salt, solvate, or pharmaceutical composition of the present disclosure and/or a desired therapeutic effect to be achieved. Obviously, the combination used can achieve a desired effect for the same disorder (e.g., the compound, salt, solvate, or pharmaceutical composition of the present disclosure can be concomitantly administered with another therapeutically active agent that is used for treating the same disorder) or can achieve different effects (e.g., controlling any one of side effects).

As used herein, "therapeutically active agent" refers to any substance used as a medicament for the treatment, prevention, slowing, reduction, or remission of a disorder, and refers to a substance that is useful in a therapy including prevention and treatment. A therapeutically active agent also encompasses, for example, a compound that enhances the effect or efficacy of another compound (e.g., in compound group [2]) by enhancing the effect or reducing a side effect of a compound, salt, solvate, or pharmaceutical composition of compound group [1] of the present disclosure.

In one embodiment, a therapeutically active agent is an anti-cancer agent, antibiotic, anti-viral agent, anti-HIV agent, anti-parasite agent, anti-protozoal agent, anesthetic, anticoagulant, inhibitor of an enzyme, steroidal agent, steroidal or non-steroidal anti-inflammatory agent, antihistamine, immunosuppressant agent, anti-neoplastic agent, antigen, vaccine, antibody, decongestant, sedative, opioid, analgesic, anti-pyretic, birth control agent, hormone, prostaglandin, progestational agent, anti-glaucoma agent, ophthalmic agent, anti-cholinergic, analgesic, antidepressant, anti-psychotic, neurotoxin, hypnotic, tranquilizer, anti-convulsant, muscle relaxant, anti-Parkinson agent, anti-spasmodic, muscle contractant, channel blocker, miotic agent, anti-secretory agent, anti-thrombotic agent, anticoagulant, anti-cholinergic, β-adrenergic blocking agent, diuretic, cardiovascular active agent, vasoactive agent, vasodilating agent, anti-hypertensive agent, angiogenic agent, modulators of cell-extracellular matrix interactions (e.g. cell growth inhibitors and anti-adhesion molecules), or inhibitors/intercalators of DNA, RNA, protein-protein interactions, or protein-receptor interactions.

The present disclosure also provides a variety of kits comprising one or more of the compound, salt, solvate, and pharmaceutical composition of the present disclosure. For example, the present disclosure provides a kit comprising a compound, salt, solvate, or pharmaceutical composition of the present disclosure and instructions for use. A kit can comprise multiple different compounds, salts, solvates, or pharmaceutical compositions of the present disclosure. A kit may comprise several additional components or reagent in any combination. All of the various combinations are not set forth explicitly but each combination is included in the scope of the present disclosure.

According to certain embodiments of the present disclosure, a kit can include, for example, (i) one or more compounds, salts, solvates, or pharmaceutical compositions of the present disclosure and, optionally, one or more particular therapeutically active agents to be delivered; and (ii) instructions for administration to a subject in need thereof.

Kits typically include instructions which may, for example, comprise protocols and/or describe diseases, disorders, or symptoms for production of the compound, salt, solvate, or pharmaceutical composition of the present disclosure, administration of the compound, salt, solvate, or pharmaceutical composition of the present disclosure to a subject in need thereof, and design of the compound, salt, solvate, or pharmaceutical composition of the present disclosure. Kits will generally include one or more vessels or containers so that some or all of the individual components and reagents may be separately housed. Kits may also include a means for enclosing individual containers in relatively close confinement for commercial sale, e.g., a plastic box, in which instructions, packaging materials such as Styrofoam^{®}, may be enclosed. An identifier, e.g., a bar code, radio frequency identification (ID) tag, may be present in or on the kit or in or one or more of the vessels or containers included in the kit. An identifier can be used, e.g., to uniquely identify the kit for purposes of quality control, inventory control, tracking, or movement between workstations.

### (Note)

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

Examples are described hereinafter.

### (Manufacturing Example 1: Manufacture of OK-1 compound)

This Example manufactured the following (OK-1, 4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid).

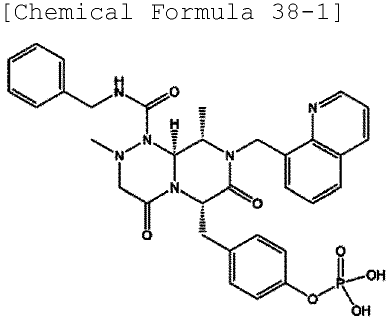

The compound described above was manufactured in accordance with the procedure described in Japanese Patent No. 5530427. Specifically, a suitable amount of dichloromethane solution of (S)-2-amino-3-(4-tert-butoxyphenyl)-N-((S)-1,1-diethoxypropan-2-yl)-N-(quinolin-8-ylmethyl)propaneamide and 4-dimethylaminopyridine was added to a suitable amount of dichloromethane solution of 2-(2-(benzylcarbamoyl)-1-methylhydrazinyl)acetic acid, hydroxybenzotriazole, and 1-ethyl-3-(3-dimethylaminopropyl)carbodiimide synthesized in accordance with the procedure in the aforementioned patent, and the mixture was stirred overnight at room temperature. The reaction mixture was diluted with ethyl acetate and washed with the same amount of saturated sodium bicarbonate, the same amount of water, and the same amount of brine. The organic phase was dried with magnesium sulfate and filtered. The filtrate was concentrated under reduced pressure. The residue was purified by Buch silica gel column chromatography (chloroform:methanol = 98:2) to obtain N-benzyl-2-(2-((S)-3-(4-tert-butoxyphenyl)-1-(((S)-1,1-diethoxypropan-2-yl) (quinolin-8-ylmethyl)amino)-1-oxopropan-2-ylamino)-2-oxoethyl)-2-methylhydrazine carboxamide (yield of approximately 58%).

To the N-benzyl-2-(2-((S)-3-(4-tert-butoxyphenyl)-1-(((S)-1,1-diethoxypropan-2-yl) (quinolin-8-ylmethyl)amino)-1-oxopropan-2-ylamino)-2-oxoethyl)-2-methylhydrazine carboxamide, 10% water/HCOOH was added, and the mixture was stirred overnight at room temperature. The reaction mixture was concentrated under reduced pressure. The residue was purified by silica gel column chromatography (chloroform:methanol = 97:3) to obtain (6S,9S)-N-benzyl-6-(4-hydroxybenzyl)-2,9-dimethyl-4,7-dioxo-8-(quinolin-8-ylmethyl)hexahydro-2H-pyrazino[2,1-c] [1,2,4]triazine-1(6H)-carboxamide (C-82).

Phosphoryl chloride was added to a tetrahydrofuran solution of (6S,9S)-N-benzyl-6-(4-hydroxybenzyl)-2,9-dimethyl-4,7-dioxo-8-(quinolin-8-ylmethyl)hexahydro-2H-pyrazino[2,1-c][1,2,4]triazine-1(6H)-carboxamide and triethylamine at 0°C, and the mixture was stirred for 4 hours at room temperature. Water was added to the reaction mixture, and the mixture was further stirred overnight. The reaction mixture was acidified with 10% citric acid and extracted with chloroform. The organic phase was dried with magnesium sulfate and filtered. The filtrate was concentrated under reduced pressure to obtain 4-{[(6S,9S)-1-(benzylcarbamoyl)-2,9-dimethyl-4,7-dioxo-8-(quinolin-8-ylmethyl)-octahydro-1H-pyrazino[2,1-c][1,2,4]triazin-6-yl]methyl}phenoxy)phosphoric acid (OK-1).

### (Example 1: Effect of a compound and effect due to combined use with physical therapy)

This Example investigated whether various diseases, disorders, or symptoms associated with sarcopenia can be treated in a mouse model prepared in a Preparation Example by using a compound manufactured in Manufacturing Example 1.

### Decrease in endurance in old mice

In order to confirm that a decrease in muscle strength is observed in mouse models (C57BL/6J) (purchased from Oriental Yeast), the muscle strength (endurance) in 15-week old young mice and 79-week-old old mice was investigated in a treadmill test in accordance with the following (Figure **1A**). Prior to recording the performance, mice were trained for 3 days to acclimate the mice to the equipment. Electric stimulation grid was adjusted to 1 mA, and the gradient was set to 15 degrees. On the first day of training, mice were induced to walk for 10 minutes at a rate of 10 m/minute, allowed to rest for 10 minutes, and then induced to walk for 10 minutes at a rate of 10 m/minute on a treadmill. On day 2 and day 3, the first two steps were the under the same conditions as day 1, and then the mice were induced to start walking at 10 m/minute. The speed was increased by 1 m/minute until reaching the maximum speed of 20 m/minute. On day 4, the maximum exercise endurance was measured. Six mice were placed on a treadmill, which was started at a belt speed of 5 m/minute for 5 minutes to let the mice warm up. The speed was increased 1 m/minute until reaching the maximum speed of 20 m/minute. After making the mice run for 5 minutes, the speed was increased from 20 m/minute to 21 m/minute for 10 minutes. The mice were then forced to run at 22 m/minute until stalling on the electric stimulation grid for 10 seconds. The distance traveled until stalling for 10 seconds was measured.

As a result, the travel distance of mice, i.e., endurance, decreased significantly with age in a treadmill test (Figure **1A**).

### Increase in muscle strength in old mice due to administration of OK-1

OK-1 manufactured in Manufacturing Example 1 was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of OK-1 administration, an increase in muscle strength due to OK-1 administration, which is specific to old mice, was observed (Figure **1A**).

OK-1 was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering OK-1 twice a week at 20 mg/kg/day over 5 weeks (i.e., until reaching 28 week old and 93 week old, respectively). The muscle mass was then measured with the following equipment and conditions through MRI (Figure 1B). MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and the volume of right gastrocnemius muscle/soleus muscle was quantified with Osirix Lite.

It was observed as a result thereof that administration of OK-1 increases muscle mass in old mice, and the effect thereof is maximized, especially when old mice administered with OK-1 were induced to exercise. This result indicates that OK-1 increases muscle strength in old mice. The result also indicates that muscle mass significantly recovers synergistically by combined use of OK-1 administration with exercise. This indicates that OK-1 can be useful as a therapeutic drug for sarcopenia.

### (Example 2: Change in gene expression in muscles by OK-1 administration in mice without exercise)

This Example investigated what gene modulation the compound of the present disclosure is involved in to identify a gene that is useful in the diagnosis or treatment of sarcopenia in mouse models.

### Sarcopenia treatment through OK-1 administration via Hnf4a and Gata4 routes

Gene analysis (RNA-seq) was performed in old mice administered with OK-1 with improved muscle strength and muscle mass. The method and reagent used were the following.

The quality of all reads was checked by using FastQC v0.11.8, and low quality reads were removed using Trimomatic (ver 0.38).
The filtering conditions were the following.

### LEADING: 20 (remove bases with a quality score of less than 20 from the leading reads), TRAILING: 20, SLIDINGWINDOW: 4:15 (window size 4, average quality 15), MINLEN: 36 (remove reads that are less than 3 bases)

Each read obtained after filtering was mapped to reference genome GRCm38 by using STAR (ver 2.7.0.f). Mapping was performed using the default values of STAR. Since paired-end had a length of 150 bp, an index for STAT for reference genome GRCm38 was prepared in alignment with length 150 to perform mapping.

### 1) Index preparation

Run Mode; genomeGenerate
Reference; GRCm38
Annotation file; genecode version M24
sjdbOverhang; 150
alignment was performed using the index prepared above.

In addition, this was performed with default values without options.
(Basic option is the following)
genomeDir; index prepared above

To count the number of genes, featuecount (ver 1.6.4) was used to count the number for each gene name defined by a symbol name, where the number was counted in gene symbol units defined in genecode version M24.

The counted numbers were filtered for genes with a count data of 0 in all samples, subjected to TMM normalization using edgeR (ver 3.22.3), and corrected among samples. Initially, for understanding of the overall picture of the data, primary component analysis and clustering analysis were performed using count data after removal of low expression genes.

### Detection of differentially expressed gene

Differential expression of a pattern designated based on the obtained expression values was compared.

Differentially expressed genes were tested using edgeR. As the definition of differentially expressed gene, uncorrected p. value < 0.05 was used as as a cut-off value, and Fold Change of "pattern not used" and "2 or more or 0.5 or less" were used as the cut-off for calculation.

Gene enrichment analysis was performed on the resulting differentially expressed gene using gprofiler2 (version 0.1.6) .

Expression values after normalization of resulting differentially expressed genes were extracted. Visual heatmap was drawn to study the change. Transcription factor binding motif enrichment analysis was conducted separately for upregulated gene and downregulated gene. This analysis was performed using RcisTarget (ver 1.8.0). RcisTarget performed analysis using a mouse database for 10kbp upstream of TSS stored in RcisTarget. Details of analysis are the following (cited from https://scenic.aertslab.org/tutorials/RcisTarget.html) .

The first step to estimate the over-representation of each motif on the gene-set is to calculate the Area Under the Curve (AUC) for each pair of motif-geneSet. This is calculated based on the recovery curve of the gene-set on the motif ranking (genes ranked decreasingly by the score of motif in its proximity, as provided in the motifRanking database) .

The selection of significant motifs is done based on the Normalized Enrichment Score (NES). The NES is calculated -for each motif- based on the AUC distribution of all the motifs for the gene-set [(x-mean)/sd]. Those motifs that pass the given threshold (3.0 by default) are considered significant. Since RcisTarget searches for enrichment of a motif within a gene list, finding a motif 'enriched' does not imply that all the genes in the gene-list have a high score for the motif. In this way, the third step of the workflow is to identify which genes (of the gene-set) are highly ranked for each of the significant motifs. These genes were identified by a faster implementation based on an approximate distribution using the average at each rank.

The results are shown in Figure 2. 181 genes increased, and 595 decreased significantly by OK-1 administration. A change in a gene group associated with the nerve and intracellular signaling was observed (Figure 2A to C).

While conventional development of a sarcopenia therapeutic drug targeting a TGF-β family known as Myostatin/GDF-8 has been reported, new transcription factors involved in differentiation and cell senescence such as Hnf4a and Gata4, Nrf1, and Hnf1a were observed as upstream factors from upstream transcription factor analysis.

This result suggests the potential for the development of a diagnostic agent and therapeutic drug for sarcopenia through a new target.

Finally, in this aspect, Ppara, Pou2f2, Foxo1, Pparg, Pou2f1, Trp63, Sp1, Tcf3, Zeb1, Srebf1, Sp3, Ebf1, Mycn, and Pbx1 were identified as a gene group associated with OK-1.

This gene group is "genes associated with regulation of muscle without exercise".

### (Example 3: Analysis of gene regulation of muscle through OK-1 administration in mice without exercise)

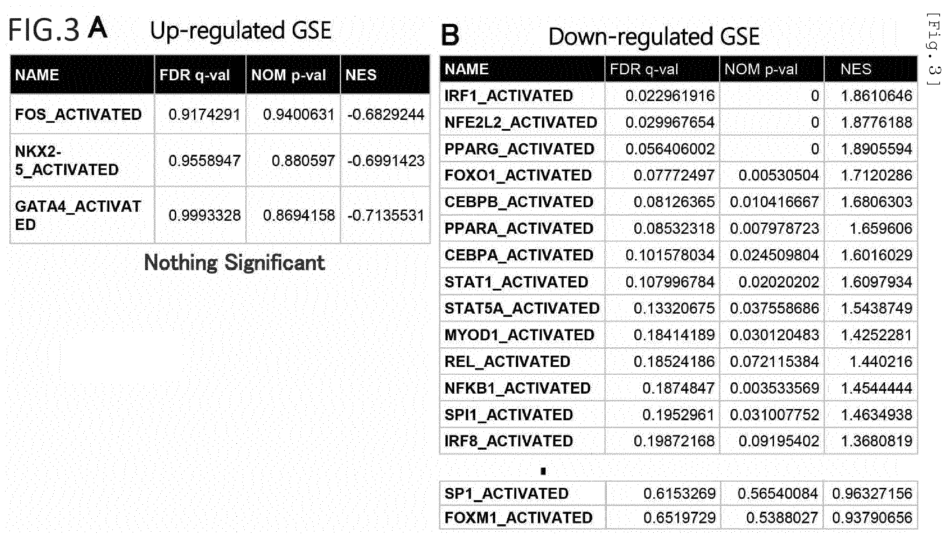

The category of genes with a change in expression identified in Example 2 was analyzed with a software (GSEA), which is different from that in Example 2 (GO) (Figure 3). Analysis with GSEA was performed as follows. GSEA performed categorization by the regulating gene. This is different from GO, which performs categorization by function.

Data was created on the relationship of activattion+unknown and repression+unknown for regulatory genes and transcription factors installed in TRRUST ver2. Gene enrichment analysis was performed using GSEA (https://www.gsea-msigdb.org/gsea/index.jsp) based on respective gene sets. local gsea-3.0 that starts up within java was used for GSEA.

Finally, in this aspect, IRF1, NFE2L2, PPARG, FOXO1, CEBPB, PPARA, CEBPA, STAT1, STAT5A, MYOD1, REL, NFKB1, SPI1, IRF8, SP1, and FOXM1 were identified as a gene group associated with OK-1.

This gene group is "involved in increase in muscle".

### (Example 4: Differentially expressed gene of muscle through OK-1 administration in mice without exercise)

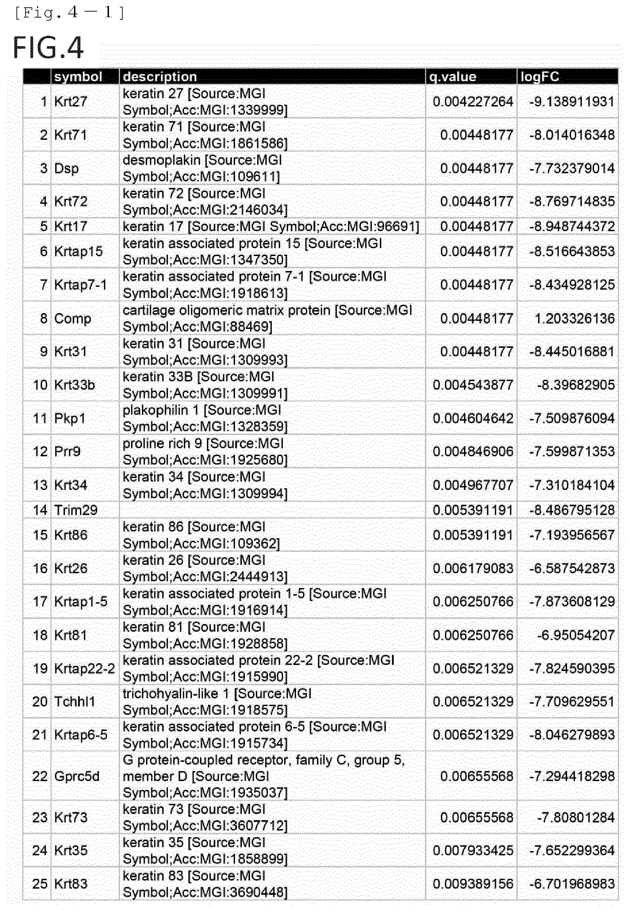

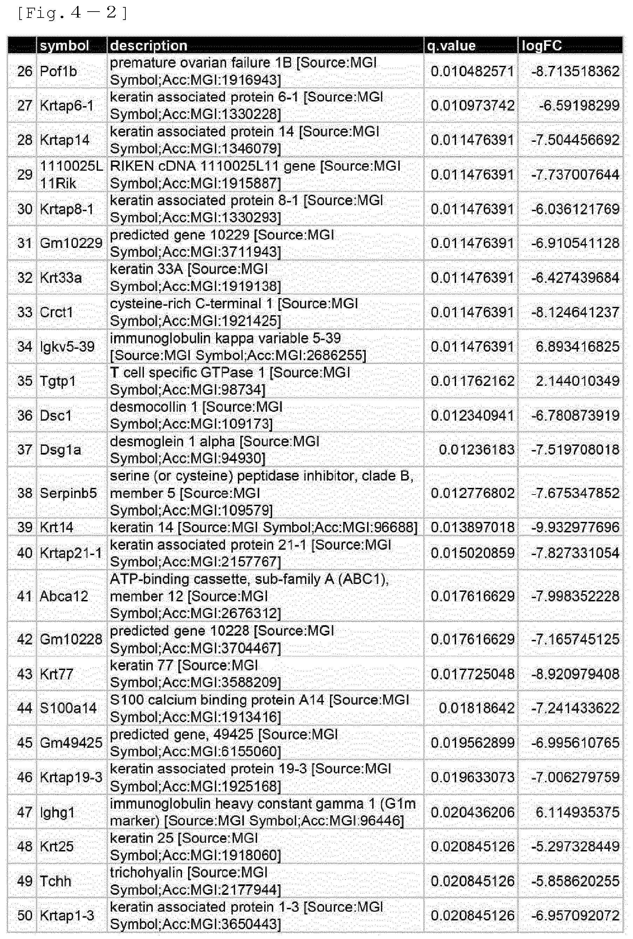

Top 50 differentially expressed genes are shown. Many differential expressions in genes associated with keratin, etc. are observed. This suggests the potential for application to connective tissue such as bones and skin (Figure **4**)**.**

Finally, in this aspect, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, and Krtap1-3 were identified as a gene group associated with OK-1.

This gene group is "differentially expressed genes of muscle".

### (Example 5: Differential expression of gene in muscle through OK-1 administration in mice with exercise)

This Example investigated what gene modulation the compound of the present disclosure is involved in to identify a gene that is useful in the diagnosis or treatment of sarcopenia in mouse models.

Gene analysis (RNA-seq) in OK-1 administered mice (C57BL/6J) with exercise was performed by the same method as Example 2.

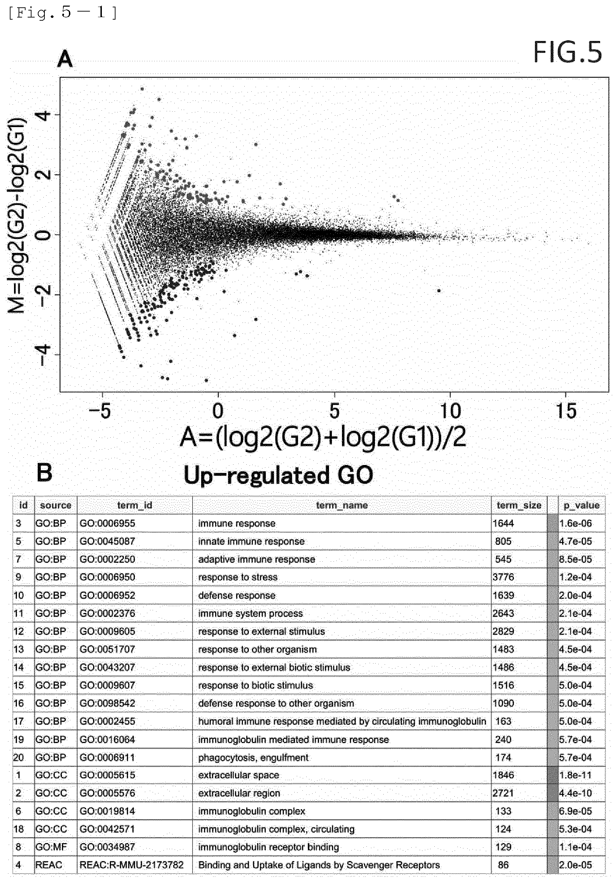

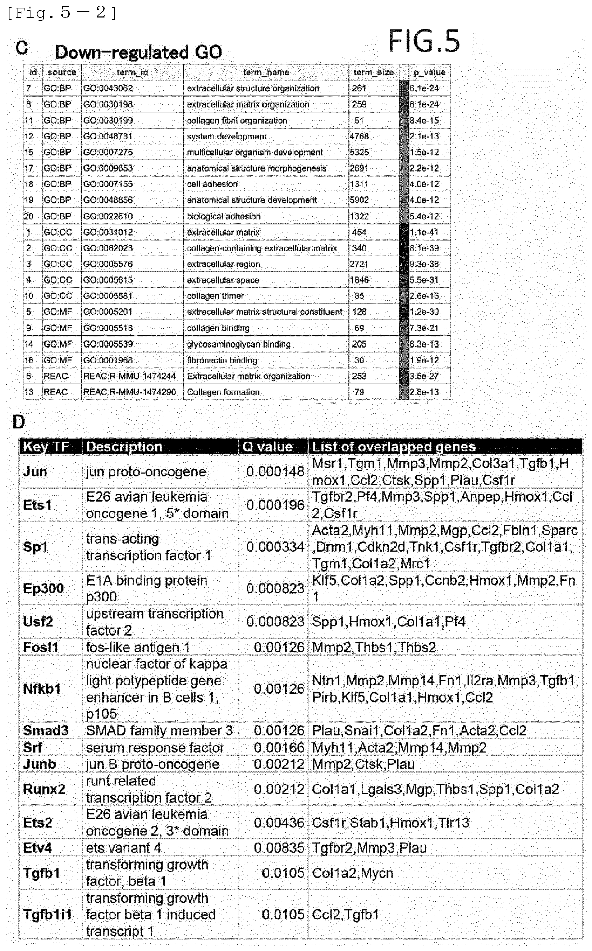

The results thereof are shown in Figure 5. 145 genes increased and 631 genes decreased significantly by OK-1 administration (Figure **5A).** Figures **5B** to **D** show categorization of biological functions (GO) in which these genes are involved (Figures **5B****,** **C),** and ranking of upstream transcription factors of changed genes (Figure **5D**)**.**

Finally, in this aspect, Jun, Ets1, Sp1, Ep300, Usf2, Fosl1, Nfkb1, Smad3, Srf, Junb, Runx2, Ets2, Etv4, Tgfb1, and Tgfb1i1 were identified as a gene group associated with OK-1.

This gene group is "genes associated with muscle control with exercise/genes useful in the diagnosis or treatment of sarcopenia".

### (Example 6: Analysis of regulation of gene of muscle through OK-1 administration in mice with exercise)

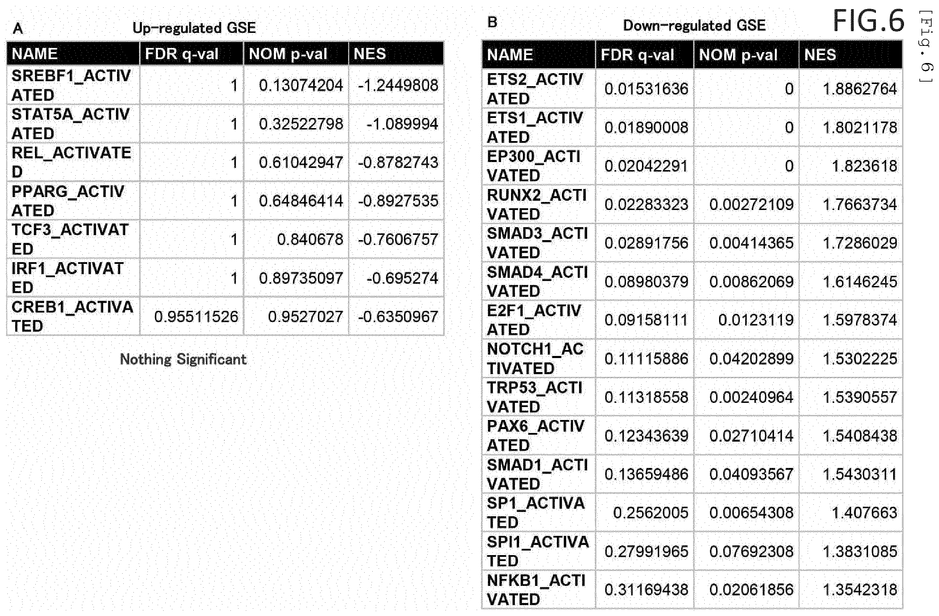

The category of genes with differential expression identified in Example 5 was analyzed by the same method (GSEA) as Example 3 (Figure **6**)**.**

Finally, in this aspect, ETS2, ETS1, EP300, RUNX2, SMAD3, SMAD4, E2F1, NOTCH1, TRP53, PAX6, SMAD1, SP1, SPI1, and NFKB1 were identified as a gene group associated with OK-1 (same as above).

This gene group is "genes regulating muscle genes with exercise".

### (Example 7: Differentially expressed gene of muscle through OK-1 administration in mice with exercise)

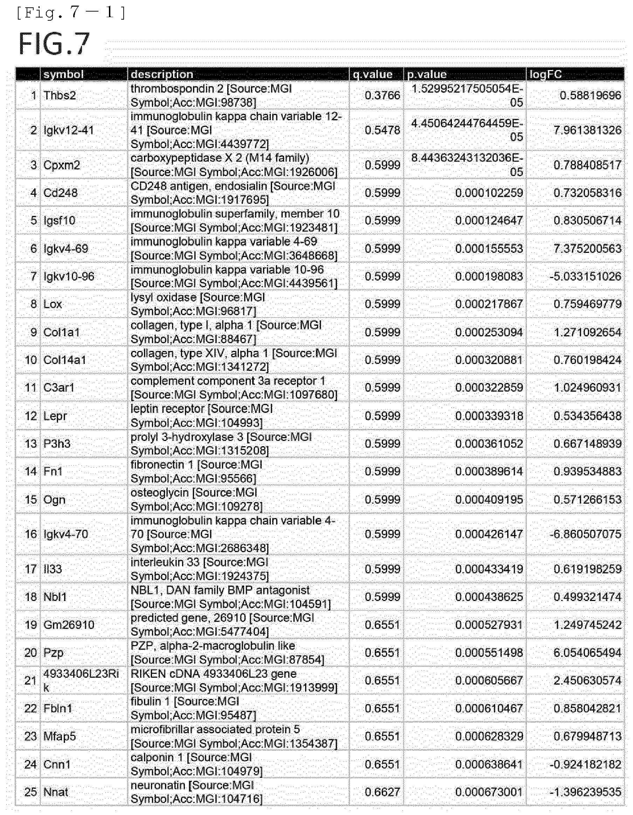

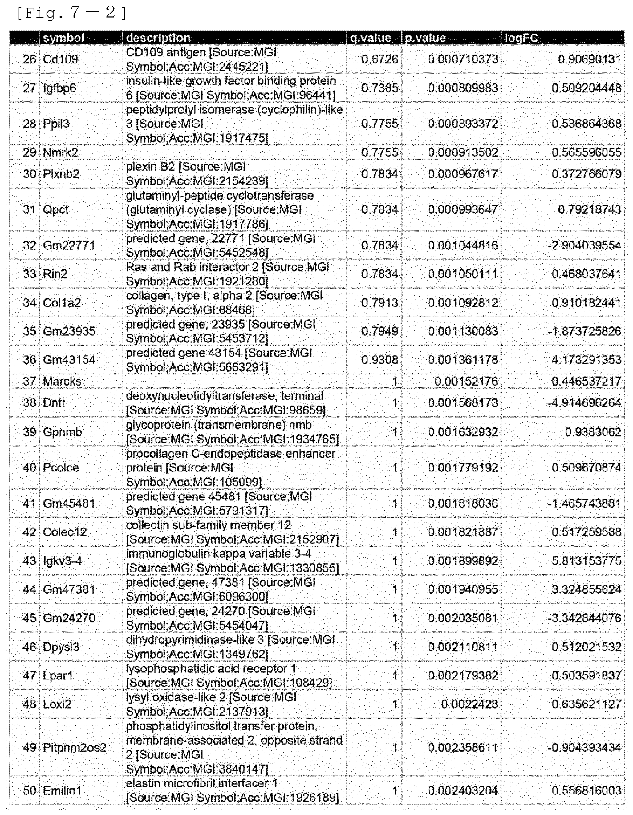

Top 50 differentially expressed genes are shown. Many differential expressions in genes associated with keratin, etc. were observed. This suggests the potential for application to connective tissue such as bones and skin (Figure 7).

Finally, in this aspect, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, 1133, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, and Emilin1 were identified as a gene group associated with OK-1.

This gene group is "genes regulating gene of muscle/connective tissue with exercise".

### (Example 8: Disease and differentially expressed gene of muscle through OK-1 administration in mice with/without exercise)

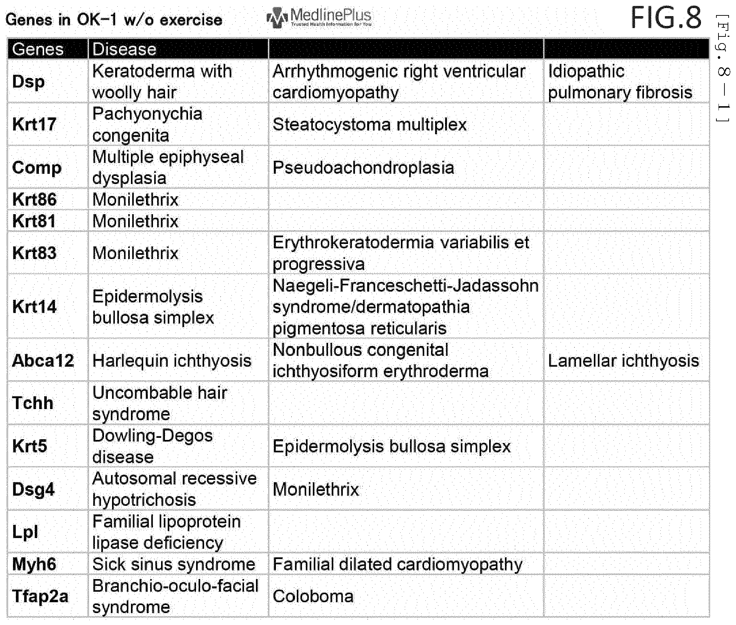

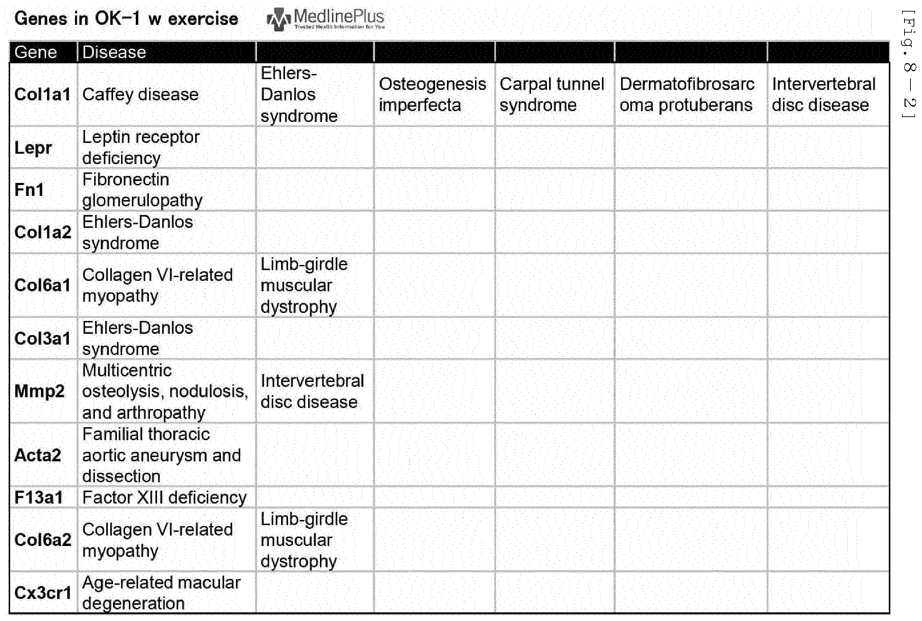

Diseases associated with top 100 genes observed to have differential expression in mice with/without exercise were found by collation with NIH MedlinePlus (Figure **8**).

Finally, in this aspect, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1 were identified as a gene group associated with OK-1.

This gene group is "genes with change in muscle control with/without exercise".

### (Example 9)

The same experiment as Example 1 was conducted with compounds of compound groups [2] to [9] of the present disclosure. An increase in muscle was observed specifically when these compounds were administered to old mice.

### (Example 9-1)

The effect from combined use of a compound of formula (I) in compound group [1] of the present disclosure 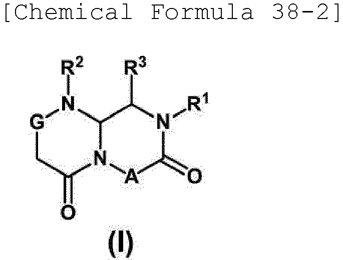 (wherein A, G, R¹, R², and R³ are defined the same as above) with physical therapy was investigated by conducting the same experiment as Example 1.

### Increase in muscle in old mice due to administration of a compound of formula (I)

A compound of formula (I) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (I), an increase in muscle strength due to administration of a compound of formula (I) was observed specifically in old mice.

A compound of formula (I) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (I) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (I) increases muscle mass in old mice, and the effect thereof is maximized, especially when old mice administered with a compound of formula (I) were induced to exercise. This result indicates that a compound of formula (I) increases muscle strength in old mice. The result also indicates that muscle mass significantly recovers synergistically by combined use of administration of a compound of formula (I) with exercise. This indicates that a compound of formula (I) can be useful as a therapeutic drug for sarcopenia.

### (Example 9-2)

The effect from combined use of a compound of formula (II) in compound group [2] of the present disclosure 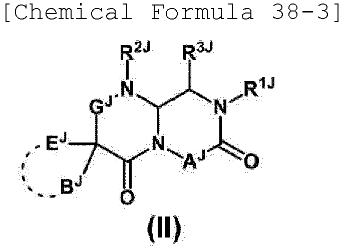 (wherein A^{J}, B^{J}, E^{J}, G^{J}, R^{1J}, R^{2J}, and R^{3J} are defined the same as above) with physical therapy was investigated by conducting the same experiment as Example 1.

### Increase in muscle strength in old mice due to administration of a compound of formula (II)

A compound of formula (II) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (II), an increase in muscle strength due to administration of a compound of formula (II) was observed specifically in old mice.

A compound of formula (II) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (II) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (II) increases muscle mass in old mice, and the effect thereof is maximized, especially when old mice administered with a compound of formula (II) were induced to exercise. This result indicates that a compound of formula (II) increases muscle strength in old mice. The result also indicates that muscle mass significantly recovers synergistically by combined use of administration of a compound of formula (II) with exercise. This indicates that a compound of formula (II) can be useful as a therapeutic drug for sarcopenia.

### (Example 9-3)

The effect from combined use of a compound of formula (III) in compound group [3] of the present disclosure 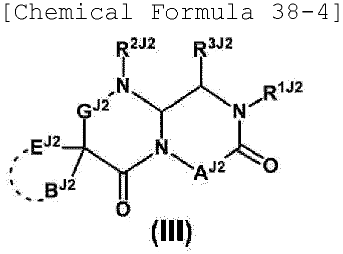 (wherein A^{J2}, B^{J2}, E^{J2}, G^{J2}, R^{1J2}, R^{2J2}, and R^{3J2} are defined the same as above) with physical therapy was investigated by conducting the same experiment as Example 1.

### Increase in muscle strength in old mice due to administration of a compound of formula (III)

A compound of formula (III) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (III), an increase in muscle strength due to administration of a compound of formula (III) was observed specifically in old mice.

A compound of formula (III) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (III) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (III) increases muscle mass in old mice, and the effect thereof is maximized, especially when old mice administered with a compound of formula (III) were induced to exercise. This result indicates that a compound of formula (III) increases muscle strength in old mice. The result also indicates that muscle mass significantly recovers synergistically by combined use of administration of a compound of formula (III) with exercise. This indicates that a compound of formula (III) can be useful as a therapeutic drug for sarcopenia.

### (Example 9-4)

The effect from combined use of a compound of formula (IV) in compound group [4] of the present disclosure 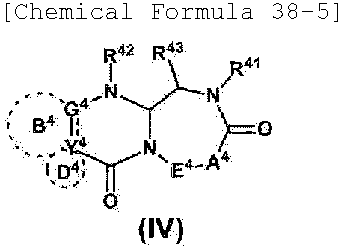 (wherein 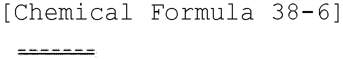 is a single bond or a double bond, and
A⁴, B⁴, D⁴, E⁴, G⁴, Y⁴, R⁴¹, R⁴², and R⁴³ are defined the same as above) with physical therapy was investigated by conducting the same experiment as Example 1.

### Increase in muscle strength in old mice due to administration of a compound of formula (IV)

A compound of formula (IV) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (IV), an increase in muscle strength due to administration of a compound of formula (IV) was observed specifically in old mice.

A compound of formula (IV) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (IV) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (IV) increases muscle mass in old mice, and the effect thereof is maximized, especially when old mice administered with a compound of formula (IV) were induced to exercise. This result indicates that a compound of formula (IV) increases muscle strength in old mice. The result also indicates that muscle mass significantly recovers synergistically by combined use of administration of a compound of formula (IV) with exercise. This indicates that a compound of formula (IV) can be useful as a therapeutic drug for sarcopenia.

### (Example 9-5)

The effect from combined use of a compound of formula (5) in compound group [V] of the present disclosure 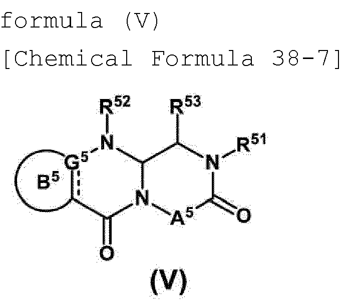 (wherein 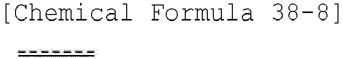 is a single bond or a double bond, and
A⁵, B⁵, G⁵, R⁵¹, R⁵², and R⁵³ are defined the same as above) with physical therapy is investigated by conducting the same experiment as Example 1.

### Increase in muscle strength in old mice due to administration of a compound of formula (V)

A compound of formula (V) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (V), an increase in muscle strength due to administration of a compound of formula (V) was observed specifically in old mice.

A compound of formula (V) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (V) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (V) increases muscle mass in old mice, and the effect thereof is maximized, especially when old mice administered with a compound of formula (V) were induced to exercise. This result indicates that a compound of formula (V) increases muscle strength in old mice. The result also indicates that muscle mass significantly recovers synergistically by combined use of administration of a compound of formula (V) with exercise. This indicates that a compound of formula (V) can be useful as a therapeutic drug for sarcopenia.

### (Example 9-6)

The effect from combined use of a compound of formula (VI) in compound group [6] of the present disclosure 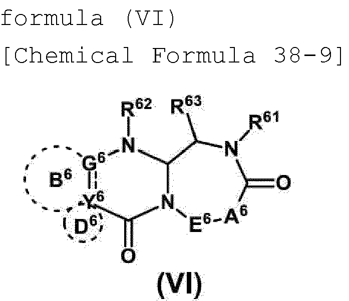 (wherein 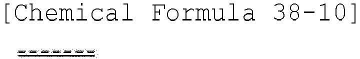 is a single bond or a double bond, and
A⁶, B⁶, D⁶, E⁶, G⁶, Y⁶, R⁶¹, R⁶², and R⁶³ are defined the same as above) with physical therapy is investigated by conducting the same experiment as Example 1.

### Increase in muscle strength in old mice due to administration of a compound of formula (VI)

A compound of formula (VI) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (VI), an increase in muscle strength due to administration of a compound of formula (VI) was observed specifically in old mice.

A compound of formula (VI) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (VI) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (VI) increases muscle mass in old mice, and the effect thereof is maximized, especially when old mice administered with a compound of formula (VI) were induced to exercise. This result indicates that a compound of formula (VI) increases muscle strength in old mice. The result also indicates that muscle mass significantly recovers synergistically by combined use of administration of a compound of formula (VI) with exercise. This indicates that a compound of formula (VI) can be useful as a therapeutic drug for sarcopenia.

### (Example 9-7)

The effect from combined use of a compound of formula (VII) in compound group [7] of the present disclosure 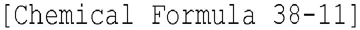 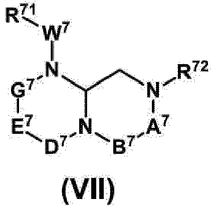 (wherein A⁷, B⁷, D⁷, E⁷, G⁷, W⁷, R⁷¹, and R⁷² are defined the same as above) with physical therapy is investigated by conducting the same experiment as Example 1.

### Increase in muscle strength in old mice due to administration of a compound of formula (VII)

A compound of formula (VII) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (VII), an increase in muscle strength due to administration of a compound of formula (VII) was observed specifically in old mice.

A compound of formula (VII) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (VII) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (VII) increases muscle mass in old mice, and the effect thereof is maximized, especially when old mice administered with a compound of formula (VII) were induced to exercise. This result indicates that a compound of formula (VII) increases muscle strength in old mice. The result also indicates that muscle mass significantly recovers synergistically by combined use of administration of a compound of formula (VII) with exercise. This indicates that a compound of formula (VII) can be useful as a therapeutic drug for sarcopenia.

### (Example 9-8)

The effect from combined use of compound 8 in compound group [8] of the present disclosure 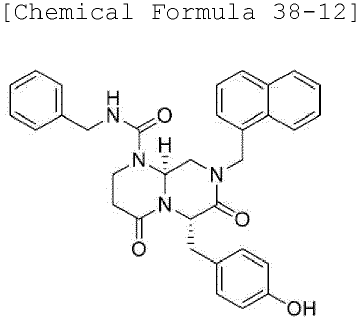 with physical therapy is investigated by conducting the same experiment as Example 1.

### Increase in muscle strength in old mice due to administration of compound 8

Compound 8 was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of compound 8, an increase in muscle strength due to administration of compound 8 was observed specifically in old mice.

Compound 8 was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering compound 8 twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of compound 8 increases muscle mass in old mice, and the effect thereof is maximized, especially when old mice administered with compound 8 were induced to exercise. This result indicates that compound 8 increases muscle strength in old mice. The result also indicates that muscle mass significantly recovers synergistically by combined use of administration of compound 8 with exercise. This indicates that compound 8 can be useful as a therapeutic drug for sarcopenia.

### (Example 9-9)

The effect from combined use of a compound of formula (IX) in compound group [9] of the present disclosure 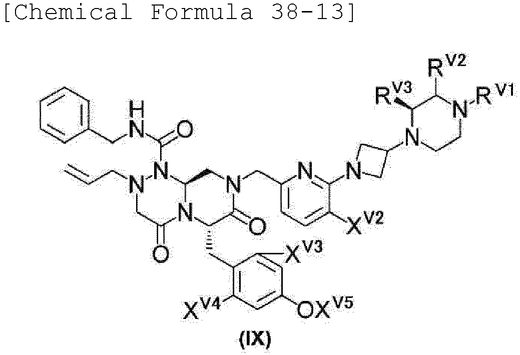 (wherein X^{V2}, X^{V3}, X^{V4}, X^{V5}, R^{V1}, R^{v2} and R^{v3} are defined the same as above) with physical therapy is investigated by conducting the same experiment as Example 1.

### Increase in muscle strength in old mice due to administration of a compound of formula (IX)

A compound of formula (IX) was intraperitoneally administered to young and old mice twice a week over three months. The dosage was 20 mg/kg/day.

During the dosing period, a significant change in body weight and behavioral abnormality were not observed. As a result of measuring the muscle strength (endurance) through a treadmill test after three months of administration of a compound of formula (IX), an increase in muscle strength due to administration of a compound of formula (IX) was observed specifically in old mice.

A compound of formula (IX) was then intraperitoneally administered to each mouse twice a week at 20 mg/kg/day over 8 weeks (i.e., until reaching 23 week old and 88 week old, respectively). Treadmill exercise was conducted three times a week at 8 m/minute for 5 minutes → 15 m/minute for 35 minutes while administering a compound of formula (IX) twice a week at 20 mg/kg/day over 5 weeks (i.e., until reaching 28 week old and 93 week old, respectively). The muscle mass was then measured by MRI with the following equipment and conditions. MRI (MRminiSA1545, Japan REDOX) (magnetic flux density: 1.5 tesla (permanent magnet) (temporal resolution: 100 ns) was used to capture images of the lower limbs of the mice. The recovered data was converted into dicom data, and then the volume of right gastrocnemius muscle/soleus muscle was quantified with Osirix Lite.

It was observed as a result thereof that administration of a compound of formula (IX) increases muscle mass in old mice, and the effect thereof is maximized, especially when old mice administered with a compound of formula (IX) were induced to exercise. This result indicates that a compound of formula (IX) increases muscle strength in old mice. The result also indicates that muscle mass significantly recovers synergistically by combined use of administration of a compound of formula (IX) with exercise. This indicates that a compound of formula (IX) can be useful as a therapeutic drug for sarcopenia.

### (Example 10: Example proving association with organ: 1 Test on functional aspect)

This Example investigated the effect of the compound of the present disclosure on an organ of a sarcopenia subject with regard to the functional aspect.

This Example used mouse models prepared in the Preparation Example.

Measurement can be taken using Kumamoto Mouse Clinic (KMC) of Kumamoto University.

Mouse models were divided into two groups. Cardiac function was tested for sarcopenia mouse models administered with OK-1 and sarcopenia mouse models without administration of OK-1. Cardiac function was tested and analyzed using an echocardiogram and treadmill. It can be understood from the results that cardiac function is improved in mice administered with OK-1 compared to mice without administration of OK-1.

### (Example 11: Example proving association with organ: 2 Test on biochemical aspect)

This Example investigated the effect of the compound of the present disclosure on an organ of a sarcopenia subject with regard to the biochemical aspect.

This Example used mouse models prepared in the Preparation Example.

Mouse models were divided into two groups. The creatine concentration, IL-6 concentration, and cholesterol concentration were measured for sarcopenia mouse models administered with OK-1 and sarcopenia mouse models without administration of OK-1. It can be understood from the results thereof that muscle improves in mice administered with OK-1 compared to mice without administration of OK-1.

### (Example 12: Example proving association with bone: 1 Test on functional aspect)

This Example investigated the effect of the compound of the present disclosure on a bone of a subject with regard to the functional aspect.

This Example tested bone density for mice administered with OK-1 and mice without administration of OK-1 in order to investigate the effect of OK-1 on mouse bones. Bone density was measured using DEXA or ultrasound. It can be understood from the results thereof that bone density is improved in mice administered with OK-1 compared to mice without administration of OK-1.

### (Example 13: Example proving association with bone: 2 Test on biochemical aspect)

This Example investigated the effect of the compound of the present disclosure on a bone of a sarcopenia subject with regard to the biochemical aspect.

This Example used mouse models prepared in the Preparation Example.

Mouse models were divided into two groups. The NTX (cross linked N-telopeptide of type I collagen) concentration was measured for sarcopenia mouse models administered with OK-1 and sarcopenia mouse models without administration of OK-1. It can be understood from the results thereof that osteoporosis is improved in mice administered with OK-1 compared to mice without administration of OK-1.

### (Example 14: Example proving associated with an organ with a metabolic disease)

This Example investigated the effect of the compound of the present disclosure on an organ with a metabolic disease of a sarcopenia subject.

This Example used mouse models prepared in the Preparation Example.

Mouse models were divided into two groups. LDH concentration, ALT concentration, TG concentration, HDL-C concentration, insulin concentration, glucose concentration, amylase concentration, AST concentration, and ALT concentration were measured for sarcopenia mouse models administered with OK-1 and sarcopenia mouse models without administration of OK-1. It can be understood from the results thereof that pancreatic function, liver function, and salivary gland function are improved in mice administered with OK-1 compared to mice without administration of OK-1.

In addition thereto, the following are investigated as blood biomarkers and cytokines anticipated to have a correlation with a related disease: insulin or glucose (type II diabetes), BAP (bone alkaline phosphatase), NTX (cross linked N-telopeptide of type I collagen) (osteoporosis), creatine (kidney disease, muscle, or cerebral disorder), cytokine such as IL-6 and cholesterol (cancer and cardiovascular disease), AST and ALT (liver function), and amylase (pancreas or salivary gland).

In view of this, various diseases described within the parentheses can be diagnosed or treated, or a therapeutic effect can be assessed based on increase/decrease in each of the cytokines and biomarkers.

In addition thereof, the following can be measured as a biochemical test: TP (i.e., total protein), Alb (i.e., albumin), AST (GOT), ALP (i.e., alkaline phosphatase), LDH (i.e., lactate dehydrogenase), ALT (GPT), γ-GTP (i.e., glutamyl transpeptidase), CHE (i.e., cholinesterase), CPK (CK): creatine kinase), CK-MB, AMY (i.e., amylase), T-Bil (i.e., total bilirubin), T-cho (i.e., total cholesterol), TG (i.e., neutral fat or triglyceride), LDL-C (i.e., LDL cholesterol), HDL-C (i.e., HDL cholesterol), BUN (i.e., urea nitrogen), UA (i.e., uric acid), Cre (i.e., creatinine), Na (i.e., sodium), K (i.e., potassium), Cl (i.e., chlorine), Ca (i.e., calcium), GLU (i.e., blood sugar or glucose), HbA1c (i.e., glycohemoglobin), procalcitonin (i.e., PCT), cardiac muscle troponin T (i.e., TnT), CRP (i.e., C reactive protein), and BNP (i.e., brain natriuretic polypeptide).

The following can be determined from the measurement thereof.
*TP (i.e., total protein)
High value
   polyclonal: autoimmune disease, chronic inflammatory disease, cirrhosis, malignant tumor, infection, etc.
   monoclonal: multiple myeloma, macroglobulinemia, etc. Low value
   insufficient nutrition: insufficient nutrition intake, intestinal malabsorption syndrome, etc.
   liver disorder: acute hepatitis, cirrhosis, etc.
   protein losing: nephrotic syndrome, protein losing enteropathy, etc.
   hypermetabolism: acute infection, chronic wasting disease, etc.
   abnormal biodistribution: generalized edema, sunburn, etc.
*Alb (i.e., albumin)
High value
   hemoconcentration (dehydration)
Low value
   insufficient nutrition: insufficient nutrition intake, intestinal malabsorption syndrome, etc.
   liver disorder: acute hepatitis, cirrhosis, etc.
   protein losing: nephrotic syndrome, protein losing enteropathy, etc.
   hypermetabolism: acute infection, chronic wasting disease, etc.
   abnormal biodistribution: generalized edema, sunburn, etc.
*AST (GOT)
High value
   liver disease: acute hepatitis, chronic hepatitis, alcoholic hepatitis, cirrhosis, liver cancer, fatty liver, etc.
   heart disease: acute myocardial infarction, etc.
   muscular disease: progressive muscular dystrophy, polymyositis, myasthenia, etc.
   others: strenuous exercise, hemolysis, etc.
*ALP (i.e., alkaline phosphatase)
High value
   liver disease: acute hepatitis, chronic hepatitis, alcoholic hepatitis, cirrhosis, liver cancer, primary biliary cirrhosis, etc.
   biliary tract disease: bile duct cancer, choledocholithiasis, sclerosing cholangitis, etc.
   bone disease: osteomalacia, osteosarcoma, metastatic bone tumor, etc.
   others: childhood, third trimester, hyperthyroidism, uremia, etc.
*LDH (i.e., lactate dehydrogenase)
High value
   liver disease: acute hepatitis, chronic hepatitis, cirrhosis, liver cancer, etc.
   heart disease: acute myocardial infarction, congested heart failure, etc.
   muscular disease: polymyositis, muscular dystrophy, etc.
   others: pernicious anemia, leukemia, hemolytic anemia, etc.
*ALT (GPT)
High value
   liver disease: acute hepatitis, chronic hepatitis, alcoholic hepatitis, cirrhosis, liver cancer, fatty liver, etc.
   heart disease: acute myocardial infarction, etc.
   muscular disease: progressive muscular dystrophy, etc.
   others: cholestasis, infectious mononucleosis, etc.
*γ-GTP (i.e., glutamyl transpeptidase)
High value
   liver disease: alcoholic liver disease, alcoholic fatty liver, habitual drinking, chronic hepatitis, cirrhosis, liver cancer, etc.
   biliary tract disease: cholangitis, biliary atresia, biliary tract cancer, etc.
   others: gastric cancer, lung cancer, etc.
*CHE (i.e., cholinesterase)
High value
   nephrotic syndrome, fatty liver, diabetes, hyperthyroidism, etc.
Low value
   liver disease: acute hepatitis, chronic hepatitis, cirrhosis, liver cancer, etc.
   others: biliary obstruction, pancreatitis, drug addiction, etc.
*CPK (CK) (i.e., creatine kinase)
High value
   liver disease: progressive muscular dystrophy, polymyositis, dermatomyositis, etc.
   heart disease: acute myocardial infarction, myocarditis, etc.
   brain disease: cerebral thrombosis, cerebral infarction, brain damage, etc.
   others: hypothyroidism, malignant tumor, drug addiction, etc.
*CK-MB
High value
   acute myocardial infarction, rhabdomyolysis, polymyositis, muscular dystrophy, other skeletal muscle diseases, hypothyroidism, epilepsy, Reye's syndrome, and other convulsive disorders
*AMY (i.e., amylase)
High value
   pancreatic disease: acute pancreatitis, chronic pancreatitis, pancreatic cyst, etc.
   others: mumps, duodenal ulcer, ileus, peritonitis, ovarian cancer, renal failure, etc.
*T-Bil (i.e., total bilirubin)
High value
   hemolytic jaundice, neonatal jaundice, acute hepatitis, cirrhosis, primary biliary cirrhosis, intrahepatic biliary obstruction, choledocholithiasis, etc.
*T-cho (i.e., total cholesterol)
High value
   primary: familial hypercholesterolemia, etc.
   secondary: diabetes, nephrotic syndrome, arteriosclerosis, multiple myeloma, obstructive jaundice, hypothyroidism, pregnancy, etc.
Low value
   primary: abetalipoproteinemia, hypobetalipoproteinemia, LCAT deficiency, etc.
   secondary: hyperthyroidism, severe liver disorder, hypopituitarism, etc.
*TG (i.e., neutral fat and triglyceride)
High value
   primary: hyperchylomicronemia, LPL deficiency, etc.
   secondary: diabetes, obesity, arteriosclerosis, gout, hypothyroidism, Cushing's syndrome, nephrotic syndrome, obstructive jaundice, acute/chronic pancreatitis, etc.
Low value
   primary: abetalipoproteinemia, hypobetalipoproteinemia, etc.
   secondary: hyperthyroidism, hypopituitarism, cirrhosis, Addison's disease, etc.
*LDL-C (i.e., LDL cholesterol)
High value
   arteriosclerosis, nephrotic syndrome, diabetes, obesity, familial hypercholesterolemia, obstructive jaundice, etc. Low value
   cirrhosis, chronic hepatitis, hyperthyroidism, familial hypocholesterolemia, etc.
*HDL-C (i.e., HDL cholesterol)
High value
   primary: familial hyperalphalipoproteinemia, etc.
   secondary: obstructive pulmonary disease, primary biliary cirrhosis, alcohol consumption, exercise, etc.
Low value
   primary: LCAT deficiency, LPL deficiency, etc.
   secondary: diabetes, obesity, cerebral infarction, coronary arteriosclerosis, chronic renal failure, cirrhosis, thyroid dysfunction, smoking, etc.
*BUN (i.e., urea nitrogen)
High value
   prerenal: dehydration, severe heart failure, gastrointestinal bleeding, etc.
   renal: nephritis, uremia, nephrotic syndrome, kidney stone, etc.
   postrenal: ureteral obstruction, bladder tumor, etc. Low value
   toxic hepatitis, fulminant hepatitis, end-stage of cirrhosis, diabetes insipidus, acromegaly, etc.
*UA (i.e., uric acid)
High value
   primary gout: juvenile gout, etc.
   secondary gout: leukemia, hyperlipidemia, renal failure, etc.
Low value
   reduced biosynthesis: cirrhosis, xanthinuria, etc.
   secondary reduction: Fanconi syndrome, Wilson's syndrome, glomerulonephritis, etc.
*Cre (i.e., creatinine)
High value
   reduced GFR (glomerular filtration rate): glomerulonephritis, renal failure, etc.
   hemoconcentration: dehydration, burn, etc.
Low value
   Increased amount of urinary extraction: diabetes insipidus, pregnancy, etc.
   muscle atrophy: muscular dystrophy, etc.
*Na (i.e., sodium)
High value
   vomiting, diarrhea, hyperhidrosis (heat stroke), diabetes insipidus, hypercalcemia, Cushing's syndrome, dry mouth sensation disorder, etc.
Low value
   renal failure, nephrotic syndrome, heart failure, cirrhosis, Addison's disease, pregnancy-induced hypertension, diuretic/antibiotic administration, etc.
*K (i.e., potassium)
High value
   Migration from within a cell: metabolic acidosis, insulin deficiency, effect of drug, etc.
   reduced excretion in kidney: renal failure, Addison's disease, hypoaldosteronism, etc.
   others: hemolysis, leukocytosis, thrombocytosis, etc.
Low value
   Migration into a cell: metabolic alkalosis, insulin administration, high concentration transfusion, etc.
   Loss from digestive tract: vomiting, diarrhea, malabsorption syndrome, etc.
   Loss from kidney: primary aldosteronism, Cushing's syndrome, effect of diuretic, etc.
*Cl (i.e., chlorine)
High value
   nephrotic syndrome, renal failure, Cushing's syndrome, dehydration, excessive salt intake, etc.
Low value
   intense vomiting, Addison's disease, diabetes insipidus, insufficient salt intake, etc.
*Ca (i.e., calcium)
High value
   milk-alkali syndrome, multiple myeloma, hyperthyroidism, primary hyperparathyroidism, acute renal failure, pheochromocytoma, familial hypocalciuric hypercalcemia, malignant tumor bone metastasis, Addison's disease
Low value
   vitamin D deficiency, malabsorption syndrome, acute pancreatitis, hypomagnesemia, uremia, hypoparathyroidism, chronic renal failure, nephrotic syndrome
*GLU (i.e., blood sugar, glucose)
High value
   diabetes (type I, type II, etc.)
   pancreatic disease: pancreatic cancer, pancreatitis, etc.
   endocrine disease: acromegaly, hyperthyroidism, Cushing's syndrome, etc.
   others: post-gastrectomy, stress, obesity, etc.
Low value
   pancreatic disease: insulinoma (excess insulin secretion)
   liver disease: cirrhosis, liver cancer, etc.
   endocrine disease: hypothyroidism, hypopituitarism
   others: excessive insulin injection, use of oral hypoglycemic agent, strenuous exercise, fasting, etc.
*HbA1c (i.e., glycohemoglobin)
High value
   diabetes, other diseases exhibiting hyperglycemia, etc. Low value
   decrease in erythrocyte lifespan due to hemolytic anemia, hemorrhage, etc., resulting in increased reticulocyte, etc.
*procalcitonin (i.e., PCT)
High value
   Bacterial infection exhibiting systemic inflammatory response, sepsis, systemic fungal infection, pancreatitis, etc.
Low value
   Viral infection, chronic systemic inflammation (rheumatoid arthritis, vasculitis, etc.), autoimmune disease, tumor, locally confirmed bacterial infection, etc.
*Cardiac muscle troponin T (i.e., TnT)
High value
   acute myocardial infarction, angina pectoris, myocarditis, etc.
*CRP (i.e., C reactive protein)
High value
   infection, malignant tumor, autoimmune disease, necrosis of tissue, inflammatory disease, etc.
*BNP (i.e., brain natriuretic polypeptide)
High value
   heart failure, myocardial infarction, hypertension, chronic renal failure, nephrotic syndrome, Cushing's syndrome, hyperthyroidism, etc.
Low value
   dehydrated state, effect of diuretic, etc.

The various cytokines, etc., described above can also be used to prepare genetically modified mice (Trans Genic, Kumamoto, Japan).

Cytokine, etc., can be tested by using a commercially available kit (e.g., Bio-Plex Pro Human Cytokine Assay Panel (Bio-Rad)).

### (Example 15: Example proving association with brain function: 1 Functional aspect)

This Example investigated the effect of the compound of the present disclosure on the brain of a sarcopenia subject with regard to the functional aspect.

This Example used mouse models prepared in the Preparation Example.

Mouse models were divided into two groups. Short term memory, long term memory, depression, activity level, social interaction, cognitive ability, etc., were tested using a Barnes maze, fear conditioning test, object recognition test, open field test, social behavior test (Crawley version), etc. on sarcopenia mouse models administered with OK-1 and sarcopenia mouse models without administration of OK-1.

These tests are described in http://www.fujita-hu.ac.jp/ to smedsci/behaviorcore/equipment/index.html, Barnes maze, a useful task to assess spatial reference memory in the mice (doi: 10.1038/nprot.2007.390), etc., which can be suitably referenced and practiced.

It can be understood that short term memory, long term memory, depression, activity level, social interaction, and cognitive ability are improved in mice administered with OK-1 compared to mice without administration of OK-1.

### (Example 16: Example proving association with brain function: 2 Biochemical aspect)

This Example investigated the effect of the compound of the present disclosure on the brain of a subject with regard to the biochemical aspect.

This Example investigated whether suppression in phosphorylation of Tau and improvement in memory or brain atrophy were observed in mice administered with OK-1 and mice without administration of OK-1 by using P301S mouse models with advanced Tau phosphorylation and brain atrophy, which are pathological conditions of Alzheimer's (FTDP-17 mouse model; Yoshiyama, Clinical Neurology, 48: 910-912, 2008). It can be understood from the results thereof that suppression in phosphorylation of Tau and improvement in memory or brain atrophy are observed in mice administered with OK-1 and mice without administration of OK-1.

This Example demonstrated whether the compound is effective on tauopathy.

It can be demonstrated whether the compound can be applied to various neurodegenerative diseases known as tauopathy through treatment of tau phosphorylation. Tauopathy refers to a condition wherein a tau protein, which is a type of microtubule-associated protein, is accumulated within a cell. Tauopathy includes Alzheimer's disease, as well as progressive supranuclear palsy, corticobasal degeneration, Pick's disease, etc.

### (Example 17: Diagnosis/treatment through gene regulation)

It is demonstrated whether this can be applied to diagnosis, treatment, or companion therapy on sarcopenia subjects in light of the results of Examples 2 to 8.

Whether a subject has sarcopenia can be diagnosed by measuring the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1, and treatment can be administered based on the diagnosis.

### (Example 18: Modulation of gene expression in cultured cell)

Mouse striated muscle derived cells, i.e., C2C12 cells, were cultured and treated with OK-1, ICG-001, or C-82. Gene expression levels of cells treated with OK-1, ICG-001, or C-82, and untreated cells are compared. A change in gene expression similar to Example 2 can be observed by comparing the gene expression levels.

### (Example 19-1: Checking gene function using gene KO cells)

Cells with knockout (KO) of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1 were prepared to study the role of the knocked out gene. KO cells can be prepared by using any genome editing technology.

Association with sarcopenia can be understood by checking the function of the KO gene.

### (Example 19-2: Checking gene function by compound treatment)

Knockdown (KD) cells were prepared using shRNA, which specifically inhibits the expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, and Cx3cr1 to study the role of the KD gene.

Association with sarcopenia can be understood by checking the function of the KD gene.

### (Example 20: Muscle amyotrophic lateral sclerosis (ALS), Alzheimer's disease, and diabetes related experiment)

### (ALS)

ALS mouse models (e.g., SOD1G93A mice) were divided into two groups. OK was administered to one of the groups, and OK was not administered to the other group. It can be understood that ALS is improved only in mice administered with OK-1 by comparing the two groups of mice.

### (Diabetes)

Diabetes mouse models (e.g., KK/TaJcl) were divided into two groups. OK was administered to one of the groups, and OK was not administered to the other group. It can be understood that at least one of insulin secretion and insulin resistance is improved only in mice administered with OK-1 by comparing the two groups of mice.

### (Metabolism and secretory system)

Mice induced to be obese by feeding high fat feed were divided into two groups. OK was administered to one of the groups, and OK was not administered to the other group. It can be understood that metabolism is improved only in mice administered with OK-1 by comparing the two groups of mice.

### (Example 21: Blood test on OK-1 treated mice)

75-week old mice (C57Bl6/J) were purchased from Oriental Yeast. Various concentrations (0 mg/kg (control), 0.2 mg/kg, 2 mg/kg, 20 mg/kg, and 40 mg/kg) of OK-1 were administered twice a week for 12 weeks until reaching 87-week old. Blood (plasma) was then recovered, and the concentrations of the following six markers were measured using a biochemical automatic analyzer (JCA-BM6050, JEOL).
LDH: lactate dehydrogenase can predict a liver, myocardial, or skeletal muscle disorder, etc. A decrease in LDH is expected to have a therapeutic effect on acute hepatitis, chronic hepatitis, cirrhosis, liver cancer, and other liver diseases, acute myocardial infarction, congested heart failure, and other heart diseases, polymyositis, muscular dystrophy, and other muscular diseases, pernicious anemia, leukemia, hemolytic anemia, etc.
ALT: This is a biomarker that can predict a liver tissue disorder. A decrease in ALT is expected to have a therapeutic effect on acute hepatitis, chronic hepatitis, alcoholic hepatitis, cirrhosis, liver cancer, fatty liver, acute myocardial infarction and other heart diseases, progressive muscular dystrophy and other muscular diseases, etc.
TG: Neutral fat is used for predicting diabetes, obesity, or arteriosclerotic disease. A decrease in TG is expected to have a therapeutic effect on hyperchylomicronemia, LPL deficiency, diabetes, obesity, arteriosclerosis, gout, hypothyroidism, Cushing's syndrome, nephrotic syndrome, obstructive jaundice, acute/chronic pancreatitis, etc.
HDL-C: This is used as a marker for arteriosclerosis, diabetes, cirrhosis, etc., of HDL cholesterol. A decrease in HDL-C is expected to have a therapeutic effect on primary biliary cirrhosis and hyperthyroidism.
UN: Urea nitrogen is a biomarker that can predict dysfunction of kidney or urinary system. An increase in UN is expected to have a therapeutic effect on toxic hepatitis, fulminant hepatitis, end-stage of cirrhosis, diabetes insipidus, acromegaly, etc.
UA: Uric acid is a biomarker for kidney or liver function. An increase in UA is expected to have a therapeutic effect on cirrhosis, xanthinuria, Fanconi syndrome, Wilson's syndrome, glomerulonephritis, etc.

### (Results)

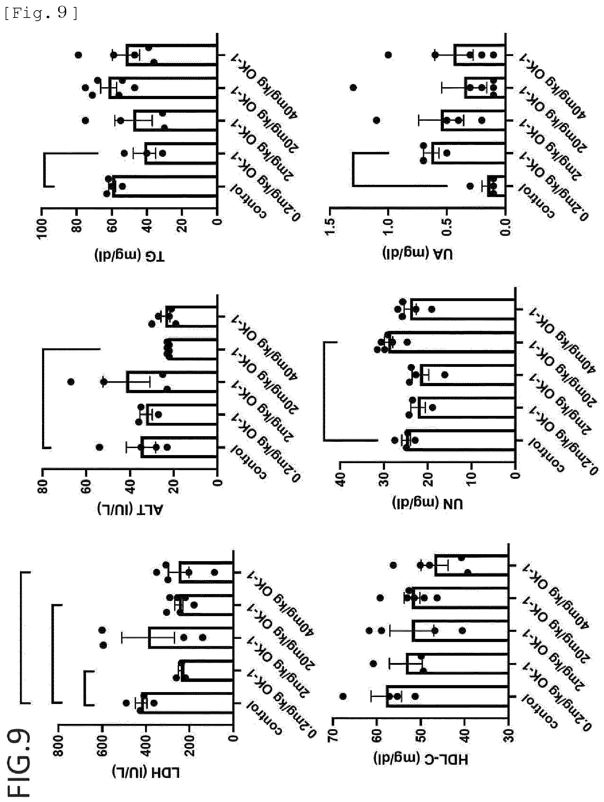

It was found that OK-1 treatment reduces the LDH concentration and increases the UA concentration at all concentrations. At a specific OK-1 concentration, a decrease in the ALT concentration, a decrease in TG concentration, and an increase in UN concentration were observed. A significant change was not observed in HDL-C (Figure 9).

### (Example 22: Change in gene expression in myoblasts due to C-82 treatment)

This Example investigated what gene modulation C-82 is involved with in myoblast C2C12 to identify a gene that is useful in the diagnosis or treatment of sarcopenia.

Gene analysis (RNA-seq) in C-82 treated myoblasts was performed by the same method as Example 2.

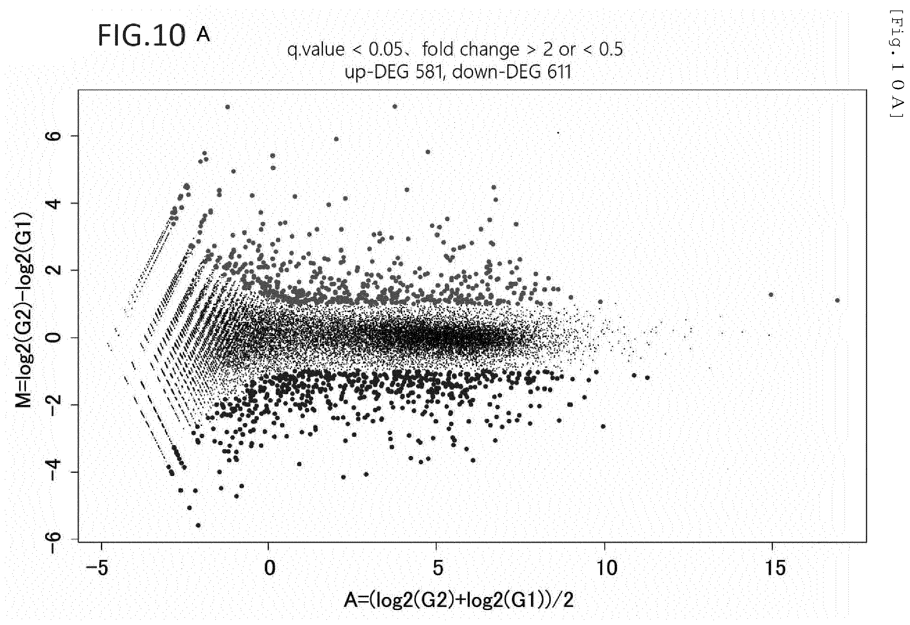

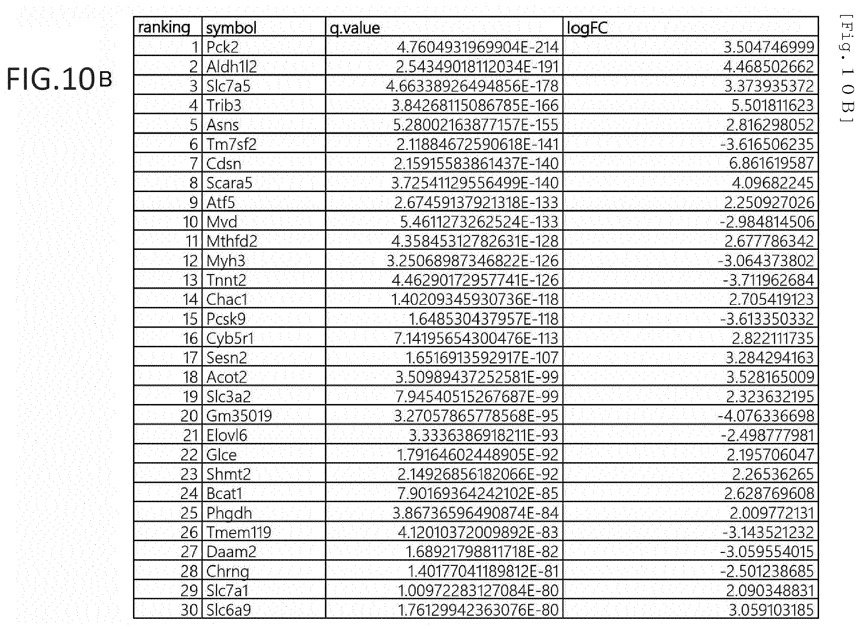

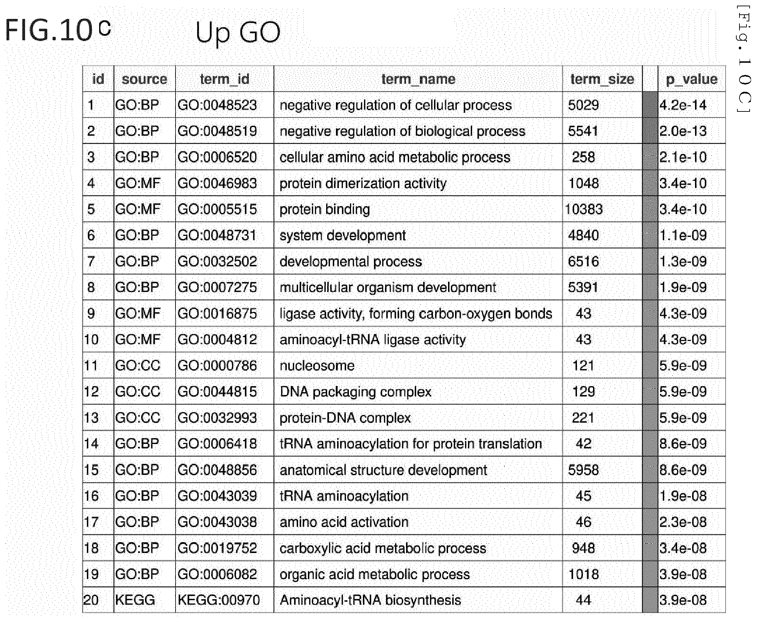

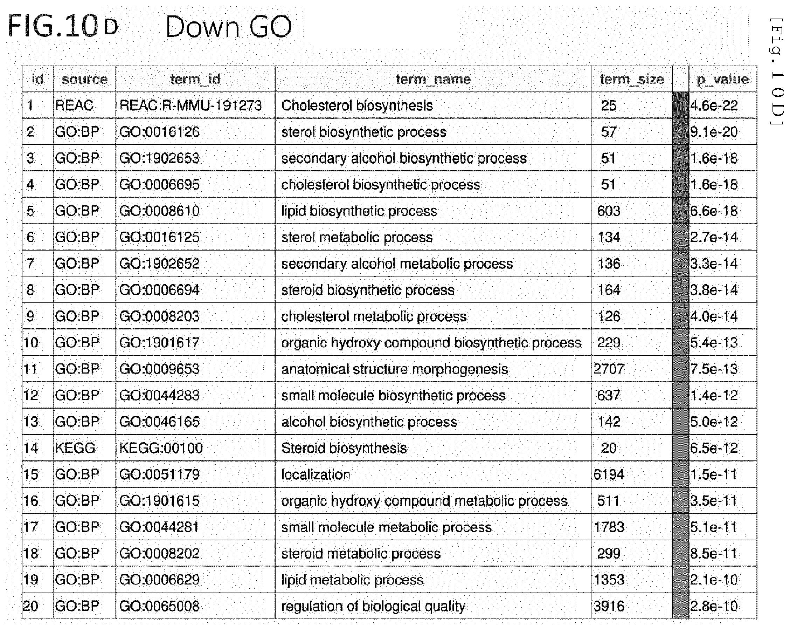

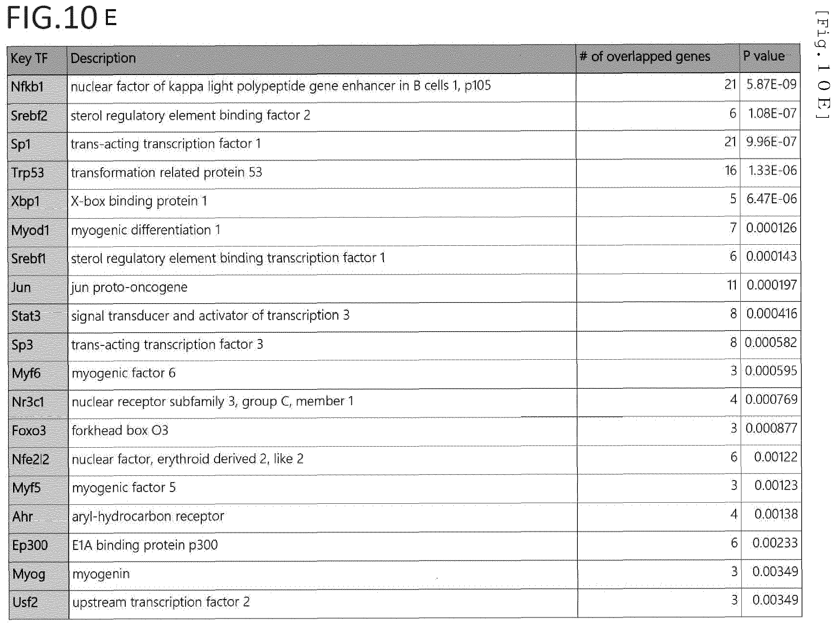

The result thereof is shown in Figure 10. 581 genes increased, and 611 genes decreased significantly in C-82 treated myoblasts (Figure **10A**). Figure 10B is a diagram showing the top 30 genes with a change in gene expression by treatment with C-82. Figures 10C to E show the categorization of biological functions (GO) with which these genes are involved (Figures **10C** and **10D****)** and the ranking of upstream transcription factors of genes with a change (Figure 10E).

Finally, in this aspect, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 were identified as a gene group associated with C-82. It was found from this upstream transcription factor analysis that Ep300 is in a C-82 related gene group and OK-1 related gene group, so the effect in C-82 cells and the effect in OK-1 muscle overlap in the expected path. It was found that results for OK-1 and C-82 cultured cells with exercise overlap.

C-82 related gene group is "genes associated with muscle control/genes that are useful in the diagnosis or treatment of sarcopenia".

### (Example 23: Example proving association with organ: 1 Test on functional aspect)

This Example investigated the effect of the compound of the present disclosure on an organ of a sarcopenia subject with regard to the functional aspect.

This Example used mouse models prepared in the Preparation Example.

Measurement can be taken using Kumamoto Mouse Clinic (KMC) of Kumamoto University.

Mouse models were divided into two groups. Cardiac function was tested for sarcopenia mouse models administered with C-82 and sarcopenia mouse models without administration of C-82. Cardiac function was tested and analyzed using an echocardiogram and treadmill. It can be understood from the results thereof that cardiac function is improved in mice administered with C-82 compared to mice without administration of C-82.

### (Example 24: Example proving association with organ: 2 Test on biochemical aspect)

This Example investigated the effect of the compound of the present disclosure on an organ of a sarcopenia subject with regard to the biochemical aspect.

This Example used mouse models prepared in the Preparation Example.

Mouse models were divided into two groups. The creatine concentration, IL-6 concentration, cholesterol concentration, UN (urea nitrogen is a biomarker that can predict the dysfunction of kidney or urinary system) concentration, and UA (uric acid is a biomarker for kidney or liver function) concentration were measured for sarcopenia mouse models administered with C-82 and sarcopenia mouse models without administration of C-82. It can be understood from the results thereof that kidney function and muscle improve in mice administered with C-82 compared to mice without administration of C-82.

### (Example 25: Example proving association with bone: 1 Test on functional aspect)

This Example investigated the effect of the compound of the present disclosure on a bone of a subject with regard to the functional aspect.

This Example tested bone density for mice administered with C-82 and mice without administration of C-82 in order to investigate the effect of C-82 on mouse bones. Bone density was measured using DEXA or ultrasound. It can be understood from the results thereof that bone density is improved in mice administered with C-82 compared to mice without administration of C-82.

### (Example 26: Example proving association with bone: 2 Test on biochemical aspect)

This Example investigated the effect of the compound of the present disclosure on a bone of a sarcopenia subject with regard to the biochemical aspect.

This Example used mouse models prepared in the Preparation Example.

Mouse models were divided into two groups. The NTX (cross linked N-telopeptide of type I collagen) concentration was measured for sarcopenia mouse models administered with C-82 and sarcopenia mouse models without administration of C-82. It can be understood from the results thereof that osteoporosis is improved in mice administered with C-82 compared to mice without administration of C-82.

### (Example 27: Example proving associated with an organ with a metabolic disease)

This Example investigated the effect of the compound of the present disclosure on an organ with a metabolic disease of a sarcopenia subject.

This Example used mouse models prepared in the Preparation Example.

Mouse models were divided into two groups. LDH concentration, ALT concentration, TG concentration, HDL-C concentration, insulin concentration, glucose concentration, amylase concentration, AST concentration, and ALT concentration were measured for sarcopenia mouse models administered with C-82 and sarcopenia mouse models without administration of C-82. It can be understood from the results thereof that pancreatic function, liver function, and salivary gland function are improved in mice administered with C-82 compared to mice without administration of C-82.

In addition thereto, the following are investigated as blood biomarkers and cytokines anticipated to have a correlation with a related disease: insulin or glucose (type II diabetes), BAP (bone alkaline phosphatase), NTX (cross linked N-telopeptide of type I collagen) (osteoporosis), creatine (kidney disease, muscle, or cerebral disorder), cytokine such as IL-6 and cholesterol (cancer and cardiovascular disease), AST and ALT (liver function), and amylase (pancreas or salivary gland).

In view of this, various diseases described within the parentheses can be diagnosed or treated, or a therapeutic effect can be assessed based on increase/decrease in each of the cytokines and biomarkers.

In addition thereof, the following can be measured as a biochemical test: TP (i.e., total protein), Alb (i.e., albumin), AST (GOT), ALP (i.e., alkaline phosphatase), LDH (i.e., lactate dehydrogenase), ALT (GPT), γ-GTP (i.e., glutamyl transpeptidase), CHE (i.e., cholinesterase), CPK (CK) (i.e., creatine kinase), CK-MB, AMY (i.e., amylase), T-Bil (i.e., total bilirubin), T-cho (i.e., total cholesterol), TG (i.e., neutral fat, triglyceride), LDL-C (i.e., LDL cholesterol), HDL-C (i.e., HDL cholesterol), BUN (i.e., urea nitrogen), UA (i.e., uric acid), Cre (i.e., creatinine), Na (i.e., sodium), K (i.e., potassium), Cl (i.e., chlorine), Ca (i.e., calcium), GLU (i.e., blood sugar or glucose), HbA1c (i.e., glycohemoglobin), procalcitonin (i.e., PCT), cardiac muscle troponin T (i.e., TnT), CRP (i.e., C reactive protein), and BNP (i.e., brain natriuretic polypeptide).

The following can be determined from the measurement thereof.
*TP (i.e., total protein)
High value
   polyclonal: autoimmune disease, chronic inflammatory disease, cirrhosis, malignant tumor, infection, etc.
   monoclonal: multiple myeloma, macroglobulinemia, etc.
Low value
   insufficient nutrition: insufficient nutrition intake, intestinal malabsorption syndrome, etc.
   liver disorder: acute hepatitis, cirrhosis, etc.
   protein losing: nephrotic syndrome, protein losing enteropathy, etc.
   hypermetabolism: acute infection, chronic wasting disease, etc.
   abnormal biodistribution: generalized edema, sunburn, etc.
*Alb (i.e., albumin)
High value
   hemoconcentration (dehydration)
Low value
   insufficient nutrition: insufficient nutrition intake, intestinal malabsorption syndrome, etc.
   liver disorder: acute hepatitis, cirrhosis, etc.
   protein losing: nephrotic syndrome, protein losing enteropathy, etc.
   hypermetabolism: acute infection, chronic wasting disease, etc.
   abnormal biodistribution: generalized edema, sunburn, etc.
*AST (GOT)
High value
   liver disease: acute hepatitis, chronic hepatitis, alcoholic hepatitis, cirrhosis, liver cancer, fatty liver, etc.
   heart disease: acute myocardial infarction, etc.
   muscular disease: progressive muscular dystrophy, polymyositis, myasthenia, etc.
   others: strenuous exercise, hemolysis, etc.
*ALP (i.e., alkaline phosphatase)
High value
   liver disease: acute hepatitis, chronic hepatitis, alcoholic hepatitis, cirrhosis, liver cancer, primary biliary cirrhosis, etc.
   biliary tract disease: bile duct cancer, choledocholithiasis, sclerosing cholangitis, etc.
   bone disease: osteomalacia, osteosarcoma, metastatic bone tumor, etc.
   others: childhood, third trimester, hyperthyroidism, uremia, etc.
*LDH (i.e., lactate dehydrogenase)
High value
   liver disease: acute hepatitis, chronic hepatitis, cirrhosis, liver cancer, etc.
   heart disease: acute myocardial infarction, congested heart failure, etc.
   muscular disease: polymyositis, muscular dystrophy, etc.
   others: pernicious anemia, leukemia, hemolytic anemia, etc.
*ALT(GPT)
High value
   liver disease: acute hepatitis, chronic hepatitis, alcoholic hepatitis, cirrhosis, liver cancer, fatty liver, etc.
   heart disease: acute myocardial infarction, etc.
   muscular disease: progressive muscular dystrophy, etc.
   others: cholestasis, infectious mononucleosis, etc.
*γ-GTP (i.e., glutamyl transpeptidase)
High value
   liver disease: alcoholic liver disease, alcoholic fatty liver, habitual drinking, chronic hepatitis, cirrhosis, liver cancer, etc.
   biliary tract disease: cholangitis, biliary atresia, biliary tract cancer, etc.
   others: gastric cancer, lung cancer, etc.
*CHE (i.e., cholinesterase)
High value
   nephrotic syndrome, fatty liver, diabetes, hyperthyroidism, etc.
Low value
   liver disease: acute hepatitis, chronic hepatitis, cirrhosis, liver cancer, etc.
   others: biliary obstruction, pancreatitis, drug addiction, etc.
*CPK (CK) (i.e., creatine kinase)
High value
   liver disease: progressive muscular dystrophy, polymyositis, dermatomyositis, etc.
   heart disease: acute myocardial infarction, myocarditis, etc.
   brain disease: cerebral thrombosis, cerebral infarction, brain damage, etc.
   others: hypothyroidism, malignant tumor, drug addiction, etc.
*CK-MB
High value
   acute myocardial infarction, rhabdomyolysis, polymyositis, muscular dystrophy, other skeletal muscle diseases, hypothyroidism, epilepsy, Reye's syndrome, and other convulsive disorders
*AMY (i.e., amylase)
High value
   pancreatic disease: acute pancreatitis, chronic pancreatitis, pancreatic cyst, etc.
   others: mumps, duodenal ulcer, ileus, peritonitis, ovarian cancer, renal failure, etc.
*T-Bil (i.e., total bilirubin)
High value
   hemolytic jaundice, neonatal jaundice, acute hepatitis, cirrhosis, primary biliary cirrhosis, intrahepatic biliary obstruction, choledocholithiasis, etc.
*T-cho (i.e., total cholesterol)
High value
   primary: familial hypercholesterolemia, etc.
   secondary: diabetes, nephrotic syndrome, arteriosclerosis, multiple myeloma, obstructive jaundice, hypothyroidism, pregnancy, etc.
Low value
   primary: abetalipoproteinemia, hypobetalipoproteinemia, LCAT deficiency, etc.
   secondary: hyperthyroidism, severe liver disorder, hypopituitarism, etc.
*TG (i.e., neutral fat, triglyceride)
High value
   primary: hyperchylomicronemia, LPL deficiency, etc.
   secondary: diabetes, obesity, arteriosclerosis, gout, hypothyroidism, Cushing's syndrome, nephrotic syndrome, obstructive jaundice, acute/chronic pancreatitis, etc.
Low value
   primary: abetalipoproteinemia, hypobetalipoproteinemia, etc.
   secondary: hyperthyroidism, hypopituitarism, cirrhosis, Addison's disease, etc.
*LDL-C (i.e., LDL cholesterol)
High value
   arteriosclerosis, nephrotic syndrome, diabetes, obesity, familial hypercholesterolemia, obstructive jaundice, etc. Low value
   cirrhosis, chronic hepatitis, hyperthyroidism, familial hypocholesterolemia, etc.
*HDL-C (i.e., HDL cholesterol)
High value
   primary: familial hyperalphalipoproteinemia, etc.
   secondary: obstructive pulmonary disease, primary biliary cirrhosis, alcohol consumption, exercise, etc.
Low value
   primary: LCAT deficiency, LPL deficiency, etc.
   secondary: diabetes, obesity, cerebral infarction, coronary arteriosclerosis, chronic renal failure, cirrhosis, thyroid dysfunction, smoking, etc.
*BUN (i.e., urea nitrogen)
High value
   prerenal: dehydration, severe heart failure, gastrointestinal bleeding, etc.
   renal: nephritis, uremia, nephrotic syndrome, kidney stone, etc.
   postrenal: ureteral obstruction, bladder tumor, etc. Low value
   toxic hepatitis, fulminant hepatitis, end-stage of cirrhosis, diabetes insipidus, acromegaly, etc.
*UA (i.e., uric acid)
High value
   primary gout: juvenile gout, etc.
   secondary gout: leukemia, hyperlipidemia, renal failure, etc.
Low value
   reduced biosynthesis: cirrhosis, xanthinuria, etc.
   secondary reduction: Fanconi syndrome, Wilson's syndrome, glomerulonephritis, etc.
*Cre (i.e., creatinine)
High value
   reduced GFR (glomerular filtration rate): glomerulonephritis, renal failure, etc.
   hemoconcentration: dehydration, burn, etc.
Low value
   increased amount of urinary extraction: diabetes insipidus, pregnancy, etc.
   muscle atrophy: muscular dystrophy, etc.
*Na (i.e., sodium)
High value
   vomiting, diarrhea, hyperhidrosis (heat stroke), diabetes insipidus, hypercalcemia, Cushing's syndrome, dry mouth sensation disorder, etc.
Low value
   renal failure, nephrotic syndrome, heart failure, cirrhosis, Addison's disease, pregnancy-induced hypertension, diuretic/antibiotic administration, etc.
*K (i.e., potassium)
High value
   Migration from within a cell: metabolic acidosis, insulin deficiency, effect of drug, etc.
   reduced excretion in kidney: renal failure, Addison's disease, hypoaldosteronism, etc.
   others: hemolysis, leukocytosis, thrombocytosis, etc.
Low value
   Migration into a cell: metabolic alkalosis, insulin administration, high concentration transfusion, etc.
   Loss from digestive tract: vomiting, diarrhea, malabsorption syndrome, etc.
   Loss from kidney: primary aldosteronism, Cushing's syndrome, effect of diuretic, etc.
*Cl (i.e., chlorine)
High value
   nephrotic syndrome, renal failure, Cushing's syndrome, dehydration, excessive salt intake, etc.
Low value
   intense vomiting, Addison's disease, diabetes insipidus, insufficient salt intake, etc.
*Ca (i.e., calcium)
High value
   milk-alkali syndrome, multiple myeloma, hyperthyroidism, primary hyperparathyroidism, acute renal failure, pheochromocytoma, familial hypocalciuric hypercalcemia, malignant tumor bone metastasis, Addison's disease Low value
   vitamin D deficiency, malabsorption syndrome, acute pancreatitis, hypomagnesemia, uremia, hypoparathyroidism, chronic renal failure, nephrotic syndrome
*GLU (i.e., blood sugar, glucose)
High value
   diabetes (type I, type II, etc.)
   pancreatic disease: pancreatic cancer, pancreatitis, etc.
   endocrine disease: acromegaly, hyperthyroidism, Cushing's syndrome, etc.
   others: post-gastrectomy, stress, obesity, etc.
Low value
   pancreatic disease: insulinoma (excess insulin secretion)
   liver disease: cirrhosis, liver cancer, etc.
   endocrine disease: hypothyroidism, hypopituitarism
   others: excessive insulin injection, use of oral hypoglycemic agent, strenuous exercise, fasting, etc.
*HbA1c (i.e., glycohemoglobin)
High value
   diabetes, other diseases exhibiting hyperglycemia, etc. Low value
   decrease in erythrocyte lifespan due to hemolytic anemia, hemorrhage, etc., resulting in increased reticulocyte, etc.
*procalcitonin (i.e., PCT)
High value
   Bacterial infection exhibiting systemic inflammatory response, sepsis, systemic fungal infection, pancreatitis, etc.
Low value
   viral infection, chronic systemic inflammation (rheumatoid arthritis, vasculitis, etc.), autoimmune disease, tumor, locally confirmed bacterial infection, etc.
*Cardiac muscle troponin T (i.e., TnT)
High value
   acute myocardial infarction, angina pectoris, myocarditis, etc.
*CRP (i.e., C reactive protein)
High value
   infection, malignant tumor, autoimmune disease, necrosis of tissue, inflammatory disease, etc.
*BNP (i.e., brain natriuretic polypeptide)
High value
   heart failure, myocardial infarction, hypertension, chronic renal failure, nephrotic syndrome, Cushing's syndrome, hyperthyroidism, etc.
Low value
   dehydrated state, effect of diuretic, etc.

The various cytokines, etc., described above can also be used to prepare genetically modified mice (Trans Genic, Kumamoto, Japan).

Cytokine, etc., can be tested by using a commercially available kit (e.g., Bio-Plex Pro Human Cytokine Assay Panel (Bio-Rad)).

### (Example 28: Example proving association with brain function: 1 Functional aspect)

This Example investigated the effect of the compound of the present disclosure on the brain of a sarcopenia subject with regard to the functional aspect.

This Example used mouse models prepared in the Preparation Example.

Mouse models were divided into two groups. Short term memory, long term memory, depression, activity level, social interaction, cognitive ability, etc., were tested using a Barnes maze, fear conditioning test, object recognition test, open field test, social behavior test (Crawley version), etc. on sarcopenia mouse models administered with C-82 and sarcopenia mouse models without administration of C-82.

These tests are described in http://www.fujita-hu.ac.jp/ to smedsci/behaviorcore/equipment/index.html, Barnes maze, a useful task to assess spatial reference memory in the mice (doi: 10.1038/nprot.2007.390), etc., which can be suitably referenced and practiced.

It can be understood that short term memory, long term memory, depression, activity level, social interaction, and cognitive ability are improved in mice administered with C-82 compared to mice without administration of C-82.

### (Example 29: Example proving association with brain function: 2 Functional aspect)

This Example investigated the effect of the compound of the present disclosure on the brain of a subject with regard to the biochemical aspect.

This Example investigated whether suppression in phosphorylation of Tau and improvement in memory or brain atrophy are observed in mice administered with C-82 and mice without administration of C-82 by using P301S mouse models with advanced Tau phosphorylation and brain atrophy, which are pathological conditions of Alzheimer's (FTDP-17 mouse model; Yoshiyama, Clinical Neurology, 48: 910-912, 2008). It can be understood from the results thereof that suppression in phosphorylation of Tau and improvement in memory or brain atrophy are observed in mice administered with C-82 and mice without administration of C-82.

This Example demonstrated whether the compound is effective on tauopathy.

It can be demonstrated whether the compound can be applied to various neurodegenerative diseases known as tauopathy through treatment of tau phosphorylation. Tauopathy refers to a condition wherein a tau protein, which is a type of microtubule-associated protein, is accumulated within a cell. Tauopathy includes Alzheimer's disease, as well as progressive supranuclear palsy, corticobasal degeneration, Pick's disease, etc.

### (Example 30: Change in gene expression in muscles by C-82 administration in mice without exercise)

This Example investigated what gene modulation C-82 of the compound of the present disclosure is involved in to identify a gene that is useful in the diagnosis or treatment of sarcopenia in mouse models by the same method as Example 2.

It was found as a result thereof that administration of C-82 achieves the same result as OK-1.

### (Example 31: Change in gene expression in muscles by C-82 administration in mice with exercise)

This Example investigated what gene modulation C-82 of the compound of the present disclosure is involved in to identify a gene that is useful in the diagnosis or treatment of sarcopenia in mouse models by the same method as Example 2.

It was found as a result thereof that administration of C-82 achieves the same result as OK-1.

### (Example 32: Disease and differentially expressed gene of muscle through C-82 administration in mice with/without exercise)

Diseases associated with the top 100 genes observed to have differential expression in mice with/without exercise were studied by collation with NIH MedlinePlus. It was found therefrom that administration of C-82 achieves the same result as OK-1.

### (Example 33: Disease and differentially expressed gene of muscle through C-82 administration in mice with/without exercise)

Diseases associated with the top 100 genes observed to have differential expression in mice with/without exercise were studied by collation with NIH MedlinePlus. It was found therefrom that administration of C-82 achieves the same result as OK-1.

### (Example 34: Change in muscle strength (endurance)/grip strength due to administration of C-82)

### Grip strength test and run experiment

74-week old mice (C57B16/J) were purchased from Oriental Yeast. Various concentrations (0 mg/kg (control), 7 mg/kg, and 20 mg/kg) of C-82 were administered twice a week through a 6-week intraperitoneal administration until reaching 80-week old. Muscle strength (endurance) and grip strength of mice administered with C-82 were then measured through a treadmill test and smart grip strength meter for rats and mice (Muromachi Kikai Co., Ltd.), respectively. The treadmill test was conducted under the same condition as Example 1.

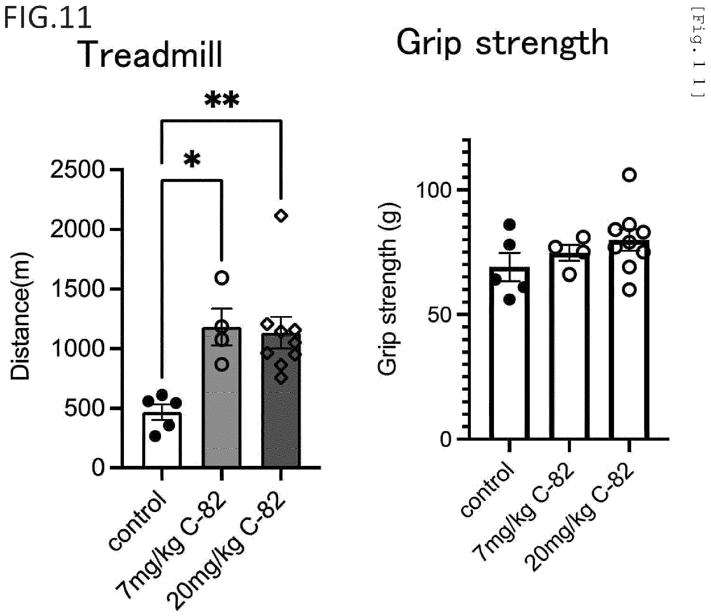

As a result, mice administered with C-82 had a significant increase in muscle strength (endurance) compared to mice without administration. While a significant difference was not observed in grip strength, a tendency for a concentration dependent increase was observed (Figure 11).

As described above, the present disclosure is exemplified by the use of its preferred embodiments. It is understood that the scope of the present disclosure should be interpreted solely based on the claims. It is understood that any patent, any patent application, and any references cited herein should be incorporated herein by reference in the same manner as the contents are specifically described herein. The present application claims priority to Japanese Patent Application No. 2020-182813 filed with the JPO on October 30, 2020. The entire content thereof is incorporated herein by reference in its entirety.

### [Industrial Applicability]

The present disclosure can improve a sarcopenia related disease, disorder, or symptom and has found utility thereof in related industries.

## Claims

1. A composition for modulating, slowing the progression of, preventing, or treating a sarcopenia related disease, disorder, or symptom, comprising a compound or a pharmaceutically acceptable salt thereof, or a solvate thereof, wherein the compound is a compound represented by [1] formula (I) 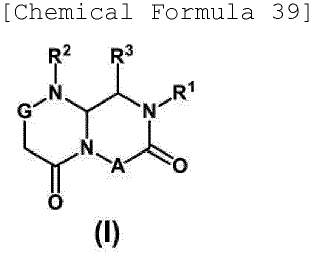 wherein
A is -CHR⁷- (wherein R⁷ is C₁₋₁₀ alkyl optionally substituted with a substituent selected from OH, -OR⁸ (wherein R⁸ represents C₁₋₁₀ alkyl), -NH₂, -COOH, and -CONH₂, C₆₋₁₄ aryl-C₁₋₁₀ alkyl optionally substituted with a substituent selected from -OH, -OP(=O)(OH)₂, -OP(=O)(ONa)₂, and C₁₋₁₀ alkyl, heteroaryl-C₁₋₁₀ alkyl, C₃₋₁₀ cycloalkyl-C₁₋₁₀ alkyl, C₆₋₁₄ aryl, or C₃₋₁₀ cycloalkyl,
G is -NR⁶- or -O- (wherein R⁶ is independently selected from C₁₋₁₀ alkyl and C₂₋₁₀ alkenyl),
R¹ is -Ra-R¹⁰ (wherein Ra is C₁₋₆ alkylene, and R¹⁰ is naphthyl or fused bicyclic heteroaryl optionally substituted with a substituent selected from -NH₂ and halogen,
R² is -W²¹-W²²-Rb-R²⁰ (wherein W²¹ is -(CO)-, W²² is -O- or -NH-, Rb is a bond or C₁₋₆ alkylene optionally substituted with C₁₋₁₀ alkyl, and R²⁰ is C₁₋₁₀ alkyl, C₆₋₁₄ aryl optionally substituted with halogen, heteroaryl, or C₃₋₁₀ cycloalkyl), and
R³ is C₁₋₁₀ alkyl,
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon,
a compound represented by 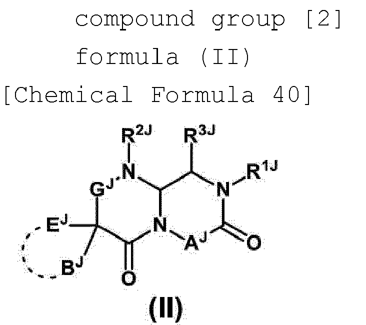 wherein
A^{J} is -(CHR^{7J})- (wherein R^{7J} is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
B^{J} and E^{J} are the same or different, independently selected from hydrogen, optionally substituted alkyl, optionally substituted alkenyl, and optionally substituted alkynyl, or form an optionally substituted spiro ring represented by a dotted line,
G^{J} is -NH-, -NR^{6J}-, -O-, -CH₂-, -CHR^{6J}-, or -C(R^{6J})₂-(wherein R^{6J} is each the same or different, independently selected from optionally substituted alkyl, optionally substituted alkenyl, and optionally substituted alkynyl),
R^{1J} is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R^{2J} is -W^{21J}-W^{22J}-Rb^{J}-R^{20J} (wherein W^{21J} is -(CO)- or -(SO₂)-, W^{22J} is a bond, -O-, -NH-, or optionally substituted lower alkylene, Rb^{J} is a bond or optionally substituted lower alkylene, and R^{20J} is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R^{3J} is hydrogen, optionally substituted alkyl, optionally substituted alkenyl, or optionally substituted alkynyl],
a compound represented by 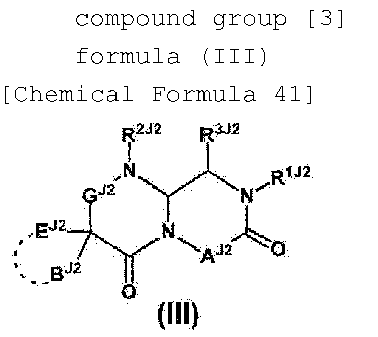 wherein
A^{J2} is -(CHR^{7J2})- (wherein R^{7J2} is C₁₋₁₀ alkyl optionally substituted with OH, -OR^{8J2} (wherein R^{8J2} represents C₆₋₁₄ aryl-C₁₋₁₀ alkyl), -NH₂, -COOH, and -CONH₂, C₆₋₁₄ aryl, or C₆₋₁₄ aryl-C₁₋₁₀ alkyl optionally substituted with OH) ;
B^{J2} and E^{J2} are hydrogen,
G^{J2} is -O-,
R^{1J2} is C₁₋₁₀ alkyl optionally substituted with a substituent selected from OH, -COOH, and -COOR^{8J2'} (wherein R^{8J2'} represents C₁₋₁₀ alkyl), C₆₋₁₄ aryl-C₁₋₁₀ alkyl, heteroaryl-C₁₋₁₀ alkyl optionally substituted with -NH₂, or C₃₋₁₀ cycloalkyl-C₁₋₁₀ alkyl,
R^{2J2} is -W^{21J2}-W^{22J2}-Rb^{J2}-R^{20J2} (wherein W^{21J2} is -(CO)- or - (SO₂)-, W^{22J2} is a bond, -O-, or C₁₋₆ alkylene, and Rb^{J2} is a bond or C₁₋₆ alkylene); and
R^{20J2} is C₁₋₁₀ alkyl, C₆₋₁₄ aryl optionally substituted with halogen, or heteroaryl; and
R^{3J2} is hydrogen;
wherein "heteroaryl" refers to a 5- to 14-membered monocyclic or polycyclic aromatic group wherein at least one ring atom is a heteroatom selected from nitrogen, oxygen, and sulfur, and the rest of the ring atoms is carbon,
compound represented by 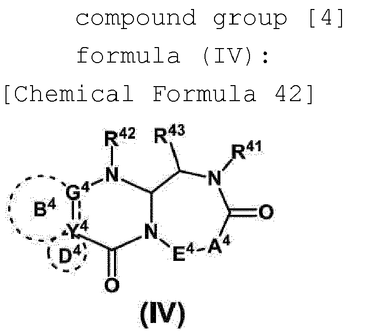 wherein 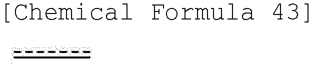 is a single bond or a double bond,
A⁴ is -CHR⁴⁷- (wherein R⁴⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
E⁴ is a bond, -CHR⁴⁵-, -O-, or -NR⁴⁸- (wherein R⁴⁵ is hydrogen, optionally substituted alkyl, optionally substituted alkenyl, optionally substituted alkynyl, optionally substituted arylalkyl, or optionally substituted heteroarylalkyl, and R⁴⁸ is hydrogen, optionally substituted alkyl, optionally substituted alkenyl, or optionally substituted alkynyl),
B⁴ is absent, or an optionally substituted monocyclic ring formed together with G⁴ and Y⁴,
D⁴ is absent, or an optionally substituted spiro ring formed together with Y⁴,
with the proviso that not both of B⁴ and D⁴ are present,
if B⁴ is present, G⁴ and Y⁴ are independently a carbon atom or a nitrogen atom,
if D⁴ is present, Y⁴ is a carbon atom, and G⁴ is -NR⁴⁶-, -O-, -CHR⁴⁶-, or -C(R⁴⁶)₂-,
if both B⁴ and D⁴ are absent, G⁴ and Y⁴ are the same or different, each -NR⁴⁶-, -O-, -CHR⁴⁶-, or -C(R⁴⁶)₂- (wherein R⁴⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl), and
if E⁴ is a bond, D⁴ is absent, B⁴ is an optionally substituted monocyclic ring, and B⁴ and Y⁴ are independently a carbon atom or a nitrogen atom,
R⁴¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁴² is -W⁴²¹-W⁴²²-Rb⁴-R⁴²⁰ (wherein W⁴²¹ is -(CO)- or -(SO₂)-, W⁴²² is a bond, -O-, -NH-, or optionally substituted lower alkylene, Rb⁴ is a bond or optionally substituted alkylene, and R⁴²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁴³ is hydrogen, optionally substituted alkyl, optionally substituted alkenyl, or optionally substituted alkynyl,
a compound represented by 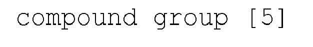 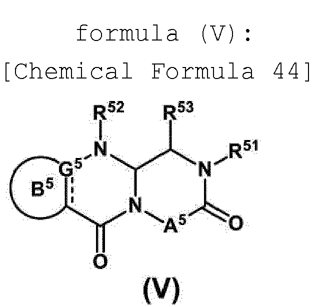 wherein 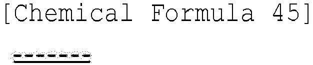 is a single bond or a double bond,
A⁵ is -(CHR⁵⁷)- (wherein R⁵⁷ is C₁₋₁₀ alkyl optionally substituted with a substituent selected from -OH, -OR⁵⁹ (wherein R⁵⁹ represents C₁₋₁₀ alkyl), -NH₂, -COOH, and -CONH₂, C₆₋₁₄ aryl, or C₆₋₁₄ aryl-C₁₋₁₀ alkyl optionally substituted with -OH),
B⁵ is a 5- or 6-membered monocyclic saturated carbon ring, or a saturated or unsaturated 4-, 5-, 6-, or 7-membered heterocyclic ring optionally substituted with a formyl group, formed together with G⁵, wherein a heteroatom is selected from S, N, and O, and the number of heteroatoms is an integer from 1 to 3,
G⁵ is a carbon atom or a nitrogen atom,
R⁵¹ is C₁₋₁₀ alkyl, C₆₋₁₄ aryl-C₁₋₁₀ alkyl optionally substituted with a substituent selected from -R⁵⁹' and halogen, or 5- to 14-membered heteroaryl-C₁₋₁₀ alkyl optionally substituted with -NHCOOR⁵⁹', wherein R⁵⁹' is C₁₋₁₀ alkyl or C₆₋₁₄ aryl,
R⁵² is -W⁵²¹-W⁵²²-Rb⁵-R⁵²⁰ (wherein
W⁵²¹ is -(CO)-,
W⁵²² is -NH-,
Rb⁵ is a bond, or C₁₋₆ alkylene optionally substituted with C₆₋₁₄ aryl, and
R⁵²⁰ is C₁₋₁₀ alkyl optionally substituted with a substituent selected from -OH and -COOR^{59"} (wherein R^{59"} represents a hydrogen atom or C₁₋₁₀ alkyl), C₆₋₁₄ aryl optionally substituted with a substituent selected from halogen, methylenedioxy, and C₁₋₁₀ alkyl, 5- to 14-membered heteroaryl, or C₃₋₁₀ cycloalkyl), and
R⁵³ is hydrogen or C₁₋₁₀ alkyl,
a compound represented by 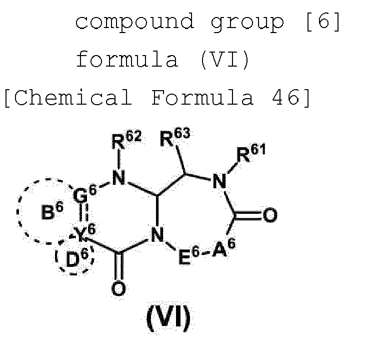 wherein 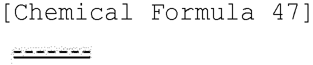 is a single bond or a double bond,
A is -CHR⁶⁷- (wherein R⁶⁷ is hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, optionally substituted heteroarylalkyl, optionally substituted cycloalkylalkyl, or optionally substituted heterocycloalkylalkyl),
E⁶ is -CHR⁶⁵-, -O-, or -NR⁶⁸- (wherein R⁶⁵ is hydrogen, optionally substituted alkyl, optionally substituted alkenyl, optionally substituted alkynyl, optionally substituted arylalkyl, or optionally substituted heteroarylalkyl, and R⁶⁸ is hydrogen, optionally substituted alkyl, optionally substituted alkenyl, or optionally substituted alkynyl),
B⁶ is absent or an optionally substituted monocyclic ring formed together with G⁶ and Y⁶,
D⁶ is absent, or an optionally substituted spiro ring formed together with Y⁶,
with the proviso that not both B⁶ and D⁶ are present,
if B⁶ is present, G⁶ and Y⁶ are independently a carbon atom or a nitrogen atom,
if D⁶ is present, Y⁶ is a carbon atom, and G⁶ is -NR⁶⁶-, -O-, -CHR⁶⁶-, or -C(R⁶⁶)₂-,
if B⁶ and D⁶ are both absent, G⁶ and Y⁶ are the same or different, each -NR⁶⁶-, -O-, -CHR⁶⁶-, or -C(R⁶⁶)₂- (wherein R⁶⁶ is each independently hydrogen, optionally substituted alkyl, optionally substituted alkenyl, optionally substituted alkynyl, optionally substituted aryl, optionally substituted heteroaryl, optionally substituted cycloalkyl, optionally substituted heterocycloalkyl, optionally substituted arylalkyl, or optionally substituted heteroarylalkyl),
R⁶¹ is optionally substituted alkyl, optionally substituted alkenyl, optionally substituted alkynyl, optionally substituted arylalkyl, optionally substituted heteroarylalkyl, optionally substituted cycloalkylalkyl, or optionally substituted heterocycloalkylalkyl,
R⁶² is -W⁶²¹-W⁶²²-Rb⁶-R⁶²⁰ (wherein W⁶²¹ is -(CO)- or -(SO₂)-, W⁶²² is a bond, -O-, -NH-, or optionally substituted lower alkylene, Rb⁶ is a bond or optionally substituted alkylene, and R⁶²⁰ is optionally substituted alkyl, optionally substituted alkenyl, optionally substituted alkynyl, optionally substituted aryl, optionally substituted heteroaryl, optionally substituted cycloalkyl, or optionally substituted heterocycloalkyl), and
R⁶³ is hydrogen, optionally substituted alkyl, optionally substituted alkenyl, or optionally substituted alkynyl,
a compound represented by 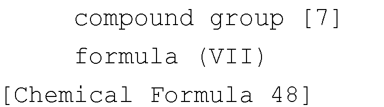 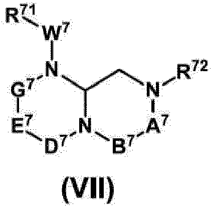 wherein
A⁷ is -(CHR⁷³)-(C=O)-, B⁷ is -(NR⁷⁴)-, D⁷ is -(CHR⁷⁵)- or -(C=O)-, E⁷ is -(Z⁷R⁷⁶)- or -(C=O)-, G⁷ is -(X⁷R⁷⁷)ₙ₇-, -(CHR⁷⁷)-(NR⁷⁸)-, -(C=O)-(X⁷R⁷⁹)-, or -(C=O)-, W⁷ is -Y⁷(C=O)-, - (C=O)NH-, -(SO₂)-, or absent, Y⁷ is oxygen or sulfur, X⁷ and Z⁷ are independently nitrogen or CH, n7 = 0 or 1, and R⁷¹, R⁷², R⁷³, R⁷⁴, R⁷⁵, R⁷⁶, R⁷⁷, R⁷⁸, and R⁷⁹ are the same or different, independently selected from an amino acid side chain, a derivative of an amino acid side chain, a linker facilitating the linkage of the compound to another moiety or compound, a linker attaching the compound to a solid support, and a solid support, and stereoisomers thereof,
a compound represented by 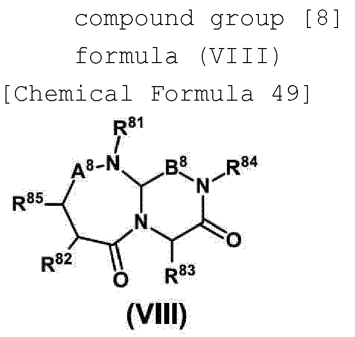 wherein
A⁸ is -(CHR⁸')ₙ₈-, B⁸ is -(CHR^{8"})ₘ₈-, n8 is 0, 1, or 2, m8 is 1, 2, or 3, R^{8'}, R^{8"}, R⁸², R⁸³, and R⁸⁵ are the same or different, each of R^{8'}, R^{8"}, R⁸², R⁸³, and R⁸⁵ that are present is independently selected from the group consisting of an amino acid side chain moiety, an amino acid side chain derivative, a linker, and a solid support, R⁸¹ and R⁸⁴ represent the remaining portions of the compound, wherein R⁸¹ and R⁸⁴ are the same or different, selected from the group consisting of a certain moiety, an active substance, a compound, a support, a molecule, a linker, an amino acid, a peptide, and a protein, or a compound represented by 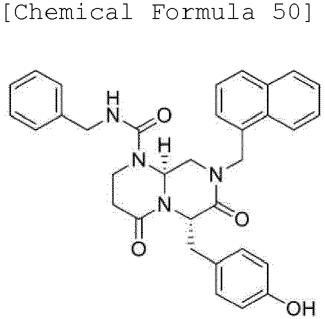 or a derivative thereof, or
a compound represented by 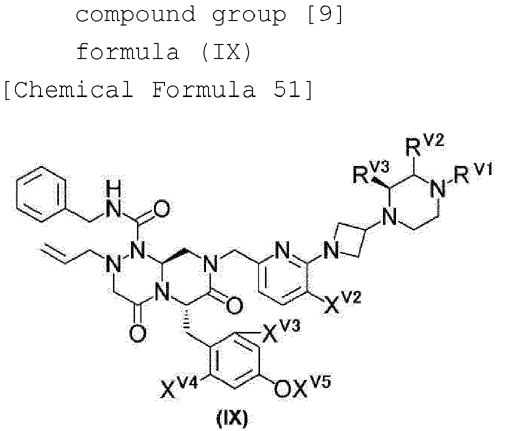 wherein R^{V1} refers to a C₁₋₆ alkyl group, R^{V2} and R^{V3} are each the same or different, referring to a hydrogen atom or a C₁₋₆ alkyl group, X^{V2}, X^{V3}, and X^{V4} are each the same or different, referring to a hydrogen atom or a halogen atom, and X^{V5} refers to a hydrogen atom or -P(=O)(OH)₂.

2. The composition of claim 1, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

3. The composition of claim 1 or 2, wherein the sarcopenia related disease, disorder, or symptom comprises at least one selected from the group consisting of primary sarcopenia, secondary sarcopenia, and a disease, disorder, or symptom accompanied therewith.

4. The composition of any one of claims 1 to 3, wherein the sarcopenia related disease, disorder, or symptom is selected from the group consisting of a sarcopenia associated life-style disease, a wasting disease, a tumor associated disease, a motor disease, a neurodegenerative disease, cognitive dysfunction, and other associated disorders.

5. The composition of any one of claims 1 to 4, wherein the sarcopenia related disease, disorder, or symptom is of a system selected from the group consisting of a digestive system, a circulatory system, a respiratory system, a urinary system, a reproductive system, an endocrine system, a sensory nervous system, a cranial nervous system, and a locomotor system.

6. The composition of any one of claims 1 to 5, wherein the compound is selected from the compound group [1].

7. The composition of any one of claims 1 to 6, wherein the compound or pharmaceutically acceptable salt thereof, or solvate thereof, can be orally administrated.

8. The composition of any one of claims 1 to 7, wherein the composition is administered in combination with at least one of physical therapy, dietary therapy, and other drug therapy.

9. The composition of any one of claims 1 to 8, wherein the composition is administered in combination with physical therapy.

10. The composition of any one of claims 1 to 9, wherein the modulation, slowing of progression, prevention, or treatment comprises modulation, slowing of progression, prevention, or treatment in at least one of reduced muscle mass, reduced (muscular) endurance, reduced short-term memory, reduced bone density, and osteoporosis.

11. The composition of any one of claims 1 to 10, wherein the modulation, slowing of progression, prevention, or treatment comprises modulation, slowing of progression, prevention, or treatment in at least one of reduced muscle mass and reduced (muscular) endurance.

12. The composition of any one of claims 1 to 11, wherein the modulation, slowing of progression, prevention, or treatment is confirmed by modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

13. The composition of claim 12, wherein the modulation, slowing of progression, prevention, or treatment is confirmed by modulation of at least one gene selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, Lox, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

14. A composition for use in diagnosis or testing for a sarcopenia related disease, disorder, or symptom of a subject, wherein the composition comprises means or a reagent for identifying the presence/absence or level of expression of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

15. The composition of claim 14, wherein the composition comprises means or a reagent for identifying the presence/absence or level of expression of at least one selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, Lox, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

16. A composition for modulating, slowing the progression of, preventing, or treating a sarcopenia related disease, disorder, or symptom, comprising a substance or agent for modulating at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Col1a1, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

17. The composition of claim 16, wherein the composition comprises a substance or agent for modulating at least one gene selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Col1a1, Itgb3, Lamb1, Nid1, Ecm2, Mmp14, Scara3, Loxl2, Col14a1, Col22a1, Scx, Fbln1, Myh11, Abi3bp, Col8a1, Smoc2, Tnxb, Lox, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

18. The composition of any one of claims 1 to 11 for modulating, slowing the progression of, preventing, or treating a sarcopenia related disease, disorder, or symptom of a subject, wherein the composition is administered when it is determined to be necessary or suitable based on a status of modulation, which is checked for at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Col1a1, Col14a1, C3ar1, Lepr, P3h3, Fn1, Ogn, Igkv4-70, Il33, Nbl1, Gm26910, Pzp, 4933406L23Rik, Fbln1, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject.

19. The composition of claim 18, wherein a status of modulation of at least one gene selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, Lox, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2, is checked.

20. The composition of claim 18 or 19, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

21. The composition of any one of claims 18 to 20, wherein the sarcopenia related disease, disorder, or symptom comprises at least one selected from the group consisting of primary sarcopenia, secondary sarcopenia, and a disease, disorder, or symptom accompanied therewith.

22. The composition of any one of claims 18 to 21, wherein the sarcopenia related disease, disorder, or symptom is selected from a sarcopenia associated life-style disease, a wasting disease, a tumor associated disease, a motor disease, a neurodegenerative disease, cognitive dysfunction, and other associated disorders.

23. The composition of any one of claims 18 to 22, wherein the sarcopenia related disease, disorder, or symptom is of a system selected from the group consisting of a digestive system, a circulatory system, a respiratory system, a urinary system, a reproductive system, an endocrine system, a sensory nervous system, a cranial nervous system, and a locomotor system.

24. The composition of any one of claims 1 to 13 and 16 to 23, wherein the compound, substance, or agent comprises OK-1. 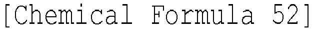 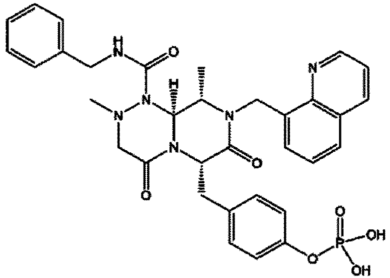 or
C-82 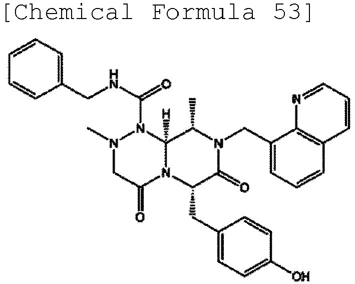 or a pharmaceutically acceptable salt thereof, or a solvate thereof.

25. A method for screening a substance or agent for modulating, slowing the progression of, preventing, or treating a sarcopenia related disease, disorder, or symptom, the method comprising:
A) administering a candidate substance or agent to a subject;
B) checking modulation of at least one gene selected from the group consisting of Ppara, Pou2f2, Foxo1, Pparg, Pou2f1, Trp63, Sp1, Tcf3, Zeb1, Srebf1, Sp3, Ebf1, Mycn, Pbx1, IRF1, NFE2L2, PPARG, FOXO1, CEBPB, PPARA, CEBPA, STAT1, STAT5A, MYOD1, REL, NFKB1, SPI1, IRF8, SP1, FOXM1, Krt27, Krt71, Dsp, Krt72, Krt17, Krtap15, Krtap7-1, Comp, Krt31, Krt33b, Pkp1, Prr9, Krt34, Trim29, Krt86, Krt26, Krtap1-5, Krt81, Krtap22-2, Tchhl1, Krtap6-5, Gprc5d, Krt73, Krt35, Krt83, Pof1b, Krtap6-1, Krtap14, 1110025L11Rik, Krtap8-1, Gm10229, Krt33a, Crct1, Igkv5-39, Tgtp1, Dsc1, Dsg1a, Serpinb5, Krt14, Krtap21-1, Abca12, Gm10228, Krt77, S100a14, Gm49425, Krtap19-3, Ighg1, Krt25, Tchh, Krtap1-3, Jun, Ets1, Sp1, Ep300, Usf2, Fosl1, Nfkb1, Smad3, Srf, Junb, Runx2, Ets2, Etv4, Tgfb1, Tgfb1i1, ETS2, ETS1, EP300, RUNX2, SMAD3, SMAD4, E2F1, NOTCH1, TRP53, PAX6, SMAD1, SP1, SPI1, NFKB1, Thbs2, Igkv12-41, Cpxm2, Cd248, Igsf10, Igkv4-69, Igkv10-96, Lox, Collal, Col14a1, C3ar1, Lepr, P3h3, Fn1, Ogn, Igkv4-70, Il33, Nbl1, Gm26910, Pzp, 4933406L23Rik, Fblnl, Mfap5, Cnn1, Nnat, Cd109, Igfbp6, Ppil3, Nmrk2, Plxnb2, Qpct, Gm22771, Rin2, Col1a2, Gm23935, Gm43154, Marcks, Dntt, Gpnmb, Pcolce, Gm45481, Colec12, Igkv3-4, Gm47381, Gm24270, Dpysl3, Lpar1, Loxl2, Pitpnm2os2, Emilin1, Dsp, Krt17, Comp, Krt86, Krt81, Krt83, Krt14, Abca12, Tchh, Krt5, Dsg4, Lpl, Myh6, Tfap2a, Collal, Lepr, Fn1, Col1a2, Col6a1, Col3a1, Mmp2, Acta2, F13a1, Col6a2, Cx3cr1, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2 of the subject, and
C) identifying the candidate substance or agent as a substance or agent that can have an ability to modulate, slow the progression of, prevent, or treat a sarcopenia related disease, disorder, or symptom based on a result of B) .

26. The method of claim 25, wherein B) checks modulation of at least one gene selected from the group consisting of Pkp1, Sprr1a, Lce1a1, Lce1b, Lce1a2, Lce1c, Lce1m, Rptn, Tchh, Klk5, Pkp3, Gm45618, Gm45337, Gm4553, Gm7579, Krtap5-5, Gm40460, Perp, Gm19402, Krtap12-1, Gm18596, Krt25, Krt26, Krt27, Krt28, Krt23, Krt39, Krt40, Krtap3-3, Krtap3-1, Krtap1-5, Krtap1-4, Krtap1-3, Krtap9-3, Gm11562, Krtap4-1, Krtap4-2, Krtap4-7, Gm11555, Gm11563, Krtap4-8, Krtap4-9, Gm11596, Krtap4-16, Gm11568, Gm11559, Krtap9-1, Gm11567, Krtap16-1, Krt33a, Krt33b, Krt34, Krt31, Krt35, Krt36, Krt14, Krt17, Evpl, Dsp, Krt80, Krt87, Krt81, Krt86, Krt83, Krt84, Krt82, Krt75, Krt6a, Krt5, Krt71, Krt72, Krt73, Krt1, Krt77, Krt79, Krtap13-1, Krtap15, Krtap19-2, Krtap19-3, Krtap19-4, Krtap16-3, Gm10229, Krtap6-1, Gm10228, Krtap6-5, Krtap6-3, Gm10061, 1110057P08Rik, Krtap8-1, Krtap11-1, Dsc3, Dsc2, Dsc1, Dsg1a, Dsg4, Spink5, Lipm, Col3a1, Col5a2, Fn1, Fmod, Ddr2, Olfml2b, Dpp4, Postn, Ctss, Col11a1, Col16a1, Angptl7, Mmp23, Emilin1, Pdgfra, Col1a2, Loxl3, Antxr1, Fbln2, Tgfb1, Gas2, Serpinh1, Comp, Mmp2, Mmp12, Mmp11, Lum, Aebp1, Adamts2, Collal, Itgb3, Lamb1, Nid1, Ecm2, Mmp14, Scara3, Loxl2, Col14a1, Col22a1, Scx, Fblnl, Myh11, Abi3bp, Col8a1, Smoc2, Tnxb, Lox, Nfkb1, Srebf2, Sp1, Trp53, Xbp1, Myod1, Srebf1, Jun, Stat3, Sp3, Myf6, Nr3c1, Foxo3, Nfe2l2, Myf5, Ahr, Ep300, Myog, and Usf2.

27. The method of claim 25 or 26, wherein the sarcopenia related disease, disorder, or symptom comprises at least one selected from the group consisting of a disease, disorder, or symptom due to a muscle strength disorder; a disease, disorder, or symptom due to an energy metabolism disorder; a disease, disorder, or symptom due to a nervous system disorder; and a disease, disorder, or symptom due to a secretory disorder.

28. The method of any one of claims 25 to 27, wherein the sarcopenia related disease, disorder, or symptom comprises at least one selected from the group consisting of primary sarcopenia, secondary sarcopenia, and a disease, disorder, or symptom accompanied therewith.

29. The method of any one of claims 25 to 28, wherein the sarcopenia related disease, disorder, or symptom is selected from a sarcopenia associated life-style disease, a wasting disease, a tumor associated disease, a motor disease, a neurodegenerative disease, cognitive dysfunction, and other associated disorders.

30. The method of any one of claims 25 to 29, wherein the sarcopenia related disease, disorder, or symptom is of a system selected from the group consisting of a digestive system, a circulatory system, a respiratory system, a urinary system, a reproductive system, an endocrine system, a sensory nervous system, a cranial nervous system, and a locomotor system.
